(19) Europäisches Patentamt
European Patent Office
Office européen des brevets

(11) EP 4 559 484 A1

(12) EUROPEAN PATENT APPLICATION
published in accordance with Art. 153(4) EPC

(43) Date of publication:
**28.05.2025 Bulletin 2025/22**

(21) Application number: **23843050.8**

(22) Date of filing: **20.07.2023**

(51) International Patent Classification (IPC):
***A61K 47/36*** *(2006.01)* ***A61K 9/08*** *(2006.01)*
***A61K 38/02*** *(2006.01)* ***A61K 38/12*** *(2006.01)*
***A61K 38/13*** *(2006.01)* ***A61K 45/00*** *(2006.01)*
***A61K 47/10*** *(2017.01)* ***A61K 47/22*** *(2006.01)*
***A61K 47/26*** *(2006.01)* ***A61K 47/40*** *(2006.01)*
***A61P 37/06*** *(2006.01)*

(52) Cooperative Patent Classification (CPC):
**A61K 9/08; A61K 38/02; A61K 38/12; A61K 38/13; A61K 45/00; A61K 47/10; A61K 47/22; A61K 47/26; A61K 47/36; A61K 47/40; A61P 37/06**

(86) International application number:
**PCT/JP2023/026679**

(87) International publication number:
**WO 2024/019127 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.07.2022 JP 2022115839**
**20.07.2022 JP 2022115841**
**22.09.2022 JP 2022151823**

(71) Applicant: **Asahi Kasei Kabushiki Kaisha**
**Tokyo 1000006 (JP)**

(72) Inventors:
- **YABUUCHI Kohei**
  **Tokyo 100-0006 (JP)**
- **NAKAI Takashi**
  **Tokyo 100-0006 (JP)**

(74) Representative: **Hoffmann Eitle**
**Patent- und Rechtsanwälte PartmbB**
**Arabellastraße 30**
**81925 München (DE)**

(54) **HYALURONIC ACID DERIVATIVE PHARMACEUTICAL COMPOSITION AND METHOD FOR PRODUCING PHARMACEUTICAL COMPOSITION**

(57) A hyaluronic acid derivative pharmaceutical composition includes a hyaluronic acid derivative (A) into which a hydrophobic group has been introduced, an association promoter (B), and an active ingredient (C). It is preferable that the association promoter (B) has at least four or more ether structures (R-O-R) and 4 or more carbon atoms.

FIG. 1

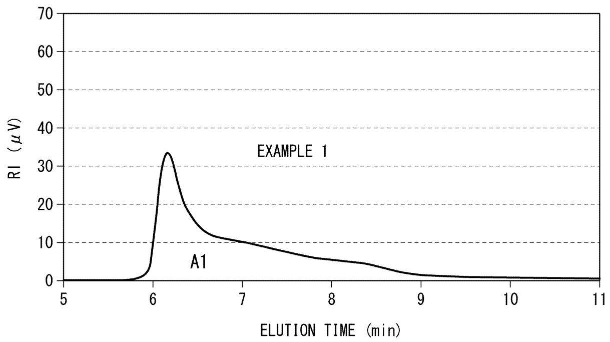




Processed by Luminess, 75001 PARIS (FR)

## Description

TECHNICAL FIELD

The present invention relates to a hyaluronic acid derivative pharmaceutical composition and a method of producing a pharmaceutical composition.

Priority is claimed on Japanese Patent Application No. 2022-115839, filed July 20, 2022, Japanese Patent Application No. 2022-151823, filed September 22, 2022, and Japanese Patent Application No. 2022-115841, filed July 20, 2022, the contents of which are incorporated herein by reference.

BACKGROUND ART

In recent years, biopharmaceuticals, which are pharmaceutical products containing proteins, peptides, or nucleic acids as active ingredients, have been put into practical use, and the number of biopharmaceuticals has been increasing year by year. Biopharmaceuticals can satisfy unmet medical needs that have not been satisfied by low-molecular-weight drugs.

However, biopharmaceuticals have problems in that the biopharmaceuticals are difficult to be absorbed from the digestive tract, the mucous membrane, and the like, and are unstable in the body and have a short half-life in blood. Therefore, the biopharmaceuticals require frequent administration by injection, which is a heavy burden on both patients and medical personnel. Therefore, there is a demand for a drug base material (a sustained-release drug delivery system base material) that can encapsulate a biopharmaceutical and gradually release an active ingredient in vivo without impairing the pharmacological activity.

From such a background, Patent Document 1 suggests a sustained-release drug delivery system base material formed of a hyaluronic acid derivative having excellent safety.

The hyaluronic acid derivative disclosed in Patent Document 1 can spontaneously associate in an aqueous solution, and can efficiently enclose a drug, particularly a biopharmaceutical, while maintaining the biological activity thereof. In this manner, the derivative aggregates at a physiological saline concentration (or is dispersed even at a physiological saline concentration) and has satisfactory blood retention properties. This hyaluronic acid derivative can be used as a carrier which can efficiently enclose a large amount of a drug while maintaining pharmacological activity, a blood sustained-release carrier having excellent blood retention properties, and a targeting carrier, particularly in a case where a biopharmaceutical is used as an active ingredient, and the hyaluronic acid derivative is also considered to be used as a local (for example, subcutaneous) sustained-release carrier which can continuously allow sustained-release of a drug.

Meanwhile, in the pharmaceutical industry, there are many pharmaceutical active ingredients that cannot exhibit the effect of a pharmaceutical active ingredient having strong biological activity due to low water solubility, and thus the development of the pharmaceutical active ingredient is relinquished, or the pharmaceutical active ingredient is marketed as a preparation that can exhibit only an activity lower than the original activity.

As a method of solubilizing a water-insoluble or poorly water-soluble pharmaceutical active ingredient, the following methods (a) to (c) are available.

(a) Method of changing a part of a structure of a drug into a soluble derivative: a soluble derivative such as a hydrochloride, a hydrobromate, a sulfate, a methanesulfonate, a sodium salt, a potassium salt, or a sodium sulfonate is used.

(b) Method of using a single organic solvent or a mixed solvent of an aqueous solvent and an organic solvent: a solubilizing method using methanol, ethanol, or the like.

(c) Method of adding a dissolution aid: a solubilizing method by adding a surfactant for micellization and emulsification. A method of using serum albumin or plasma protein.

However, in the method (a), a part of the structure of the pharmaceutical drug substance itself, which is the active ingredient, is converted, and thus the solubility of the drug substance itself cannot be increased. In addition, in a case of being converted into a derivative, various problems such as a decrease in activity as a pharmaceutical product and precipitation of a drug due to a change in pH may occur, which indicates that the method (a) is not a desired method.

In the method (b) using an organic solvent such as methanol described above, there are extremely few safe organic solvents that are biologically inactive and do not cause hemolysis, and the method (b) is not practically used in the pharmaceutical field. For example, Patent Document 2 describes a production method including a step of dissolving a poorly water-soluble cyclosporin composition in an organic solvent, a solubilizing aid, or a mixed solvent of water and an organic solvent or water and a solubilizing aid, or water, an organic solvent, and a solubilizing aid.

However, the obtained solution is a turbid solution, and it can be seen that partial precipitation is confirmed and solubilization is not sufficient. This indicates that the poorly water-soluble drug active ingredient is precipitated, and thus

sufficient activity cannot be obtained, and the toxicity to a living body due to the precipitation is not improved.

In the method (c) using a surfactant described above, the reality is that there are extremely few surfactants that are safe for a living body and exhibit effective solubility. Although a surfactant obtained by dissolving a drug Taxol using polyoxyethylated castor oil (Cremophor EL) is present in the related art, Non-Patent Document 1 has reported that polyoxyethylated castor oil causes acute immunotoxicity exhibited by a hypersensitivity reaction (HSR) occurring as a result of activation of the complement system without involving IgE. Since there are extremely few safe and useful surfactants, there is a preparation in which paclitaxel is dissolved using toxic Cremophor.

In Patent Document 3, in a case where a poorly water-soluble active ingredient is enclosed in a hyaluronic acid derivative, it is necessary to dissolve the poorly water-soluble active ingredient in an organic solvent such as methanol. Therefore, the use of an organic solvent is unavoidable. In addition, in Patent Document 3, the amount of the poorly water-soluble active ingredient to be solubilized is not sufficient. In addition, a formulation method of efficiently solubilizing a poorly water-soluble active ingredient in a powdery state has not been specifically examined, and there is room for improvement.

In Patent Document 2 or the like, even in a case where a solubilizing aid such as polyethylene glycol 300 is used for a poorly water-soluble active ingredient, only a suspension containing a relatively stable poorly water-soluble active ingredient is provided, and it is difficult to completely solubilize a poorly water-soluble active ingredient at a high concentration.

Citation List

Patent Documents

Patent Document 1: PCT International Publication No. WO2010/053140

Patent Document 2: United States Patent Application, Publication No. 2020/0237859

Patent Document 3: Published Japanese Translation No. 2014-534410 of the PCT International Publication


Non-Patent Document


Non-Patent Document 1: Toxicology 216 (2005) 106-121


SUMMARY OF INVENTION

Technical Problem

A sustained-release drug base material is required to maintain the concentration of the active ingredient in vivo for a longer period of time and to gradually release the active ingredient. In addition, it is required that the active ingredient can be solubilized at a high concentration.

The present invention has been made in consideration of the above-described circumstances, and an object thereof is to provide a hyaluronic acid derivative pharmaceutical composition capable of maintaining a concentration of an active ingredient in vivo for a longer period of time, gradually releasing the active ingredient, and solubilizing the active ingredient at a high concentration, and a method of producing the same.

Further, the present invention has been made in consideration of the above-described circumstances, and another object thereof is to provide a hyaluronic acid derivative pharmaceutical composition capable of solubilizing a poorly water-soluble active ingredient at a high concentration without using an organic solvent while reducing the amount of a highly toxic surfactant to be used, and a method of producing the same. Solution to Problem

That is, the present invention includes the following aspects.

[1] A hyaluronic acid derivative pharmaceutical composition including: a hyaluronic acid derivative (A) into which a hydrophobic group has been introduced; an association promoter (B); and an active ingredient (C).

[2] The hyaluronic acid derivative pharmaceutical composition according to [1], in which the association promoter (B) has at least four or more ether structures (R-O-R) and has 4 or more carbon atoms.

[3] The hyaluronic acid derivative pharmaceutical composition according to [1] or [2], in which the association promoter (B) is one or more selected from the group consisting of polysorbate 80, polysorbate 20, poloxamer, oxyethylene castor oil, polyethylene glycol 300, polyethylene glycol 400, polyethylene glycol 4000, fatty acid sorbitan ester, tocopheryl polyethylene glycol succinate, and polyvinyl alcohol.

[4] The hyaluronic acid derivative pharmaceutical composition according to any one of [1] to [3], in which a precipitate

is generated at a physiological saline concentration.

[5] The hyaluronic acid derivative pharmaceutical composition according to any one of [1] to [4], in which the active ingredient (C) is at least one selected from a protein or a poorly water-soluble drug. [6] The hyaluronic acid derivative pharmaceutical composition according to [5], in which the poorly water-soluble drug has a solubility of 1 mg/mL or less in water.

[7] The hyaluronic acid derivative pharmaceutical composition according to [5] or [6], in which the poorly water-soluble drug has a molecular weight of 200 or greater.

[8] The hyaluronic acid derivative pharmaceutical composition according to any one of [5] to [7], in which the poorly water-soluble drug is a poorly water-soluble peptide.

[9] The hyaluronic acid derivative pharmaceutical composition according to [8], in which in the poorly water-soluble peptide, at least one nitrogen atom constituting an amide bond has a methyl group.

[10] The hyaluronic acid derivative pharmaceutical composition according to [8] or [9], in which the poorly water-soluble peptide includes at least one or more selected from the group consisting of a cyclic peptide and a long-chain peptide.

[11] The hyaluronic acid derivative pharmaceutical composition according to any one of [8] to [10], in which the poorly water-soluble peptide is a cyclic peptide.

[12] The hyaluronic acid derivative pharmaceutical composition according to any one of [1] to [11], in which the amount of the active ingredient (C) is 10 parts by mass or greater and 100 parts by mass or less with respect to 100 parts by mass of the hyaluronic acid derivative (A) into which the hydrophobic group has been introduced.

[13] The hyaluronic acid derivative pharmaceutical composition according to any one of [1] to [12], in which the hyaluronic acid derivative (A) into which the hydrophobic group has been introduced has one or more repeating units represented by General Formula (I).

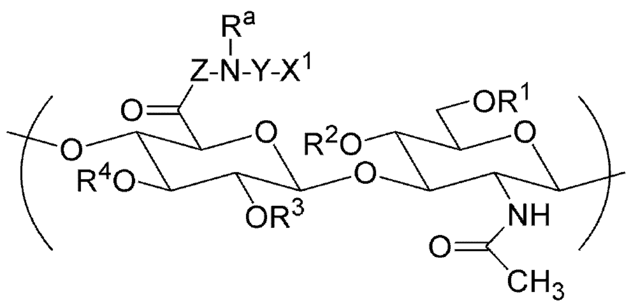

(I)

(In the formula, $R^1$, $R^2$, $R^3$, and $R^4$ each independently represent a group selected from the group consisting of a hydrogen atom, $C_{1-6}$ alkyl, formyl, and $C_{1-6}$ alkylcarbonyl. Z represents a direct bond or a peptide linker formed of 2 or more and 30 or less of any amino acid residues.

$X^1$ represents a group selected from the group consisting of a group represented by $-NR^b-R$, $-NR^b-COO-R$, $-NR^b-CO-R$, $-NR^b-CO-NR^c-R$, -COO-R, -O-COO-R, -S-R, - $CO-Y^a-S-R$, $-O-CO-Y^b-S-R$, $-NR^b-CO-Y^b-S-R$, and -S-S-R.

$R^a$, $R^b$, and $R^c$ each independently represent a group selected from the group consisting of a hydrogen atom, $C_{1-20}$ alkyl, amino $C_{2-20}$ alkyl, and hydroxy $C_{2-20}$ alkyl. A group selected from the group consisting of -O- and $-NR^f-$ may be inserted into an alkyl moiety of each of $R^a$, $R^b$, and $R^c$.

$R^f$ represents a group selected from the group consisting of a hydrogen atom, $C_{1-12}$ alkyl, amino $C_{2-12}$ alkyl, and hydroxy $C_{2-12}$ alkyl. A group selected from the group consisting of -O- and -NH- may be inserted into an alkyl moiety of $R^f$.

R represents a steryl group.

Y represents $C_{2-30}$ alkylene or $-(CH_2CH_2O)_m-CH_2CH_2-$. Here, a group selected from the group consisting of -O-, $-NR^g-$, and -S-S- may be inserted into alkylene of Y.

$R^g$ represents a group selected from the group consisting of a hydrogen atom, $C_{1-20}$ alkyl, amino $C_{2-20}$ alkyl, and hydroxy $C_{2-20}$ alkyl. A group selected from the group consisting of -O- and -NH- may be inserted into an alkyl moiety of $R^g$.

$Y^a$ represents $C_{1-5}$ alkylene.

$Y^b$ represents $C_{2-8}$ alkylene or $C_{2-8}$ alkenylene.

m represents an integer of 1 or greater and 100 or less.)

[14] The hyaluronic acid derivative pharmaceutical composition according to [13], in which the steryl group is a cholesteryl group.

[15] The hyaluronic acid derivative pharmaceutical composition according to [13] or [14], in which an introduction rate of the steryl group into the hyaluronic acid derivative is 7% or greater and less than 35%. [16] The hyaluronic acid derivative pharmaceutical composition according to any one of [1] to [15], in which in the hyaluronic acid derivative pharmaceutical composition, no precipitate is visually observed.

[17] The hyaluronic acid derivative pharmaceutical composition according to any one of [1] to [16], in which the hyaluronic acid derivative pharmaceutical composition is filter-sterilizable.

[18] A method of producing a hyaluronic acid derivative pharmaceutical composition including a hyaluronic acid derivative (A) into which a hydrophobic group has been introduced, an association promoter (B), and an active ingredient (C), the method including: a step of mixing the hyaluronic acid derivative (A) into which a hydrophobic group has been introduced with the association promoter (B) to obtain an association promoter-containing hyaluronic acid derivative aqueous solution; and a mixing step of mixing the active ingredient (C) with the association promoter-containing hyaluronic acid derivative aqueous solution.

[19] A method of producing a hyaluronic acid derivative pharmaceutical composition including a hyaluronic acid derivative (A) into which a hydrophobic group has been introduced, an association promoter (B), and an active ingredient (C), the method including: a step of dispersing the active ingredient (C) in the association promoter (B) to obtain a dispersion liquid (I); a step of preparing a hyaluronic acid derivative aqueous solution or an association promoter-containing hyaluronic acid aqueous solution to obtain an aqueous solution (II); and a step of mixing the dispersion liquid (I) with the aqueous solution (II).

[20] The method of producing a hyaluronic acid derivative pharmaceutical composition according to [18] or [19], in which the method does not include a step of removing an organic solvent.

Further, the present invention includes the following aspects.

[21] A hyaluronic acid derivative pharmaceutical composition including: a hyaluronic acid derivative (A1) into which a hydrophobic group has been introduced; a solubilizing aid (B1); and an active ingredient (C1), in which the solubilizing aid (B1) has at least four or more ether structures (R-O-R) and 4 or more carbon atoms, and the amount of the solubilizing aid (B1) is 0.0001 parts by mass or greater and 15,000 parts by mass or less with respect to 100 parts by mass of the hyaluronic acid derivative (A1) into which a hydrophobic group has been introduced.

[22] The hyaluronic acid derivative pharmaceutical composition according to [21], in which the solubilizing aid (B1) is one or more selected from the group consisting of a nonionic surfactant, polyethylene glycol having a molecular weight of 190 g/moL or greater and 4,000 g/moL or less, and a cyclodextrin derivative.

[23] The hyaluronic acid derivative pharmaceutical composition according to [21] or [22], in which the solubilizing aid (B1) is one or more selected from the group consisting of polysorbate 80, polysorbate 65, polysorbate 60, polysorbate 40, polysorbate 20, poloxamer, polyoxyethylene hydrogenated castor oil, cyclodextrin derivative, polyethylene glycol 300, polyethylene glycol 400, polyethylene glycol 4000, and tocopheryl polyethylene glycol succinate.

[24] The hyaluronic acid derivative pharmaceutical composition according to any one of [21] to [23], in which the solubilizing aid (B1) is a nonionic surfactant, and the amount of the nonionic surfactant is 0.0001 parts by mass or greater and 150 parts by mass or less with respect to 100 parts by mass of the hyaluronic acid derivative (A) into which the hydrophobic group has been introduced.

[25] The hyaluronic acid derivative pharmaceutical composition according to any one of [21] to [24], in which the solubilizing aid (B1) is polyethylene glycol having a molecular weight of 190 g/moL or greater and 4,000 g/moL or less, and the amount of the polyethylene glycol is 25 parts by mass or greater and 15,000 parts by mass or less with respect to 100 parts by mass of the hyaluronic acid derivative (A) into which the hydrophobic group has been introduced.

[26] The hyaluronic acid derivative pharmaceutical composition according to any one of [21] to [25], in which the active ingredient (C1) is a poorly water-soluble drug having a solubility of 1 mg/mL or less in water.

[27] The hyaluronic acid derivative pharmaceutical composition according to [26], in which the poorly water-soluble drug has a molecular weight of 500 or greater.

[28] The hyaluronic acid derivative pharmaceutical composition according to [26] or [27], in which the poorly water-soluble drug is a poorly water-soluble peptide.

[29] The hyaluronic acid derivative pharmaceutical composition according to [28], in which, in the poorly water-soluble peptide, at least one nitrogen atom constituting an amide bond has a methyl group.

[30] The hyaluronic acid derivative pharmaceutical composition according to [28] or [29], in which the poorly water-soluble peptide includes at least one or more selected from the group consisting of a cyclic peptide and a long-chain peptide.

[31] The hyaluronic acid derivative pharmaceutical composition according to any one of [28] to [30], in which the poorly water-soluble peptide is a cyclic peptide.

[32] The hyaluronic acid derivative pharmaceutical composition according to any one of [26] to [31], in which an amount of the poorly water-soluble drug to be blended is 21 parts by mass or greater and less than 100 parts by mass with respect to 100 parts by mass of the hyaluronic acid derivative (A1) into which the hydrophobic group has been introduced.

[33] The hyaluronic acid derivative pharmaceutical composition according to [31] or [32], in which the hyaluronic acid derivative has one or more repeating units represented by General Formula (I).

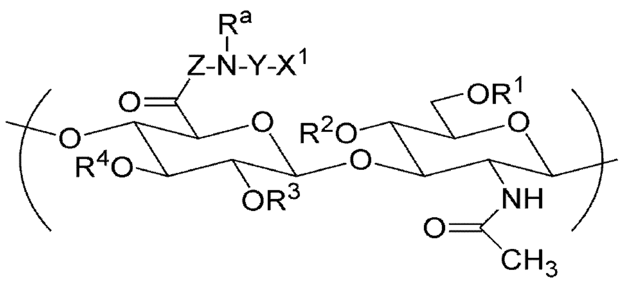
(I)

(In the formula, $R^1$, $R^2$, $R^3$, and $R^4$ each independently represent a group selected from the group consisting of a hydrogen atom, $C_{1-6}$ alkyl, formyl, and $C_{1-6}$ alkylcarbonyl. Z represents a direct bond or a peptide linker formed of 2 or more and 30 or less of any amino acid residues.

$X^1$ represents a group selected from the group consisting of a group represented by $-NR^b-R$, $-NR^b-COO-R$, $-NR^b-CO-R$, $-NR^b-CO-NR^c-R$, -COO-R, -O-COO-R, -S-R, $-CO-Y^a-S-R$, $-O-CO-Y^b-S-R$, $-NR^b-CO-Y^b-S-R$, and -S-S-R.

$R^a$, $R^b$, and $R^c$ each independently represent a group selected from the group consisting of a hydrogen atom, $C_{1-20}$ alkyl, amino $C_{2-20}$ alkyl, and hydroxy $C_{2-20}$ alkyl. A group selected from the group consisting of -O- and $-NR^f-$ may be inserted into an alkyl moiety of each of $R^a$, $R^b$, and $R^c$.

$R^f$ represents a group selected from the group consisting of a hydrogen atom, $C_{1-12}$ alkyl, amino $C_{2-12}$ alkyl, and hydroxy $C_{2-12}$ alkyl. A group selected from the group consisting of -O- and -NH- may be inserted into an alkyl moiety of $R^f$.

R represents a steryl group.

Y represents $C_{2-30}$ alkylene or $-(CH_2CH_2O)_m-CH_2CH_2-$. Here, a group selected from the group consisting of -O-, $-NR^g-$, and -S-S- may be inserted into alkylene of Y.

$R^g$ represents a group selected from the group consisting of a hydrogen atom, $C_{1-20}$ alkyl, amino $C_{2-20}$ alkyl, and hydroxy $C_{2-20}$ alkyl. A group selected from the group consisting of -O- and -NH- may be inserted into an alkyl moiety of $R^g$.

$Y^a$ represents $C_{1-5}$ alkylene.

$Y^b$ represents $C_{2-8}$ alkylene or $C_{2-8}$ alkenylene.

m represents an integer of 1 or greater and 100 or less.)

[34] The hyaluronic acid derivative pharmaceutical composition according to [33], in which the steryl group is a cholesteryl group.

[35] The hyaluronic acid derivative pharmaceutical composition according to [33] to [34], in which an introduction rate of the steryl group to the hyaluronic acid derivative is 35% or greater and less than 50%. [36] The hyaluronic acid derivative pharmaceutical composition according to any one of [21] to [35], in which the amount of the hyaluronic acid derivative is 6 mg/mL or greater and less than 45 mg/mL with respect to the hyaluronic acid derivative pharmaceutical composition.

[37] The hyaluronic acid derivative pharmaceutical composition according to any one of [21] to [36], in which the amount of an organic solvent with respect to the hyaluronic acid derivative pharmaceutical composition is less than 0.8%.

[38] A method of producing a pharmaceutical composition including a hyaluronic acid derivative (A1) into which a hydrophobic group has been introduced, a solubilizing aid (B1), and an active ingredient (C1), the method including: a step of mixing the hyaluronic acid derivative (A1) with the solubilizing aid (B1) to obtain a solubilizing aid-containing hyaluronic acid derivative aqueous solution; and a mixing step of mixing the active ingredient (C1) with the solubilizing aid-containing hyaluronic acid derivative aqueous solution.

[39] A method of producing a pharmaceutical composition including a hyaluronic acid derivative (A1) into which a hydrophobic group has been introduced, a solubilizing aid (B1), and an active ingredient (C1), the method including: a step of dispersing the active ingredient (C1) in the solubilizing aid (B1) to obtain a dispersion liquid (I); a step of preparing a hyaluronic acid derivative aqueous solution or a solubilizing aid-containing hyaluronic acid aqueous solution to obtain an aqueous solution (II); and a step of mixing the dispersion liquid (I) with the aqueous solution (II).

[40] The method of producing a pharmaceutical composition according to [38] or [39], which includes the hyaluronic acid derivative (A1) into which a hydrophobic group has been introduced, the solubilizing aid (B1), and the active ingredient (C1), in which the method does not include a step of removing an organic solvent.

Advantageous Effects of Invention

According to the hyaluronic acid derivative pharmaceutical composition of the above-described aspect, it is possible to provide a hyaluronic acid derivative pharmaceutical composition capable of maintaining the concentration of an active ingredient in vivo for a longer period of time, gradually releasing the active ingredient, and solubilizing the active ingredient at a high concentration, and a method of producing the same.

According to the hyaluronic acid derivative pharmaceutical composition of the above-described aspect, it is possible to provide a hyaluronic acid derivative pharmaceutical composition capable of solubilizing a poorly water-soluble active ingredient at a high concentration without using an organic solvent while reducing the amount of a highly toxic surfactant to be used, and a method of producing the same.

BRIEF DESCRIPTION OF DRAWINGS

[FIG. 1] A chromatogram of a hyaluronic acid derivative.
[FIG. 2] A chromatogram of a hyaluronic acid derivative pharmaceutical composition.
[FIG. 3] A graph showing a change in blood plasma concentration of cyclosporin.
[FIG. 4] A graph showing a change in blood plasma concentration of cyclosporin.

DESCRIPTION OF EMBODIMENTS

Hereinafter, embodiments of the present invention (hereinafter, referred to as "present embodiment") will be described in detail, but the present invention is not limited thereto, and various modifications can be made without departing from the scope of the present invention.

Hereinafter, the terms used in the present specification will be described.

The term "$C_{1-20}$ alkyl" used in the present specification means a linear or branched alkyl group having 1 or more and 20 or less carbon atoms, and includes, for example, "$C_{1-4}$ alkyl" such as methyl, ethyl, n-propyl, iso-propyl, n-butyl, sec-butyl, isobutyl, or tert-butyl, and further includes n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl, or 2-ethylbutyl. The $C_{1-20}$ alkyl also includes a $C_{1-12}$ alkyl having 1 or more and 12 or less carbon atoms and a $C_{1-6}$ alkyl group having 1 or more and 6 or less carbon atoms.

The term "$C_{1-6}$ alkylcarbonyl" used in the present specification means an alkylcarbonyl group in which the alkyl moiety is the above-described $C_{1-6}$ alkyl, and includes, for example, "$C_{1-4}$ alkylcarbonyl" such as acetyl, propionyl, n-propylcarbonyl, iso-propylcarbonyl, n-butylcarbonyl, sec-butylcarbonyl, iso-butylcarbonyl, or tert-butylcarbonyl.

The term "amino $C_{2-20}$ alkyl" used in the present specification means a linear or branched alkyl having 2 or more and 20 or less carbon atoms, which has an amino group as a substituent, and for example, the amino group may be positioned on a carbon atom at a terminal of the alkyl group. The amino $C_{2-20}$ alkyl also includes an amino $C_{2-12}$ alkyl having 2 or more and 12 or less carbon atoms.

The term "hydroxy $C_{2-20}$ alkyl" used in the present specification means a linear or branched alkyl group having 2 or more and 20 or less carbon atoms, which has a hydroxy group as a substituent, and for example, the hydroxy group may be positioned on a carbon atom at a terminal of the alkyl group. The hydroxy $C_{2-20}$ alkyl also includes a hydroxy $C_{2-12}$ alkyl having 2 or more and 12 or less carbon atoms.

The term "$C_{2-30}$ alkylene" used in the present specification means a linear or branched divalent saturated hydrocarbon group having 2 or more and 30 or less carbon atoms, and includes, for example, ethylene and propylene, and $C_{2-20}$ alkylene having 2 or more and 20 or less carbon atoms, $C_{2-8}$ alkylene having 2 or more and 8 or less carbon atoms, and a group "$-(CH_2)_n-$" (here, n represents 2 or greater and 30 or less, preferably 2 or greater and 20 or less, and more preferably 2 or greater and 15 or less).

The term "$C_{1-5}$ alkylene" used in the present specification means a linear or branched divalent saturated hydrocarbon group having 1 or more and 5 or less carbon atoms, and includes, for example, methylene, ethylene, and propylene.

The term "$C_{2-8}$ alkenylene" described in the present specification means a divalent saturated hydrocarbon group which is linear or branched and has 2 or more and 8 or less carbon atoms and includes one or more double bonds, and includes, for example, $-CH=CH-$, $-C(CH_3)=CH-$, 2-butene-1,4-diyl, hepta-2,4-dien-1,6-diyl, and octa-2,4,6-triene-1,8-diyl. In a case where geometric isomers are present, each of the isomers and a mixture thereof are also included.

<Hyaluronic acid derivative pharmaceutical composition 1 >

The present embodiment is a hyaluronic acid derivative pharmaceutical composition containing a hyaluronic acid derivative (A) into which a hydrophobic group has been introduced, an association promoter (B), and an active ingredient (C). Hereinafter, "the hyaluronic acid derivative pharmaceutical composition containing a hyaluronic acid derivative (A) into which a hydrophobic group has been introduced, the association promoter (B), and the active ingredient (C)" may be abbreviated as "pharmaceutical composition 1".

The hyaluronic acid derivative can be used as a pharmaceutical composition by formulation with an active ingredient.

In the pharmaceutical composition 1 according to the present embodiment, the hyaluronic acid derivative forms a complex with the active ingredient (hereinafter, also referred to as "active ingredient-hyaluronic acid derivative complex"). Specifically, it is presumed that a steryl group in the hyaluronic acid derivative and a hydrophobic site of the active ingredient form a complex by a hydrophobic interaction, the active ingredient and the hydrophobic site such as the steryl group are present in the central portion, and the hydrophilic site such as a site derived from hyaluronic acid in the hyaluronic acid derivative is present in the outer edge portion, thereby exhibiting a cylinder structure or a core-shell type cylinder structure. That is, it is presumed that the active ingredient exhibits a structure in which the active ingredient is enclosed or encapsulated in the hyaluronic acid derivative.

In the pharmaceutical composition 1 according to the present embodiment, the average particle diameter of the structure containing the active ingredient-hyaluronic acid derivative complex can be set to 20 nm or greater and 220 nm or less, 20 nm or greater and 150 nm or less, or 30 nm or greater and 100 nm or less. In a case where the average particle diameter is within the above-described numerical ranges, sterilization filtration can be performed, and the effect as a sustained-release base material can be exhibited by stably aggregating and precipitating in vivo. The average particle diameter can be measured by, for example, dynamic light scattering (DLS), a nanotracking particle measurement device, size exclusion chromatography, high performance liquid chromatography, an electron microscope method, or the like. More specifically, for example, the measurement is carried out by diluting the sample with a 10 mM phosphate buffer solution containing 10 w/v% sucrose at a proportion at which the concentration of the hyaluronic acid derivative is 1 mg/mL, using a DLS device.

It is preferable that the association promoter (B) contained in the pharmaceutical composition 1 according to the present embodiment has at least four or more ether structures (R-O-R) and 4 or more carbon atoms.

The pharmaceutical composition 1 according to the present embodiment contains an association promoter (B).

The association promoter (B) in the present specification interacts with a hyaluronic acid derivative into which a hydrophobic group has been introduced, and promotes the association between the hyaluronic acid derivative molecules. The association promoter of the present embodiment forms a complex with a hyaluronic acid derivative, increases the drug compounding ability, and improves the precipitation performance at a physiological saline concentration. In this regard, after the gel is precipitated subcutaneously, the drug in the gel is released in a sustained manner by an exchange reaction in which the drug is replaced with hydrophobic components such as albumin in vivo or other hydrophobic proteins or by decomposition of the gel. It is considered that the sustained-release period can be controlled by controlling the sustained-release of the drug by the exchange reaction, suppressing the inflow of the gel-decomposing enzyme, and shielding the gel-decomposing enzyme to impart a steric property.

Next, constituent components of the present embodiment will be described in detail.

<<Hyaluronic acid derivative into which hydrophobic group has been introduced>>

The pharmaceutical composition 1 according to the present embodiment contains a hyaluronic acid derivative (A) into which a hydrophobic group has been introduced. In the following description, "hyaluronic acid derivative (A) into which a hydrophobic group has been introduced" may be referred to as "hyaluronic acid derivative (A)".

The hyaluronic acid derivative (A) contains a steryl group as a hydrophobic group. The steryl group may be directly bonded to hyaluronic acid or may be bonded to hyaluronic acid via a linker.

As the linker referred to herein, any peptide linker or synthetic compound linker that can be introduced by genetic engineering can be used, but in the hyaluronic acid derivative (A) used in the present embodiment, a peptide linker is preferable.

The length of the peptide linker is not particularly limited, and can be appropriately selected by those skilled in the art according to the purpose, but the length is preferably 2 amino acids or more and particularly preferably 15 amino acids. The upper limit of the length of the peptide linker is not particularly limited, but is usually 30 amino acids or less and preferably 20 amino acids or less. **In** the peptide linker contained in the hyaluronic acid derivative (A), peptide linkers having the same length or peptide linkers having different lengths may be used.

**In** a case where the hyaluronic acid derivative (A) has a steryl group as a hydrophobic group, the steryl group in the hyaluronic acid derivative self-associates in water, and a single molecule or a plurality of molecules associate to form a

hydrogel having a nanosize.

[Steryl group]

The term "steryl group" used in the present specification is not particularly limited as long as the group has a steroid skeleton. Here, specific examples of the steroid include cholesterol, cholestanol, campesterol, ergostanol, stigmasterol, coprostanol, stigmasterol, sitosterol, lanosterol, ergosterol, simianerol, bile acid, testosterone, estradiol, progesterone, cortisol, cortisone, aldosterone, corticosterone, and deoxycorticosterone. Examples of the steryl group include a cholesteryl group, a stigmasteryl group, a lanosterol group, and an ergosterol group. Among these, a cholesteryl group (particularly, a cholesta-5-en-3β-yl group) is preferable.

[Introduction rate of steryl group]

The introduction rate of the steryl group to the hyaluronic acid derivative (A) (hereinafter, also simply referred to as "steryl group introduction rate") is preferably 0.1% or greater and less than 50%, more preferably 5% or greater and less than 45%, still more preferably 10% or greater and 40% or less, and particularly preferably 15% or greater and 35% or less.

In a case where the introduction rate of the steryl group is within the above-described ranges, the hyaluronic acid derivative (A) can strongly interact with the hydrophobic moiety of the poorly water-soluble drug or protein and the hydrophobic moiety of the association promoter. In addition, in a case where the introduction rate of the steryl group is within the above-described ranges, the hyaluronic acid derivative-drug complex in which the hyaluronic acid derivative (A) in the pharmaceutical composition 1 is compounded with the drug improves the stability of the preparation and enables the drug to be released in a sustained manner by aggregation and precipitation at a physiological saline concentration.

The introduction rate of the steryl group can be measured by $^1$H-NMR measurement. That is, the introduction rate of the steryl group can be calculated based on the following equation using the integral value of the peak derived from the steryl group of the hyaluronic acid derivative (A) in the $^1$H-NMR spectrum of the pharmaceutical composition 1 and the integral value of the peak ($COCH_3$, 1.6 ppm or more and 2.0 ppm or less, 3H) derived from the acetyl group of N-acetyl-D-glucosamine contained in the hyaluronic acid derivative (A). Further, in the equation, $n_H$ represents the number of hydrogen atoms corresponding to the peak. Specifically, the measurement can be carried out, for example, by a method described in examples described below.

[Introduction rate of steryl group] (%) = [(peak integral value derived from steryl group $\times$ 3/$n_H$)/(peak integral value derived from acetyl group of N-acetyl-D-glucosamine)] $\times$ 100

The molecular weight of the hyaluronic acid derivative (A) is not particularly limited, but from the viewpoint of improving the sustained-release function derived from the diffusion delay in local administration, the hyaluronic acid derivative (A) having a relatively large molecular weight is preferable. Meanwhile, in a case where the final formulation is a solution preparation, the hyaluronic acid derivative (A) having a relatively low molecular weight is preferable from the viewpoint of syringe availability. The molecular weight of the hyaluronic acid derivative (A) may be appropriately adjusted depending on the intended use and dosage form.

Example of the molecular weight of the hyaluronic acid derivative (A) is preferably 1,000 (1 k) or greater and 1,000,000 (1,000 k) or less, more preferably 5 k or greater and 300 k or less, still more preferably 5 k or greater and 120 k or less, and particularly preferably 7 k or greater and 100 k or less. The molecular weight of the hyaluronic acid derivative (A) can usually be adjusted by using a raw material having a corresponding molecular weight.

In a case where the weight-average molecular weight of the hyaluronic acid derivative is greater than or equal to the above-described lower limits, an increase in viscosity can be suppressed, and a higher concentration of the hyaluronic acid derivative can be dissolved in the pharmaceutical composition 1. The weight-average molecular weight of the hyaluronic acid derivative can usually be adjusted by using a raw material having a corresponding molecular weight.

More specifically, the weight-average molecular weight of the hyaluronic acid derivative is preferably 100 k or less, more preferably 50 k or less, and particularly preferably 40 k or less, from the viewpoint of viscosity.

From the viewpoint of producing the hyaluronic acid derivative, the molecular weight is preferably 4k to 100k and particularly preferably 6k to 50k.

From the viewpoint that the hyaluronic acid derivative strongly exhibits the properties of hyaluronic acid, for example, in order to be strongly recognized by the CD44 receptor, the molecular weight is preferably 100 kDa or greater, particularly preferably 200 kDa or greater, and most preferably 300 kDa or greater.

The term "molecular weight of the hyaluronic acid derivative" described herein is a weight-average molecular weight determined by a size exclusion chromatography multi-angle light scattering detector (SEC-MALS).

Specific preferred examples of the hyaluronic acid derivative include a hyaluronic acid derivative having one or

more repeating units represented by General Formula (I) (hereinafter, also referred to as "repeating unit (I)").

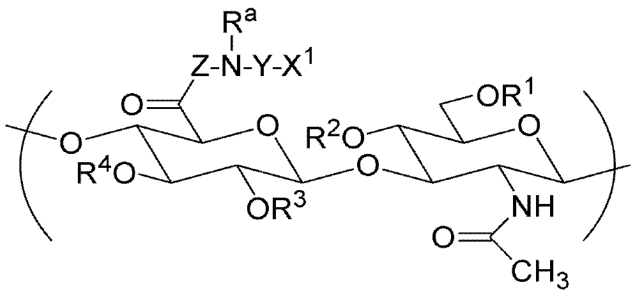

(I)

(In the formula, $R^1$, $R^2$, $R^3$, and $R^4$ each independently represent a group selected from the group consisting of a hydrogen atom, $C_{1-6}$ alkyl, formyl, and $C_{1-6}$ alkylcarbonyl.

Z represents a direct bond or a peptide linker formed of 2 or more and 30 or less of any amino acid residues.

$X^1$ represents a group selected from the group consisting of a group represented by $-NR^b-R$, $-NR^b-COO-R$, $-NR^b-CO-R$, $-NR^b-CO-NR^c-R$, -COO-R, -O-COO-R, -S-R, $-CO-Y^a-S-R$, $-O-CO-Y^b-S-R$, $-NR^b-CO-Y^b-S-R$, and -S-S-R.

$R^a$, $R^b$, and $R^c$ each independently represent a group selected from the group consisting of a hydrogen atom, $C_{1-20}$ alkyl, amino $C_{2-20}$ alkyl, and hydroxy $C_{2-20}$ alkyl. Here, a group selected from the group consisting of -O- and $-NR^f-$ may be inserted into the alkyl moiety of each of $R^a$, $R^b$, and $R^c$.

$R^f$ represents a group selected from the group consisting of a hydrogen atom, $C_{1-12}$ alkyl, amino $C_{2-12}$ alkyl, and hydroxy $C_{2-12}$ alkyl. A group selected from the group consisting of -O- and -NH- may be inserted into an alkyl moiety of $R^f$.

R represents a steryl group.

Y represents $C_{2-30}$ alkylene or $-(CH_2CH_2O)_m-CH_2CH_2-$. Here, a group selected from the group consisting of -O-, $-NR^g-$, and -S-S- may be inserted into alkylene of Y.

$R^g$ represents a group selected from the group consisting of a hydrogen atom, $C_{1-20}$ alkyl, amino $C_{2-20}$ alkyl, and hydroxy $C_{2-20}$ alkyl. A group selected from the group consisting of -O- and -NH- may be inserted into an alkyl moiety of $R^g$.

$Y^a$ represents $C_{1-5}$ alkylene.

$Y^b$ represents $C_{2-8}$ alkylene or $C_{2-8}$ alkenylene.

m represents an integer of 1 or greater and 100 or less.)

The hyaluronic acid derivative preferably includes a hyaluronic acid derivative having one or more repeating units represented by General Formula (Ia) (hereinafter, also referred to as "repeating unit (Ia)").

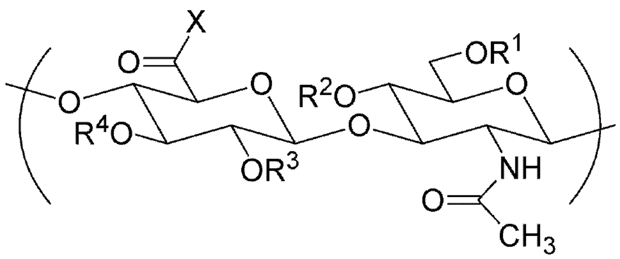

(Ia)

(In the formula, $R^1$, $R^2$, $R^3$, and $R^4$ each independently represent a group selected from the group consisting of a hydrogen atom, $C_{1-6}$ alkyl, formyl, and $C_{1-6}$ alkylcarbonyl. X represents a hydrophobic group represented by $-NR^a-Y-NR^b-COO-R$. $R^a$ and $R^b$ each independently represent a group selected from the group consisting of a hydrogen atom and $C_{1-6}$ alkyl. R represents a steryl group. Y represents $C_{2-30}$ alkylene or $-(CH_2CH_2O)_m-CH_2CH_2-$, where m represents an integer of 1 or greater and 100 or less)

Here, in a case where the hyaluronic acid derivative has two or more of the repeating unit (I) or two or more of the repeating units (Ia), the repeating units may be the same as or different from each other.

The hyaluronic acid derivative may be modified at a position other than the repeating unit (I) or the repeating unit (Ia), for example, the hydroxy group may be converted into $-O(C_{1-6}$ alkyl), -O(formyl), $-O(C_{1-6}$ alkylcarbonyl), or the like, and the carboxy group may be converted into an amide or an ester or form a salt.

[Repeating unit (I)]

The group "$-Z-N(R^a)Y-X^1$" in General Formula (I) includes a group selected from the group consisting of groups represented by the following formulae: $-NH-(CH_2)_{mz}-NH-R$; $-NH-(CH_2)_{mz}-NH-COO-R$; $-NH-(CH_2CH_2O)_m-CH_2CH_2-NH-$

COO-R; - NH-$(CH_2)_{mz}$-COO-R; -NH-$(CH_2CH_2O)_m$,-$CH_2CH_2$-COO-R, -NH-$(CH_2)_{mz}$-O-COO-R; -NH-$(CH_2CH_2O)_m$-$CH_2CH_2$-O-COO-R, -NH-$(CH_2)_{mz}$-S-R; -NH-$(CH_2CH_2O)_m$-$CH_2CH_2$-S-R; -NH-$(CH_2)_{mz}$-O-CO-CH($R^8$)-$CH_2$-S-R; -NH-$(CH_2)_{mz}$-NHCO-CH($R^8$)-$CH_2$-S-R; - NH-$(CH_2CH_2O)_m$-$CH_2CH_2$-NHCO-CH($R^8$)-$CH_2$-S-R; -NH-$(CH_2CH_2O)_m$-$CH_2CH_2$-O-CO-CH($R^8$)-$CH_2$-S-R; -NH-$(CH_2)_{mz}$-S-S-R; and -Z-N$R^a$-Y N$R^b$-COO-R (here, mz represents an integer of 2 or greater and 30 or less, $R^8$ represents a hydrogen atom or a methyl group, and R and m are as defined in the present specification).

As the group, a group selected from the group consisting of -NH-$(CH_2)_{mz}$-NH-COO-R; -NH-$(CH_2CH_2O)_m$-$CH_2CH_2$-NH-COO-R; and -NH-$(CH_2)_{mz}$-S-S-R (here, mz, R, and m are as defined in the present specification) is preferable.

(Z)

In General Formula (I), it is preferable that Z represents a direct bond. In addition, in another aspect, in a case where Z represents a peptide linker, it is preferable that $X^1$ represents -N$R^b$-COO-R. Further, in another aspect, Z may represent a peptide linker represented by -NH-[CH(-$Z^a$)-CONH]$_{n-1}$-CH(-$Z^a$)-CO-, where n represents an integer of 2 or greater and 30 or less, and $Z^a$'s each independently represent a substituent in an $\alpha$-amino acid represented by $H_2$N-CH(-$Z^a$)-COOH. The peptide linker is bonded to a carboxy group of a glucuronic acid moiety at the N-terminal and is bonded to a group-N(-$R^a$)-Y-$X^1$ at the C-terminal. Examples of the amino acid that can be used as the amino acid residue of the peptide linker include natural type (L-type) amino acids such as $\alpha$-amino acids such as alanine, arginine, asparagine (Asn), aspartic acid, cysteine, glutamine, glutamic acid, glycine (Gly), histidine, isoleucine, leucine (Leu), lysine, methionine, phenylalanine (Phe), proline, serine, threonine, tryptophan, tyrosine, and valine, and D-forms thereof, and all $\alpha$-amino acids including the synthesized amino acids can be used. That is, examples of $Z^a$ include -$CH_3$, $H_2$NC(NH)NH$(CH_2)_3$-, and $H_2$NCO$CH_2$-. In addition, n pieces of Z's may be the same as or different from each other. n represents an integer of 2 or greater and 30 or less, preferably 2 or greater and 10 or less, and more preferably 2 or greater and 4 or less. Preferred examples of the peptide linker include -Gly-Phe-Leu-Gly-, -Asn-Phe-Phe-, -Phe-Phe-, and Phe-Gly-.

(Y)

In General Formula (I), it is preferable that Y represents a group selected from the group consisting -$(CH_2)_{n1}$- and -$(CH_2CH_2O)_{m1}$-$CH_2CH_2$- (here, n1 represents an integer of 2 or greater and 20 or less, preferably an integer of 2 or greater and 15 or less, more preferably an integer of 2 or greater and 12 or less, and still more preferably an integer of 2 or greater and 6 or less. m1 represents an integer of 1 or greater and 4 or less). Specifically, -$(CH_2)_2$-, -$(CH_2)_6$-, -$(CH_2)_8$-, -$(CH_2)_{12}$-, or -$(CH_2CH_2O)_2$-$CH_2CH_2$- is preferable. In addition, from the viewpoint of realizing high solubility in pure water or a low salt concentration and exhibiting high precipitate forming ability at a physiological saline concentration, Y represents preferably a group selected from the group consisting of -$(CH_2)_2$-, -$(CH_2)_6$-, -$(CH_2)_8$-, and -$(CH_2)_{12}$- and more preferably -$(CH_2)_6$-.

Y may represent, for example, -$CH_2CH_2$O-$CH_2CH_2$-S-S-$CH_2CH_2$O-$CH_2CH_2$-, - $(CH_2CH_2O)_2$-$CH_2CH_2$-S-S-$CH_2CH_2$O-$CH_2CH_2$-, -$CH_2CH_2$O-$CH_2CH_2$-S-S-$(CH_2CH_2O)_2$-$CH_2CH_2$-, or -$(CH_2CH_2O)_2$-$CH_2CH_2$-S-S-$(CH_2CH_2O)_2$-$CH_2CH_2$-.

($Y^a$)

It is preferable that $Y^a$ represents -$CH_2$- or -$CH_2$-$CH_2$-.

($Y^b$)

$Y^b$ represents preferably -$CH_2$-$CH_2$-, -CH($CH_3$)$CH_2$-, 2-butene-1,4-diyl, hepta-2,4-diene-1,6-diyl, or octa-2,4,6-triene-1,8-diyl and more preferably -$CH_2$-$CH_2$- or - CH($CH_3$)$CH_2$-.

Specific examples of the group "-Z-N($R^a$)Y-$X^1$" include -NH-$(CH_2)_2$-NH-CO-cholesteryl, -NH-$(CH_2)_4$-NH-$(CH_2)_3$-NH-$(CH_2)_3$-NH-COO-cholesteryl, -NH-$(CH_2)_3$-NH-$(CH_2)_4$-NH-$(CH_2)_3$-NH-COO-cholesteryl, -NH-$(CH_2)_4$-NH-$(CH_2)_3$-NH-COO-cholesteryl, -NH-$(CH_2)_4$-N(-$(CH_2)_3$-$NH_2$)-COO-cholesteryl, -NH-$(CH_2)_3$-NH-$(CH_2)_4$-N(-$(CH_2)_3$-$NH_2$)-COO-cholesteryl, -NH-$(CH_2)_3$-NH-$(CH_2)_4$-N(-$(CH_2)_3$-NH-$(CH_2)_3$-$NH_2$)-COO-cholesteryl, -NH-$(CH_2)_3$-NH-$(CH_2)_4$-N(-$(CH_2)_3$-$NH_2$)-CO-NH-cholesteryl, -NH-$(CH_2)_3$-NH-$(CH_2)_4$-N(-$(CH_2)_3$-$NH_2$)-CO-cholesteryl, and -NH-$(CH_2)_3$-NH-$(CH_2)_4$-N(-$(CH_2)_3$-$NH_2$)-cholesteryl. As the preferable group "-Z-N($R^a$)Y-$X^1$", $R^a$, $R^b$, and $R^c$ each represent a hydrogen atom, Y represents linear $C_{2-30}$ alkylene or -$(CH_2CH_2O)_m$-$CH_2CH_2$-, $Y^a$ represents linear $C_{1-5}$ alkylene, or $Y^b$ represents linear $C_{2-8}$ alkylene or linear $C_{2-8}$ alkenylene.

[Repeating unit (Ia)]

In General Formula (Ia), X represents preferably -NH-$(CH_2)_2$-NH-COO-cholesteryl, -NH-$(CH_2)_6$-NH-COO-cholesteryl, -NH-$(CH_2)_{12}$-NH-COO-cholesteryl, or - NH-$(CH_2CH_20)_2$-$CH_2CH_2$-NH-COO-cholesteryl and more preferably -NH-$(CH_2)_2$-NH-COO-cholesteryl, -NH-$(CH_2)_6$-NH-COO-cholesteryl, or -NH-$(CH_2CH_2O)_2$-$CH_2CH_2$-NH-COO-cholesteryl.

The hyaluronic acid derivative (A) can further have a repeating unit represented by General Formula (II) (hereinafter, also referred to as "repeating unit (II)") in addition to the repeating unit (I).

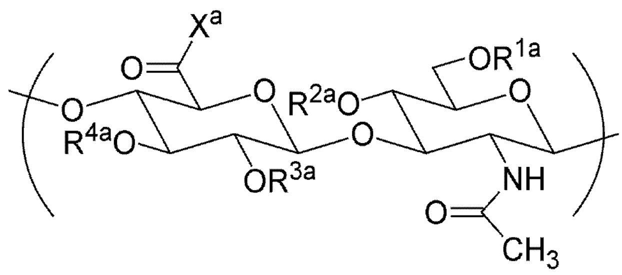

(II)

(In the formula, $R^{1a}$, $R^{2a}$, $R^{3a}$, and $R^{4a}$ each independently represent a group selected from the group consisting of a hydrogen atom, $C_{1-6}$ alkyl, formyl, and $C_{1-6}$ alkylcarbonyl. $X^a$ represents a group selected from the group consisting of hydroxy and -O-$Q^+$. $Q^+$ represents a counter cation.)

Here, in a case where the hyaluronic acid derivative (A) has two or more repeating units (II), the repeating units may be the same as or different from each other.

In another aspect, the hyaluronic acid derivative (A) may be a hyaluronic acid derivative substantially having the repeating unit (I), the repeating unit (Ia), and the repeating unit (II).

[Repeating unit (II)]

In General Formula (II), $Q^+$ is not particularly limited as long as $Q^+$ represents a counter cation that forms a salt with a carboxy group in water, and in a case of being divalent or higher, the counter cation forms a salt with a plurality of carboxy groups according to the valence. Examples of the counter cation include metal ions such as a lithium ion, a sodium ion, a rubidium ion, a cesium ion, a magnesium ion, and a calcium ion; and an ammonium ion represented by Formula: $N^+R^jR^kR^lR^m$ (in the formula, $R^j$, $R^k$, $R^l$, and $R^m$ are each independently selected from the group consisting of a hydrogen atom and $C_{1-6}$ alkyl). Among these, the $Q^+$ represents preferably a sodium ion, a potassium ion, or a tetraalkylammonium ion (for example, a tetra-n-butylammonium ion). $R^j$, $R^k$, $R^l$, and $R^m$ represent preferably the same group selected from the group consisting of $C_{1-6}$ alkyls and preferably an n-butyl group.

It is preferable that all of $R^1$, $R^2$, $R^3$, $R^4$, $R^{1a}$, $R^{2a}$, $R^{3a}$, and $R^{4a}$ represent a hydrogen atom. In addition, it is preferable that both $R^a$ and $R^b$ represent a hydrogen atom.

Among these, the hyaluronic acid derivative (A) is preferably a hyaluronic acid derivative substantially formed of the repeating unit (I) and the repeating unit (II). In the hyaluronic acid derivative (A), for example, 80% or greater, preferably 90% or greater, and more preferably 95% or greater of the repeating units of the disaccharide consisting of D-glucuronic acid and N-acetyl-D-glucosamine contained in the derivative are the repeating unit (I) and the repeating unit (II). The hyaluronic acid derivative (A) may be formed of only the repeating unit (I) and the repeating unit (II).

The amount of the hyaluronic acid derivative (A) is preferably 1 mg/mL or greater and less than 50 mg/mL, more preferably 3 mg/mL or greater and 45 mg/mL or less, and still more preferably 5 mg/mL or greater and 40 mg/mL or less with respect to the total amount of the pharmaceutical composition.

A method of producing the hyaluronic acid derivative (A) will be described below.

<<Association promoter (B)>>

In the present invention, the association promoter can be more specifically measured by gel permeation chromatography, and it can be confirmed by the ratio A2/A1 of the areas A1 and A2 shown below that the association promoter is the association promoter defined in the present embodiment.

FIG. 1 is a chromatogram of the hyaluronic acid derivative. An example of an area A1 shown in FIG. 1 is 1814. FIG. 2 is a chromatogram of the hyaluronic acid derivative pharmaceutical composition 1. An example of an area A2 shown in FIG. 2 is 2813.

FIG. 2 shows a gel permeation chromatogram of the hyaluronic acid derivative of the present embodiment. Here,

the refractive index intensity at the start of measurement is set to zero, and a line drawn horizontally from this point is set as a baseline. For example, the refractive index intensity is adjusted so that the increase or decrease thereof is within a range of ±0.5 mV before the start of the measurement, and is adjusted so that the increase or decrease thereof is within 0.5 mV or less for 5 minutes. For example, the first point at which the amount of an increase in refractive index intensity exceeds the amount corresponding to 5 times the noise value by three or more times is defined as "starting point" of the chromatogram, and the elution time is set to 0 minutes.

For example, a point at which the refractive index intensity is 1/1000 of the maximum refractive index intensity is defined as "end point" of the chromatogram, and in a case where the refractive index intensity does not reach 1/1000 of the maximum refractive index intensity, "Tlim" is defined as "end point". "Tlim" is an elution time at which a maximal refractive index intensity is exhibited in a case where a polyacrylic acid having a molecular weight of 2 kDa is measured.

**In** the present device, the refractive index intensity is calculated every 0.00167 minutes. Here, each area value is calculated using an analysis application of GPC workstation EcoSEC Elite-WS.

In a case of calculating each area value, a peak caused by a developing solvent used in gel permeation chromatography or the like, or a pseudo peak caused by a fluctuation of a baseline due to a column or a device used is excluded.

In a case where the ratio A2/A1 of the area A2 to the area A1 is 1.2 or greater, it means that the association between the hyaluronic acid derivative molecules or the association between the hyaluronic acid derivative and the association promoter is promoted. In a case where the association between the hyaluronic acid derivative molecules or the hyaluronic acid derivative and the association promoter is not promoted, the ratio A2/A1 is in a range of 0.95 to 1.19.

The ratio A2/A1 is preferably 1.20 or greater, more preferably 1.30 or greater, still more preferably 1.40 or greater, and particularly preferably 1.50 or greater. In a case where the ratio A2/A1 of the area A2 to the area A1 is greater than or equal to the above-described lower limits, it is possible to include a relatively large amount of the hyaluronic acid derivative in which the association is promoted, and in a case of formulating the active ingredient (C), it is possible to retain a large amount of the active ingredient (C) and to solubilize the active ingredient (C) at a high concentration. In addition, the association promoter (B) controls the release of the active ingredient (C), and thus the sustained-release period of the drug in vivo can be maintained for a long period of time.

Meanwhile, in the upper limit of A2/A1, the ratio of the area A2 is preferably as higher as possible than the ratio of the area A1. Therefore, the upper limit of A2/A1 is not particularly limited, but may be 4.0 or less, 3.5 or less, 3.0 or less, 2.5 or less, 2.1 or less, 2.0 or less, 1.9 or less, or 1.5 or less.

The association promoter (B) is not particularly limited as long as the association promoter is a surfactant that can be commonly used for pharmaceutical applications.

The association promoter (B) is preferably a component which has at least four or more ether structures (R-O-R) and 4 or more carbon atoms.

Examples of the association promoter (B) include polysorbate (having 5 or more ether structures and 10 or more carbon atoms), polyoxyethylene fatty acid ester, fatty acid sorbitan ester, and polyoxyethylene castor oil.

Examples of the association promoter (B) include polysorbate 80 (having 20 or more ether structures and 64 or more carbon atoms), polysorbate 65 (having 20 or more ether structures and 100 or more carbon atoms), polysorbate 60 (having 20 or more ether structures and 64 or more carbon atoms), polysorbate 40 (having 20 or more ether structures and 62 or more carbon atoms), polysorbate 20 (having 20 or more ether structures and 57 or more carbon atoms), polyethylene glycol monolaurate (having 7 or more ether structures and 28 or more carbon atoms), polyoxyethyl stearate 40 (having 39 or more ether structures and 98 or more carbon atoms), polyoxyethyl stearate 45 (having 44 or more ether structures and 108 or more carbon atoms), polyoxyethyl stearate 55 (having 54 or more ether structures and 128 or more carbon atoms), and CREMOFOL EL (having 35 or more ether structures and 127 or more carbon atoms). Among these, polysorbate 20, polysorbate 80, and polyoxyethylene castor oil are particularly preferable.

The association promoter (B) is not limited to the above-described surfactant, and examples thereof include Poloxamer 188 (having 98 or more ether structures and 225 or more carbon atoms), Poloxamer 124 (having 26 or more ether structures and 74 or more carbon atoms), Poloxamer 237 (having 93 or more ether structures and 225 or more carbon atoms), Poloxamer 338 (having 177 or more ether structures and 400 or more carbon atoms), Poloxamer 407 (having 147 or more ether structures and 352 or more carbon atoms), polyethylene glycol 300 (having 5 or more ether structures and 12 or more carbon atoms), polyethylene glycol 400 (having 7 or more ether structures and 16 or more carbon atoms), polyethylene glycol 4000 (having 59 or more ether structures and 120 or more carbon atoms), and polyvinyl alcohol (average degree of polymerization: 500, having 4 or more ether structures and 4 or more carbon atoms).

The addition amount of the association promoter (B) in the pharmaceutical composition 1 is preferably 0.001 parts by mass or greater and 15,000 parts by mass or less, more preferably 0.05 parts by mass or greater and 5,000 parts by mass or less, and still more preferably 1 part by mass or greater and 4,000 parts by mass or less with respect to 100 parts by mass of the hyaluronic acid derivative.

In a case where the association promoter (B) is at least one selected from the group of surfactants, the addition amount of the association promoter (B) in the pharmaceutical composition 1 is preferably 0.01 parts by mass or greater and

150 parts by mass or less and more preferably 0.05 parts by mass or greater and 100 parts by mass or less with respect to 100 parts by mass of the hyaluronic acid derivative.

In a case where the association promoter (B) is at least one selected from the group other than the surfactant, the addition amount of the association promoter (B) in the pharmaceutical composition 1 is preferably 10 parts by mass or greater and 15,000 parts by mass or less, more preferably 100 parts by mass or greater and 10,000 parts by mass or less, still more preferably 1,000 parts by mass or greater and 5,000 parts by mass or less, particularly preferably 1,500 parts by mass or greater and 4,000 parts by mass or less, and most preferably 2,000 parts by mass or greater and 3,500 parts by mass or less with respect to 100 parts by mass of the hyaluronic acid derivative.

The amount of the association promoter (B) may be as small as possible, but in a case where the amount thereof is less than or equal to the above-described lower limits, the active ingredient cannot be sufficiently solubilized. In addition, in a case where the amount thereof is greater than the upper limit, the toxicity is likely to be high due to an excessive amount of the (B) solubilizing aid, and the viscosity of the preparation is also improved, and thus the above-described ranges are preferable.

The means for dissolving the association promoter (B) is not particularly limited, and examples thereof include stirring means, shaking means, and ultrasonic treatment means.

The pharmaceutical composition 1 according to the present embodiment contains an association promoter (B), and thus a large amount of the active ingredient can be retained. Further, the release rate of the active ingredient can be controlled by preventing the inflow of hydrophobic biological components into the subcutaneously gelated hyaluronic acid derivative composition, and the active ingredient can be released in a sustained manner for a long period of time. In addition, a desired effect may be obtained by a mechanism different from the above-described mechanism.

<<Active ingredient (C)>>

The active ingredient is not particularly limited, and examples thereof include a pharmaceutically active peptide or protein, a nucleic acid, a low-molecular-weight compound, a medium-molecular-weight compound, and an antigen (a cancer antigen, an antigen derived from an infectious disease, an autoantigen in an immune disease, and the like). Among these, a low-molecular-weight compound, a medium-molecular-weight compound, or a peptide is more preferable.

The diseases to which the pharmaceutical composition 1 of the present embodiment is applied are not particularly limited, and the pharmaceutical composition 1 can be widely used for the prevention or treatment of diseases known at present or diseases that will be found in the future. The disease may be either a chronic disease or an acute disease. Examples of diseases known at present include cancer, infectious disease, immune disease, inflammatory laxity, allergic disease, skin disease, hypertension, diabetes, nervous disease, hereditary disease, cardiovascular disease, cerebrovascular disease, respiratory disease, eye disease, ear disease, bone and joint disease, and pain disease.

[Pharmaceutically active peptide or protein]

The pharmaceutically active peptide or protein means a peptide or protein having a positive or favorable effect on the state or medical condition of the subject in a case where a therapeutically effective amount thereof is administered to the subject. The preferred pharmaceutically active peptide or protein is one that has a curative or symptomatic property and can be administered to improve, alleviate, reduce, restore, delay the onset of, or reduce the severity of one or more symptoms of a disease or disorder. The pharmaceutically active peptide or protein may have a preventive property, and can be used to delay the onset of a disease or to reduce the severity of such a disease or a pathological condition. The term "pharmaceutically active peptide or protein" implies a full-length protein or polypeptide, and may also refer to a pharmaceutically active fragment thereof. This term also includes a pharmaceutically active analog of a peptide or a protein.

Examples of the pharmaceutically active protein are not limited to the following, and include cytokines such as immunoactive compounds and immune system proteins (for example, interleukin, colony stimulating factor (CSF), granulocyte colony stimulating factor (G-CSF), granulocyte macrophage colony stimulating factor (GM-CSF), erythropoietin, tumor necrosis factor (TNF), interferon, integrin, adrenosin, selectin, homing receptors, T cell receptors, immunoglobulins, antibodies, hormones (insulin, thyroid hormone, catecholamine, gonadotropin, stimulating hormone, prolactin, oxytocin, dopamine, bovine somatotropin, leptin, and the like), growth hormones (for example, human growth hormone), proliferation factors (for example, epithelial proliferation factor, nerve growth factor, insulin-like growth factor, and the like), proliferation factor receptors, enzymes (tissue plasminogen activator, streptokinase, cholesterol biosynthetic enzyme or decomposition enzyme, steroidogenic enzymes, kinases, phosphodiesterases, methylases, demethylases, dehydrogenases, cellulases, proteases, lipases, phospholipases, aromatases, cytochromes, adenylate cyclases or guanylate cyclases, neuramidases, and the like), receptors (steroid hormone receptors, peptide receptors), binding proteins (growth hormone binding proteins, growth factor binding proteins, and the like), transcription factors and translation factors, tumor growth suppressive proteins (for example, proteins that inhibit angiogenesis), structural proteins

(collagen, fibroin, fibrinogen, elastin, tubulin, actin, myosin, and the like), and blood proteins (thrombin, serum albumin, factor VII, factor VIII, insulin, factor IX, factor X, tissue plasminogen activator, protein C, von Willebrand factor, antithrombin III, glucocerebrosidase, erythropoietin, modified factor VIII, and anticoagulant factors).

[Nucleic acid]

The nucleic acid includes DNA and RNA, and includes, for example, short interfering RNA (siRNA), double-stranded RNA (dsRNA), microRNA (miRNA), short hairpin RNA (shRNA), and a nucleic acid aptamer.

[Low-molecular-weight compound]

The low-molecular-weight compound is a compound having a molecular weight of less than about 500, and examples thereof include an anticancer agent (for example, an alkylating agent, a metabolite antagonist, an alkaloid, and the like), an immunosuppressant, an anti-inflammatory agent (a steroid agent, a non-steroid anti-inflammatory agent, and the like), an anti-rheumatic agent, and an antibacterial agent (a β-lactam antibiotic, an aminoglycoside antibiotic, a macrolide antibiotic, a tetracycline antibiotic, a new quinolone antibiotic, a sulfa drug, and the like).

The active ingredient may be a Rho kinase inhibitor, an endothelin A receptor inhibitor, a transmembrane conductance regulator (CFTR) modulator, a TRPV1 inhibitor, an NK1 receptor inhibitor, a purine receptor inhibitor, an angiotensin receptor inhibitor, a peroxisome proliferator-activated receptor, a P2Y receptor inhibitor, a VEGF inhibitor, or the like, or may be an active ingredient having two inhibitory actions at the same time.

In addition, as the active ingredient, a hydrophobic, that is, poorly water-soluble component can also be preferably used since the interaction with the above-described steryl group of the hyaluronic acid derivative can be sufficiently exhibited. The term "poorly water-soluble" means that an amount of water of 30 mL or greater is required to dissolve 1 g of a solute, according to the 17th revised Japanese Pharmacopoeia.

Examples of the poorly water-soluble and solid active ingredient include antipyretic and analgesic agents such as acetaminophen, ibuprofen, benzoic acid, ethenzamide, caffeine, camphor, quinine, calcium gluconate, dimethylcaprol, sulfamine, theophylline, theopromine, riboflavin, mephenesine, phenobarbital, aminophylline, thioacetazone, quercetin, rutin, salicylic acid, theophylline sodium salt, pyrapital, quinine hydrochloride, irgacure, dikitoxin, griseofulvin, and phenacetin, neurological medicines, sedative-hypnotic drugs, muscle relaxants, antihypertensive agents, antihistamines; antibiotics such as acetylspiramycin, ampicillin, erythromycin, xylatamycin, chloramphenicol, triazetylolerythromycin, nystatin, colistin sulfate; steroid hormones such as methyltestosterone, methylandrosteronedione, progesterone, estradiol benzoate, ethinylestradiol, deoxycorticosterone acetate, cortisone acetate, hydrocortisone, hydrocortisone acetate, and bredonizolone; non-steroidal yolk hormone agents such as dienestrol, hexestrol, diethylstilbestrol, diethylstilbestrol dibrohydionate, and chlorotrianisene; and other pharmaceutical active ingredients such as fat-soluble vitamins described in "Japanese Pharmacopoeia", "Japanese Pharmaceutical Codex", "USP", "NF", and "EP". Such active ingredients may be used alone or a combination of two or more kinds selected from the above-described active ingredients may be used.

The active ingredient may be a poorly water-soluble oily or liquid component. Examples of the poorly water-soluble oily or liquid active ingredients include, for example, vitamins such as teprenone, indomethacin pharnesil, menatetrenone, phytomenadione, vitamin A oil, phenipentol, vitamin D, and vitamin E, highly unsaturated fatty acids such as DHA (docosahexaenoic acid), EPA (eicosapentaenoic acid), and cod liver oil, coenzyme Q, and oil-soluble flavoring agents such as orange oil, lemon oil, and peppermint oil, which are described in the "Japanese Pharmacopoeia", " Japanese Pharmaceutical Codex", "USP", "NF", and "EP". Vitamin E has various homologues and derivatives, and the vitamin E is not particularly limited as long as it is in a liquid state at room temperature. Examples of the vitamin E include dl-α-tocopherol, dl-α-tocopherol acetate, d-α-tocopherol, and d-α-tocopherol acetate. Such active ingredients may be used alone or a combination of two or more kinds selected from the above-described active ingredients may be used.

The active ingredient may be a poorly water-soluble semi-solid active ingredient. Examples of the poorly water-soluble semi-solid active ingredient include a Chinese medicine or a crude drug extract such as earthworm, kanzo, keihi, shakuyaku, buttonpi, kanokosou, sanshou, shoukyou, chinki, maou, nantengitsu, ouhi, onji, kikyou, shazenshi, shazenso, ishibaraso, seneka, baimo, uikyou, oubaku, uren, gajutsu, kamitsure, gentiana, goou, joudan, shazin, shoukyou, soujutsu, chouji, chinki, byakujutsu, chikusetsuninjin, ninjin, kakkontou, keishitou, kousosan, shikokeito, shoushikuto, shoukeiryoutou, bakumondoutou, hanboukutouboku, and maoutou; and oyster meat extract, propolis and propolis extract, and coenzyme Q. Such active ingredients may be used alone or a combination of two or more kinds selected from the above-described active ingredients may be used.

[Medium-molecular-weight compound]

In the present specification, "medium-molecular-weight" refers to a peptide having a molecular weight of about 500 to 5,000, a macrolide compound, a nucleic acid, a natural product, or a derivative thereof, which is neither a low

molecular weight (an organic compound having a molecular weight of about 500) nor a high molecular weight (a protein having a molecular weight of 10,000 or greater or the like). The peptide is preferably a peptide having a molecular weight of about 500 to 2000, that is, a linear or cyclic peptide having about 5 to 20 amino acid residues. The peptide is preferably a cyclic peptide, and the details thereof will be described below. The macrolide compound is a macrocyclic lactone, and is a general term for a compound having 12 or more ring members.

Examples of the medium-molecular-weight compound include FK506 and rapamycin.

[Antigen]

(Cancer antigen)

The cancer antigen is an antigen that is highly expressed in cancer cells, and in some cases, is expressed only by cancer cells. The cancer antigen can be expressed in a cancer cell or on a surface of a cancer cell.

The antigen protein that can be used in the pharmaceutical composition 1 of the present embodiment is not limited, and examples thereof include ERK1, ERK2, WT1, MART-1/Melan-A, gp100, adenosine deaminase binding protein (ADAbp), FAP, cyclophilin b, colorectal cancer antigen (CRC)-C017-1A/GA733, carcinoembryonic antigen (CEA), CAP-1, CAP-2, etv6, AML1, prostate specific antigen (PSA), PSA-1, PSA-2, PSA-3, prostate specific membrane antigen (PSMA), T cell receptor/CD3-zeta chain, CD20, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5, GAGE-1, GAGE-2, GAGE-3, GAGE-4, GAGE-5, GAGE-6, GAGE-7, GAGE-8, GAGE-9, BAGE, RAGE, LAGE-1, NAG, GnT-V, MUM-1, CDK4, tyrosinase, p53, MUC family, HER2/neu, p21ras, RCAS1, α-fetoprotein, E-cadherin, α-catenin, β-catenin, γ-catenin, p120ctn, gp100Pmel117, PRAME, NY-ESO-1, cdc27, adenomatous polyposis coli protein (APC), fodrin, connexin 37, Ig idiotype, p15, gp75, GM2 ganglioside, GD2 ganglioside, human papillomavirus protein, Smad family of tumor antigens, lmp-1, P1A, nuclear antigen (EBNA)-1 encoded by EBV, brain glycogen phosphorylase, SSX-1, SSX-2 (HOM-MEL-40), SSX-1, SSX-4, SSX-5, SCP-1, CT-7, CD20, and c-erbB-2.

As the antigen protein, the entire sequence thereof may be used, or a sequence in which a part thereof is deleted may be used.

The antigen peptide that can be used in the pharmaceutical composition 1 of the present embodiment is an antigen peptide including one or more epitopes selected from the group consisting of a CD8-positive cytotoxic T cell-recognizing epitope and a CD4-positive helper T cell-recognizing epitope, among sequences of antigen proteins. In one embodiment, from the viewpoint that the antigen peptide is loaded on the MHC class I molecule or the MHC class II molecule after undergoing decomposition in an antigen-presenting cell, the antigen peptide is preferably an antigen peptide containing two or more epitopes. Specific examples of the antigen peptide include an antigen peptide including an epitope of an antigen protein of a tumor cell.

In one embodiment, the antigen peptide has, for example, 8 to 120 amino acids, preferably 8 to 80 amino acids, more preferably 15 to 80 amino acids, still more preferably 16 to 80 amino acids, even still more preferably 23 to 80 amino acids, even still more preferably 23 to 60 amino acids, and particularly preferably 23 to 50 amino acids.

In one embodiment, from the viewpoint of inducing activation of cytotoxic T lymphocytes (CTLs) by helper T cells, the antigen peptide is an antigen peptide including one or more CD8-positive cytotoxic T cell recognition epitopes and one or more CD4-positive helper T cell recognition epitopes.

In one embodiment, in a case where the antigen peptide has two or more epitopes, an amino acid linker may be disposed between the epitopes. The linker has, for example, 2 to 10 amino acids, preferably 4 to 10 amino acids, and more preferably 4 to 8 amino acids. Examples of the amino acid used in the linker include glycine (G), tyrosine (Y), leucine (L), and tryptophan (W). Among these, tyrosine (Y), leucine (L), or tryptophan (W) is preferable. Specific examples of the amino acid linker include a linker (4Y) consisting of four consecutive tyrosines (Y), a linker (4L) consisting of four consecutive leucines (L), a linker (4W) consisting of four consecutive tryptophans (W), a linker (6G) consisting of six consecutive glycines (G), a linker (6Y) consisting of six consecutive tyrosines (Y), a linker (6L) consisting of six consecutive leucines (L), a linker (6W) consisting of six consecutive tryptophans (W), a linker (8Y) consisting of eight consecutive tyrosines (Y), a linker (8L) consisting of six consecutive leucines (L), and a linker (8W) consisting of eight consecutive tryptophans (W). Among these, the linker 6Y, the linker 6L, or the linker 6W is preferable.

(Infectious disease-derived antigen)

The infectious disease-derived antigen is not particularly limited as long as the antigen is an infectious pathogen or an antigen derived from an infectious pathogen. Examples of the infectious pathogen include a virus, a bacterium, a fungus, and a nematode. The infectious disease pathogen-derived antigen may be an antigen protein or an antigen peptide.

The disease from which the subject suffers from the infectious pathogen is not particularly limited, and examples thereof include viral diseases such as diseases from viral infections such as adenovirus, herpes virus (for example, HSV-I, HSV-II, CMV, VZV), pox virus (for example, orthopox virus such as smallpox, vaccinia, or molluscum contagiosum), picornavirus (for example, rhinovirus, enterovirus), orthomyxovirus (for example, influenza virus), paramyxovirus (for example, parainfluenza virus, mumps virus, measles virus, respiratory syncytial virus (RSV)), coronavirus (for example, SARS coronavirus (SARS-CoV), MERS coronavirus (MERS-CoV), SARS-CoV-2), papovavirus (for example, papilloma virus causing genital warts, common warts, plantar warts, and the like), hepatitis virus (for example, hepatitis B virus), flavivirus (for example, hepatitis C virus, dengue virus), retrovirus (for example, lentivirus such as HIV); bacterial diseases such as diseases from bacterial infections such as Escherichia, Enterobacter, Salmonella, Staphylococcus, Shigella, Listeria, Aerobacter, Helicobacter, Klebsiella, Proteus, Pseudomonas, Streptococcus, Chlamydia, Mycoplasma, Streptococcus pneumoniae, Neisseria, Clostridium, Bacillus, Corynebacterium, Mycobacterium, Campylobacter, Vibrio, Serratia, Providencia, Chromobacterium, Brucella, Yersinia, Haemophilus, Bordetella; fungal diseases such as fungal diseases including but not limited to Chlamydia, candidiasis, aspergillosis, histoplasmosis, cryptococcal meningitis; and malaria, pneumocystis carinii pneumonia, leishmaniasis, cryptosporidiosis, toxoplasmosis, and trypanosomiasis.

The structure of the antigen that can be used in the pharmaceutical composition 1 according to the present embodiment is not particularly limited as long as the structure is at least a part of various components constituting a pathogen, and examples thereof include a live vaccine, inactivated whole particles, a part thereof, a protein subunit, a protein, and a peptide. Among these, from the viewpoint of the compounding with the hyaluronic acid derivative, a protein subunit, a protein, or a peptide is preferable.

Here, the influenza virus is an RNA enveloped virus belonging to Orthomyxoviridae, having a particle size of about 100 nm, and is classified into types A, B, and C based on the antigenicity of the internal protein. The influenza virus consists of a core of ribonucleic acid (RNA) associated with an internal nucleocapsid or a nuclear protein surrounded by a virus envelope having a lipid bilayer structure, and an external glycoprotein. The inner layer of the viral envelope is mainly formed of matrix proteins, and the outer layer is mostly formed of host-derived lipid substances. In addition, the RNA of the influenza virus has a segmented structure. Influenza that is pandemic worldwide is caused by an A-type influenza virus, and the A-type influenza virus has two types of envelope glycoproteins, hemagglutinin (HA) and neuraminidase (NA), and is classified into 16 subtypes in HA and 9 subtypes in NA according to the difference in antigenicity.

As the infectious disease-derived antigen, an antigen derived from an A-type or B-type influenza virus is suitably used. The subtype of the A type and the B type influenza virus described above is not particularly limited, and may be a subtype that has been isolated so far or a subtype that will be isolated in the future.

**In** addition, the influenza virus-derived antigen is not particularly limited as long as the antigen is at least a part of various components constituting the influenza virus, and examples thereof include all virus particles in which the purified virus particles have been inactivated with an organic solvent/surfactant or other reagents, and virus subunits obtained by removing impurities from all virus particles and purifying HA and/or NA. From the viewpoint of immunogenicity, the HA subunit or the whole virus particle is preferable. It is more preferable that the virus particles are inactivated with formalin or the like. **In** addition, the present invention is particularly effective for an HA subunit (split) in which impurities are small and an adjuvant such as an immunostimulant is essential.

The method of preparing the influenza virus antigen is not particularly limited, and a known method can be used without limitation. Examples thereof include a method of infecting a chicken egg or the like with a virus strain isolated from an influenza-infected animal or an influenza patient, culturing the virus strain by a method of the related art, and preparing an antigen from a purified virus stock solution. In addition, a virus-derived antigen prepared in a cultured cell by genetic engineering may be used.

(Antigen in immune disease)

The antigen in the immune disease is not particularly limited as long as the antigen includes an epitope of a target protein of the immune disease. The immune disease is not particularly limited, and examples thereof include psoriasis vulgaris, ankylosing spondylitis, rheumatoid arthritis, psoriatic arthritis, axial spondyloarthritis, Crohn's disease, ulcerative colitis, bronchial asthma, chronic urticaria, pollen allergy, and atopic dermatitis. The target protein is not particularly limited, and examples thereof include IL-17A, DPP4, S100A9, PCSK9, IL-23, IgE, TNF$\alpha$, IL-12/23p40, IL-6, $\alpha 4\beta 7$ integrin, IL-4/13, IL-5, BLyS, and IL-13. Reference Document 1 (PCT International Publication No. WO2017/164409) describes a peptide derived from IL-17A.

In a case where the active ingredient (C) in the present invention is a poorly water-soluble drug, the present invention can be more effectively used.

The poorly water-soluble drug means a drug classified as slightly easily soluble, slightly poorly soluble, poorly soluble, extremely poorly soluble, or hardly soluble among drugs which are described as extremely easily soluble, easily soluble, slightly easily soluble, slightly poorly soluble, poorly soluble, extremely poorly soluble, or hardly soluble in the terms indicating solubility in the 17th revised Japanese Pharmacopoeia.

Specific examples of the active ingredient (C) include poorly water-soluble drugs having a solubility of 1 mg/mL or less in water.

Even in the case of such a poorly water-soluble drug, the pharmaceutical composition 1 according to the present invention can be solubilized at a high concentration without using an organic solvent while the amount of a highly toxic surfactant to be used is reduced.

The molecular weight of the active ingredient (C) is preferably 200 or greater, more preferably 300 or greater, still more preferably 400 or greater, even still more preferably 500 or greater, even still more preferably 600 or greater, even still more preferably 700 or greater, even still more preferably 800 or greater, even still more preferably 900 or greater, particularly preferably 1000 or greater, particularly preferably 1100 or greater, and most preferably 1200 or greater.

The molecular weight of the active ingredient (C) is a value calculated from the molecular formula of a compound.

The poorly water-soluble drug which is the active ingredient (C) is preferably a poorly water-soluble peptide. The poorly water-soluble peptide may have an acidic amino acid, a basic amino acid, or a neutral amino acid. Among these, a basic amino acid or a neutral amino acid is preferable. In a case of the compounding, the pH of the solution may be appropriately selected in consideration of the isoelectric point. In addition, the amino acid may include a natural amino acid or an unnatural amino acid.

In the poorly water-soluble peptide, it is preferable that at least one nitrogen atom constituting an amide bond has a methyl group. The imparting of hydrophobicity by methylation increases the interaction with the hydrophobic moiety of the hyaluronic acid derivative, and thus more solubilization is expected. In addition, it is presumed that the interaction is enhanced and the stability as a preparation is also improved.

The poorly water-soluble peptide preferably includes at least one or more selected from the group consisting of a cyclic peptide, a long-chain peptide, a hydrophobic group-modified peptide, a membrane-damaging peptide, or a peptide-drug complex.

The poorly water-soluble peptide is preferably a cyclic peptide. Being cyclic and rigid enhances the interaction with the hydrophobic moiety of hyaluronic acid, and forms a stable structure with the hyaluronic acid derivative.

As the cyclic size, a cyclic peptide formed of 4 to 49 amino acids is preferable, a cyclic peptide formed of 6 to 30 amino acids is more preferable, and a cyclic peptide formed of 8 to 25 amino acids is most preferable.

The number of rings contained in the molecule is not particularly limited, but is preferably 1 or greater and 8 or less.

Examples of the hydrophobized peptide include an alkylated polypeptide. "Insulin detemir" sold by Novo Nordisk Pharma Ltd. is an insulin analog designed to bind a C14 fatty acid side chain to lysine at position 29 of the human insulin B chain and to exhibit affinity to albumin. This fatty acid side chain promotes self-association between insulin detemir hexamers and binds to albumin at a subcutaneous injection site, and thus the absorption rate from the administration site is decreased due to the stabilization effect. By using the hyaluronic acid derivative pharmaceutical composition 1 of the present embodiment or the pharmaceutical composition 2 described below, a hydrophobic moiety of a complex of the hyaluronic acid derivative and the association promoter or solubilizing aid in vivo and a hydrophobic moiety of a peptide strongly interact with each other, which makes it possible to extend the sustained-release period.

Furthermore, in the hyaluronic acid derivative pharmaceutical composition 1 including the peptide or the pharmaceutical composition 2 described below, the hyaluronic acid derivative effectively suppresses the decomposition of the peptide by the peptide-decomposing enzyme in vivo, and thus the long-term sustained-release can be expected.

In the present embodiment, the blending amount of the active ingredient (C) is preferably 10 parts by mass or greater and 100 parts by mass or less, more preferably 15 parts by mass or greater and 50 parts by mass or less, and still more preferably 20 parts by mass or greater and 40 parts by mass or less with respect to 100 parts by mass of the hyaluronic acid derivative (A).

The active ingredient (C) is not particularly limited, but the poorly water-soluble drug may be blended alone or a combination of two or more kinds thereof may be used.

In a case where the active ingredient (C), which is a poorly water-soluble drug, is formulated into the pharmaceutical composition 1 according to the present embodiment, the active ingredient (C) can be solubilized without using an organic solvent, and the solubilizing aid having high toxicity in the related art can be reduced. Further, the solubilizing aid (B) can promote the hydrophobic interaction between the steryl groups of the hyaluronic acid derivative (A). Accordingly, it is presumed that the powdery active ingredient (C) can be solubilized at a high concentration. In addition, a desired effect may be obtained by a mechanism different from the above-described mechanism.

That is, the pharmaceutical composition 1 according to the present embodiment can also be referred to as a composition not using an organic solvent or a composition for powder drug solubilization.

<<Other additives>>

The pharmaceutical composition 1 according to the present embodiment can be administered alone or can be administered together with a pharmaceutically acceptable carrier according to means of the related art. In a case of being

used in combination with a pharmaceutically acceptable carrier, for example, the above-described hyaluronic acid derivative and the above-described active ingredient, and as necessary, water or other physiologically acceptable liquids (for example, physiological saline, phosphate buffered physiological saline (PBS)), and the like may be mixed, and a physiologically acceptable buffer solution, an excipient, a vehicle, a preservative, a stabilizer, a binder, a lyophilization adjuvant, and the like may be included.

Examples of the buffer solution include Tris, sodium phosphate, potassium phosphate, histidine, and citric acid.

The hyaluronic acid derivative is used by being dissolved in a pharmaceutically acceptable medium at any concentration. For example, water for injection or a buffer solution such as a phosphate buffer solution is preferable. The buffer is not particularly limited as long as it is a compound having a buffering capacity in a pH range of 4 to 10 of the composition. Examples of the buffer include acetates such as sodium acetate, phosphates such as sodium dihydrogen phosphate, disodium hydrogen phosphate, potassium dihydrogen phosphate, and dipotassium hydrogen phosphate, amino acid salts such as ε-aminocaproic acid and sodium glutamate, boric acid and salts thereof, and mixtures thereof. Among these, water for injection, a phosphate buffer solution, water for injection containing sucrose, a phosphate buffer solution containing sucrose, or glycerol is particularly preferable.

The pharmaceutical composition 1 according to the present embodiment or the pharmaceutical composition 2 described below may contain a pH adjuster, and examples of the pH adjuster include hydrochloric acid, citric acid, phosphoric acid, acetic acid, tartaric acid, sodium hydroxide, potassium hydroxide, sodium carbonate, and sodium hydrogen carbonate.

An acid such as an organic acid or an inorganic acid can also be blended as necessary.

In addition, these pH adjusters may be used alone or a combination of two or more kinds thereof may be used.

The pH of the pharmaceutical composition 1 of the present embodiment or the pharmaceutical composition 2 described below is not particularly limited within a range allowed as a drug, but is, for example, in a range of 4.0 to 9.0, preferably 4.0 to 8.8, and more preferably 6.5 to 8.8.

Examples of the preservative of the pharmaceutical composition 1 of the present embodiment or the pharmaceutical composition 2 described below include benzalkonium chloride, methyl paraben, propyl paraben, chlorobutanol, sorbic acid, and alkyl polyaminoethyl glycine.

One or two or more kinds of preservatives can be further blended as necessary, and there is no particular limitation as long as the preservatives are allowed as a drug.

As the preservative in the present invention, for example, benzalkonium chloride, benzethonium chloride, chlorhexidine gluconate, paraoxybenzoic acid esters such as ethyl paraoxybenzoate, benzyl alcohol, m-cresol, phenol, phenethyl alcohol, sorbic acid or a salt thereof, and thimerosal are used.

One or two or more kinds of thickening agents can be further blended in the pharmaceutical composition 1 of the present embodiment or the pharmaceutical composition 2 described below as necessary, and the thickening agent is not particularly limited as long as the thickening agent is allowed as a drug.

Examples of the thickener in the pharmaceutical composition 1 according to the present invention or the pharmaceutical composition 2 described below include cellulose-based polymers (such as methyl cellulose, ethyl cellulose, hydroxyethyl cellulose, and hydroxypropyl methyl cellulose), vinyl-based polymers (polyvinylpyrrolidone and polyvinyl alcohol), saccharides (such as hyaluronic acid and mucopolysaccharides such as salts thereof, gellan gum, sodium alginate, dextran, and polysaccharides such as cyclodextrin), and oxyalkylene-based polymers (a polyoxyethylene polyoxypropylene block copolymer). The molecular weight of the thickener in the present invention can be selected from, for example, a range of a number average molecular weight of about $0.5 \times 10^4$ to $100 \times 10^4$.

Examples of the stabilizer include a sodium edetate hydrate and polyvinylpyrrolidone (povidone).

The pharmaceutical composition 1 according to the present embodiment or the pharmaceutical composition 2 described below may contain a chelating agent, and examples of the chelating agent include disodium edetate, trisodium edetate, tetrasodium edetate, diethylenetriaminepentaacetic acid, and a mixture thereof, and the chelating agent is used for stabilizing a drug or a preparation.

Examples of the isotonic agent also include sucrose, glucose, dextrose, lactose, mannitol, and a calcium or magnesium compound such as $CaCl_2$.

According to Japanese Patent No. 4758893, it is described that the efficacy of antibacterial and preservative is improved by blending glycerol having a concentration (2% to 2.5% (v/v)) substantially equal to the isotonicity. Therefore, not only the function as an isotonic agent but also antibacterial and preservative effects are expected. The amount of glycerol is, for example, 0.01% to 10% (w/v), preferably 0.05% to 5% (w/v), more preferably 0.1% to 3.0% (w/v), still more preferably 0.3% to 3.0% (w/v), and particularly preferably 0.3% to 2.5% (w/v).

A base can be blended in the pharmaceutical composition 1 according to the present embodiment or the pharmaceutical composition 2 described below as necessary. The base is not particularly limited as long as the base is allowed as a drug, and examples thereof include sodium hydroxide, potassium hydroxide, monoethanolamine, diethanolamine, triethanolamine, trometamol, and meglumine.

An inorganic salt can be blended in the pharmaceutical composition 1 according to the present embodiment or

the pharmaceutical composition 2 described below as necessary. The base is not particularly limited as long as the base is acceptable as a drug, and examples thereof include zinc chloride and zinc acetate.

The pharmaceutical composition 1 according to the present embodiment or the pharmaceutical composition 2 described below may be used in a formulated form. The preparation can be in the form of a solid, a semi-solid, or a liquid.

In a case of a solid, examples thereof include a powder, a granule, a tablet, a pellet, and a capsule. Among these, the solid is preferably a freeze-dried powder.

In a case of a semi-solid, examples thereof include a gel.

In a case of a liquid, examples thereof include a suspension in which a powder is diluted or suspended with water, a phosphate buffer (PB) solution, or a buffer solution such as phosphate buffered saline (PBS).

[Physical properties of pharmaceutical composition]

The pharmaceutical composition of the present embodiment is preferably a composition in which a precipitate is generated at a physiological saline concentration.

Specifically, the pharmaceutical composition is preferably a pharmaceutical composition in which the precipitation rate in vitro is 20%, preferably 50% or greater, more preferably 70% or greater, still more preferably 80% or greater, particularly preferably 90% or greater, and most preferably 92% or greater under the following test conditions.

(Test Conditions)

· Preparation of precipitated sample

200 μL of a concentrated buffer solution (40 mM PB, 600 mM NaCl aqueous solution) is put into a microtube (1.5 mL), and 600 μL of a preparation formed of a hyaluronic acid derivative pharmaceutical composition is put into the microtube. Thereafter, the mixture is mixed with a voltex for 30 seconds and incubated at 37°C for 20 min, and then the precipitate is sedimented by a centrifuge (2,000 G, 5 min). Subsequently, the hyaluronic acid derivative in the supernatant is subjected to GPC measurement.

· Preparation of blank sample

200 μL of water for injection is put into a microtube (1.5 mL), and 600 μL of a preparation formed of the hyaluronic acid derivative pharmaceutical composition (the theoretical concentration of the hyaluronic acid derivative is set to Xm g/mL, X > 1.33) is put into the microtube. Thereafter, the mixture is mixed with a voltex for 30 seconds, incubated at 37°C for 20 minutes, and then subjected to a centrifuge (2,000 G, 5 minutes). Subsequently, the supernatant is diluted with water for injection such that the concentration of the hyaluronic acid derivative in the supernatant is 1 mg/mL, and the diluted solution is subjected to GPC measurement.

· GPC measurement conditions

Device: HLC8420-GPC (manufactured by Tosoh Corporation)
Column: G4000SWXL (manufactured by Tosoh Corporation, particle diameter: 8 μm, inner diameter: 7.8 mm, length: 30 cm, product number: 8542)
Eluent: 10 mM phosphate buffer solution (pH of 7.4)
Flow rate: 1 mL/min
Injection volume: 50 μL
Detector: RI
Temperature: 30°C

· Confirmation of precipitation

In the present specification, it is evaluated that a precipitate occurs in a hyaluronic acid derivative pharmaceutical composition in which a precipitation rate represented by the following equation is 20% or greater at a physiological saline concentration.

Precipitation rate (%) = {1 - (area value of hyaluronic acid derivative of precipitated sample) ÷ ((area value of hyaluronic acid derivative of blank sample) × (0.75X))} × 100

In the above-described test, during the preparation of the precipitated sample, the centrifugation conditions and the incubation time may be optionally set. For example, it is determined that a precipitate is generated at the physiological saline concentration in a case where a precipitate having a precipitation rate of 20% or greater is obtained after being allowed to stand at 37°C for 2 weeks under the above-described conditions.

It is preferable that the pharmaceutical composition 1 according to the present embodiment or the pharmaceutical composition 2 described below does not have precipitates observed visually in an environment at 20°C.

In a case where precipitates are confirmed, the precipitates can be detected by a light-shielding type automatic fine particle measuring device (liquid particle counter "KL-05") used in the insoluble fine particle test method of an injection agent, which is defined in the Japanese Pharmacopoeia, and the first method "light-shielding particle counting method". Drug crystals having a micrometer-order size may be precipitated from a supersaturated solution in which an amorphous drug is completely dissolved. In a case where the precipitate is not present, the particles are not observed with the light-shielding type automatic fine particle measuring device.

In addition, it can also be determined by verifying the presence of particles having a size of 1 μm or greater using a DLS device.

The pharmaceutical composition 1 according to the present embodiment or the pharmaceutical composition 2 described below is preferably filter-sterilizable.

The filtration sterilizability can be measured by the following evaluation of passing liquid using a syringe filter, and specifically, the evaluation is performed by determining whether the liquid can pass through a sterile filtration filter having a pore diameter of 0.45 μm or 0.22 μm. More specifically, in a case where a 13 mmϕ sterilized syringe filter having a pore diameter of 0.45 μm, which is made of polyether sulfone (PES), and having a volume of 3 mL is used as a filtration membrane, the hyaluronic acid derivative that has passed through the filtration membrane of the 2 mL hyaluronic acid derivative composition is capable of liquid-passing in an amount of 50% or greater, preferably 60%, more preferably 70% or greater, still more preferably 80% or greater, and most preferably 90% or greater.

<Hyaluronic acid derivative pharmaceutical composition 2>

The present embodiment is a hyaluronic acid derivative pharmaceutical composition containing the hyaluronic acid derivative (A1) into which a hydrophobic group has been introduced, the solubilizing aid (B1), and the active ingredient (C1). Hereinafter, "hyaluronic acid derivative pharmaceutical composition containing the hyaluronic acid derivative (A1) into which a hydrophobic group has been introduced, the solubilizing aid (B1), and the active ingredient (C1)" may be abbreviated as "pharmaceutical composition 2".

In the pharmaceutical composition 2 of the present embodiment, the average particle diameter of the spherical structure containing the active ingredient-hyaluronic acid derivative complex can be set to 20 nm or greater and 220 nm or less, 20 nm or greater and 150 nm or less, or 30 nm or greater and 100 nm or less. In a case where the average particle diameter is within the above-described numerical ranges, the nanoparticles can exist in a stable structure in vivo and can more easily pass through the lymph nodes. The average particle diameter can be measured by, for example, dynamic light scattering (DLS), a nanotracking particle measurement device, size exclusion chromatography, high performance liquid chromatography, an electron microscope method, or the like. More specifically, for example, the measurement is carried out by diluting the sample with a 10 mM phosphate buffer solution containing 10 mM phosphate buffer solution or 10 w/v% sucrose so that the concentration of the hyaluronic acid derivative is 1 mg/mL, using a DLS device.

The solubilizing aid (B 1) contained in the pharmaceutical composition 2 according to the present embodiment has at least four or more ether structures (R-O-R) and 4 or more carbon atoms.

In the pharmaceutical composition 2 of the present embodiment, the amount of the solubilizing aid (B1) is 0.0001 parts by mass or greater and 15,000 parts by mass or less with respect to 100 parts by mass of the hyaluronic acid derivative (A1) into which a hydrophobic group has been introduced.

The pharmaceutical composition 2 according to the present embodiment is blended with a specific amount of a specific solubilizing aid.

The pharmaceutical composition of the present embodiment containing the solubilizing aid (B1) can interact with the hyaluronic acid derivative (A1) into which a hydrophobic group has been introduced due to the appropriate polarity of the ether structure and the appropriate hydrophobicity due to the alkyl skeleton, and can promote the interaction between the hydrophobic groups in the hyaluronic acid derivative (A1) into which a hydrophobic group has been introduced. In this manner, it is possible to provide a pharmaceutical composition capable of solubilizing a poorly water-soluble active ingredient at a high concentration.

In a case where the amount of the solubilizing aid (B1) is greater than or equal to the above-described lower limits, the active ingredient can be sufficiently solubilized in the pharmaceutical composition.

In a case where the amount of the solubilizing aid (B1) is less than or equal to the above-described upper limits, the viscosity of the pharmaceutical composition is not excessively increased, and the active ingredient can be sufficiently

solubilized in the pharmaceutical composition within a range where a living body is not affected.

<<Hyaluronic acid derivative (A1) into which hydrophobic group has been introduced >>

The description of the hyaluronic acid derivative (A1) which is contained in the pharmaceutical composition 2 and into which a hydrophobic group has been introduced is generally the same as the description of the hyaluronic acid derivative (A) which is contained in the pharmaceutical composition 1 and into which a hydrophobic group has been introduced.

[Introduction rate of steryl group]

The introduction rate of the steryl group to the hyaluronic acid derivative (A1) is preferably 0.1% or greater and less than 50%, more preferably 5% or greater and less than 48%, still more preferably 35% or greater and 47% or less, and particularly preferably 37% or greater and 45% or less.

**In** a case where the introduction rate of the steryl group is within the above-described range, the hyaluronic acid derivative (A1) can strongly interact with the hydrophobic moiety of the poorly water-soluble drug and the surfactant. In addition, in a case where the introduction rate of the steryl group is in the above-described ranges, the stability of the preparation can be improved in the hyaluronic acid derivative-drug complex in which the hyaluronic acid derivative (A1) in the pharmaceutical composition 2 is compounded with the drug.

As an example, the molecular weight of the hyaluronic acid derivative (A1) is preferably 1,000 (1 k) or greater and 1,000,000 (1,000 k) or less, more preferably 5 k or greater and 300 k or less, still more preferably 5 k or greater and 120 k or less, and particularly preferably 7 k or greater and 100 k or less. The molecular weight of the hyaluronic acid derivative (A1) can usually be adjusted by using a raw material having a corresponding molecular weight.

In a case where the weight-average molecular weight of the hyaluronic acid derivative is greater than or equal to the above-described lower limits, the entanglement of molecules can be further enhanced, and the retention properties in blood can be further enhanced. Meanwhile, in a case where the weight-average molecular weight of the hyaluronic acid derivative is less than or equal to the above-described upper limits, an increase in viscosity can be suppressed, and the hyaluronic acid derivative at a higher concentration can be dissolved in the pharmaceutical composition. The weight-average molecular weight of the hyaluronic acid derivative can usually be adjusted by using a raw material having a corresponding molecular weight.

More specifically, from the viewpoint of viscosity and dispersibility, the weight-average molecular weight of the hyaluronic acid derivative is preferably 100 k or less, more preferably 50 k or less, particularly preferably 20 k or less, and most preferably 15 k or less. The molecular weight thereof is in units of Da.

From the viewpoint of producing a hyaluronic acid derivative, the weight-average molecular weight thereof is preferably 4 k to 20 k, particularly preferably 6 k to 16 k, and most preferably 8 k to 12 k.

From the viewpoint that the hyaluronic acid derivative strongly exhibits the properties of hyaluronic acid, for example, the weight-average molecular weight of the hyaluronic acid derivative is preferably 100 k or greater, particularly preferably 200 k or more, and most preferably 300 k or greater in order for the hyaluronic acid derivative to be strongly recognized by the CD44 receptor.

The amount of the hyaluronic acid derivative (A1) is preferably 6 mg/mL or greater and less than 65 mg/mL, more preferably 8 mg/mL or greater and 50 mg/mL or less, and still more preferably 10 mg/mL or greater and 30 mg/mL or less with respect to the total amount of the pharmaceutical composition 2.

<<Solubilizing aid (B 1)>>

In the present invention, "solubilization" denotes that the active ingredient (C1) described below is capable of being dissolved in water until the active ingredient is transparent by visual observation.

The term "solubilizing aid" denotes an agent having an effect of increasing the solubility of the active ingredient (C) in a case where both the active ingredient (C1) and the hyaluronic acid derivative (A1) are added to water.

The solubilizing aid (B 1) is not particularly limited as long as the solubilizing aid has an action of dissolving the active ingredient (C1) in a water phase (solubilization), and the solubilizing aid can be appropriately selected according to the purpose, and examples thereof include a nonionic surfactant. The solubilizing aid (B1) used in the present invention is an agent that is usually used for pharmaceutical applications, and is an agent that has at least four or more ether structures (R-O-R) and 4 or more carbon atoms.

The solubilizing aid (B1) is preferably one or more selected from the group consisting of a nonionic surfactant, polyethylene glycol having a molecular weight of 190 g/moL or greater and 4,000 g/moL or less, and a cyclodextrin derivative.

Examples of the solubilizing aid (B 1) include polysorbate, polyoxyethylene fatty acid ester, fatty acid sorbitan

ester, and polyoxyethylene castor oil.

As the solubilizing aid (B 1), one or more selected from the group consisting of polysorbate 80 (having 20 or more ether structures and having 64 or more carbon atoms), polysorbate 65 (having 20 or more ether structures and having 100 or more carbon atoms), polysorbate 60 (having 20 or more ether structures and having 64 or more carbon atoms), polysorbate 40 (having 20 or more ether structures and having 62 or more carbon atoms), polysorbate 20 (having 20 or more ether structures and having 57 or more carbon atoms), poloxamer (having 28 or more ether structures and having 74 or more carbon atoms), polyoxyethylene hydrogenated castor oil (having 35 or more ether structures and having 57 or more carbon atoms), cyclodextrin derivative (having 12 or more ether structures and having 36 or more carbon atoms), polyethylene glycol 300 (having 5 or more ether structures and having 12 or more carbon atoms), polyethylene glycol 400 (having 7 or more ether structures and having 16 or more carbon atoms), polyethylene glycol 4000 (having 59 or more ether structures and having 120 or more carbon atoms), and tocopheryl polyethylene glycol succinate (having 21 or more ether structures and having 35 or more carbon atoms) are preferable.

In addition to the description, other examples thereof include polyethylene glycol monolaurate (having 7 or more ether structures and having 28 or more carbon atoms), polyoxyethyl stearate 40 (having 39 or more ether structures and having 98 or more carbon atoms), polyoxyethyl stearate 45 (having 44 or more ether structures and having 108 or more carbon atoms), polyoxyethyl stearate 55 (having 54 or more ether structures and having 128 or more carbon atoms), and CREMOFOL EL (having 35 or more ether structures and having 127 or more carbon atoms).

Examples of the solubilizing aid (B 1) include Poloxamer 188 (having 98 or more ether structures and having 225 or more carbon atoms), Poloxamer 124 (having 26 or more ether structures and having 74 or more carbon atoms), Poloxamer 237 (having 93 or more ether structures and having 225 or more carbon atoms), Poloxamer 338 (having 177 or more ether structures and having 400 or more carbon atoms), Poloxamer 407 (having 147 or more ether structures and having 352 or more carbon atoms), polyethylene glycol 300 (having 5 or more ether structures and having 12 or more carbon atoms), polyethylene glycol 400 (having 7 or more ether structures and having 16 or more carbon atoms), polyethylene glycol 4000 (having 59 or more ether structures and having 120 or more carbon atoms), and polyvinyl alcohol (average degree of polymerization: 500, having 4 or more ether structures and having 4 or more carbon atoms).

The amount of the solubilizing aid (B1) is 0.0001 parts by mass or greater and 15,000 parts by mass or less, preferably 0.01 parts by mass or greater and 150 parts by mass or less, more preferably 0.05 parts by mass or greater and 100 parts by mass or less, still more preferably 1 part by mass or greater and 50 parts by mass or less, particularly preferably 5 parts by mass or greater and 30 parts by mass or less, and most preferably 10 parts by mass or greater and 20 parts by mass or less with respect to 100 parts by mass of the hyaluronic acid derivative (A1).

In a case where the solubilizing aid (B1) is a nonionic surfactant, the amount of the nonionic surfactant is preferably 0.0001 parts by mass or greater and 150 parts by mass or less, more preferably 0.001 parts by mass or greater and 100 parts by mass or less, and still more preferably 0.005 parts by mass or greater and 50 parts by mass or less with respect to 100 parts by mass of the hyaluronic acid derivative (A1).

In a case where the solubilizing aid (B1) is a polyethylene glycol having a molecular weight of 190 g/moL or greater and 4,000 g/moL or less, the amount of the polyethylene glycol is preferably 25 parts by mass or greater and 15,000 parts by mass or less, more preferably 250 parts by mass or greater and 10,000 parts by mass or less, and still more preferably 500 parts by mass or greater and 5,000 parts by mass or less with respect to 100 parts by mass of the hyaluronic acid derivative (A1).

The amount of the solubilizing aid (B1) may be as small as possible, but the active ingredient cannot be sufficiently solubilized in a case where the amount thereof is less than or equal to the above-described lower limits. In addition, in a case where the amount thereof is greater than the upper limits, the toxicity is likely to be high due to an excessive amount of the solubilizing aid (B1), and the viscosity of the preparation is also improved. Therefore, it is preferable that the amount thereof is in the above-described ranges.

Means for dissolving the solubilizing aid (B1) is not particularly limited, and examples thereof include stirring means, shaking means, and ultrasonic treatment means.

<<Active ingredient (C1)>>

The description of the active ingredient (C1) contained in the pharmaceutical composition 2 is generally the same as the description of the active ingredient (C) contained in the pharmaceutical composition 1.

In the pharmaceutical composition 2, the amount of the poorly water-soluble drug, which is the active ingredient (C1), to be blended is preferably 21 parts by mass or greater and less than 100 parts by mass, more preferably 22 parts by mass or greater and 70 parts by mass or less, and still more preferably 23 parts by mass or greater and 50 parts by mass or less with respect to 100 parts by mass of the hyaluronic acid derivative (A1).

The active ingredient (C1) is not particularly limited, but the poorly water-soluble drug may be blended alone or a combination of two or more kinds thereof may be used.

In a case where the active ingredient (C1), which is a poorly water-soluble drug, is formulated into the

pharmaceutical composition 2 according to the present embodiment, the active ingredient (C1) can be solubilized without using an organic solvent, and the amount of the solubilizing aid having high toxicity in the related art to be used can be reduced.

Further, the hydrophobic interaction between the steryl groups of the hyaluronic acid derivative (A1) can be promoted by containing the solubilizing aid (B1). In this manner, it is considered that the hydrophobic moiety of the hyaluronic acid derivative pharmaceutical composition is expanded, and the amount of the hydrophobic drug carried is increased. In addition, it is presumed that since the solubilizing aid can function as a hydrophobic moiety region having high mobility in the hyaluronic acid derivative pharmaceutical composition in addition to the hydrophobic moiety that is bound to the hyaluronic acid polymer by a chemical bond, the powdery active ingredient (C1) can be solubilized at a high concentration. In addition, a desired effect may be obtained by a mechanism different from the above-described mechanism.

That is, the pharmaceutical composition 2 according to the present embodiment can also be referred to as a composition not using an organic solvent or a composition for powder drug solubilization.

The amount of the organic solvent is preferably less than 0.8% with respect to the pharmaceutical composition 2.

Here, the organic solvent contained in the pharmaceutical composition 2 is a solvent corresponding to Classes 1 to 3 defined in the Guideline for Residual Solvent in Pharmaceuticals.

Specific examples of the organic solvent of Class 1 include benzene, carbon tetrachloride, 1,2-dichloroethane, 1,1-dichloroethene, and 1,1,1-trichloroethane.

Examples of the organic solvent of Class 2 include acetonitrile, chlorobenzene, chloroform, cyclohexane, 1,2-dichloroethene, dichloromethane, 1,2-dimethoxyethane, N,N-dimethylacetamide, N,N-dimethylformamide, 1,4-dioxane, 2-ethoxyethanol, formamide, hexane, methanol, 2-methoxyethanol, methyl butyl ketone, methylcyclohexane, N-methylpyrrolidone, nitromethane, pyridine, sulfolane, tetralin, toluene, 1,1,2-trichloroethene, and xylene.

Examples of the organic solvent of Class 3 include acetic acid, acetone, anisole, 1-butanol, 2-butanol, n-butyl acetate, t-butyl methyl ether, cumene, dimethyl sulfoxide, ethanol, ethyl acetate, diethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, and tetrahydrofuran.

The organic solvents of Classes 1 to 3 are preferably not included in the pharmaceutical composition 2, but are components that remain inevitably in the production steps of the hyaluronic acid derivative. Even in a case of the organic solvents of Classes 1 to 3, as long as the amount of the organic solvent with respect to the pharmaceutical composition 2 is less than 0.8%, it is within a range where the pharmaceutical composition can be safely used.

<<Other additives>>

The pharmaceutical composition 2 according to the present embodiment can be administered alone or can be administered together with a pharmaceutically acceptable carrier according to means of the related art. In a case of being used in combination with a pharmaceutically acceptable carrier, for example, the above-described hyaluronic acid derivative and the above-described active ingredient, and as necessary, water or other physiologically acceptable liquids (for example, physiological saline, phosphate buffered physiological saline (PBS)), and the like may be mixed, and a physiologically acceptable buffer solution, an excipient, a vehicle, a preservative, a stabilizer, a binder, a lyophilization adjuvant, and the like may be included. The description regarding the additive is the same as the description regarding <<Other additives>> in the pharmaceutical composition 1.

<<Method of producing hyaluronic acid derivative>>

The hyaluronic acid derivative can be obtained, for example, by converting a carboxy group of glucuronic acid into an amide and introducing a steryl group. In addition, the introduction rate of the steryl group can be controlled by adjusting the blending amount of the compound having a steryl group to be reacted with hyaluronic acid or a derivative thereof as a raw material.

Specific examples of the method of converting the carboxy group of glucuronic acid into an amide and introducing a steryl group include a method of exchanging a raw material hyaluronic acid or a derivative thereof, preferably hyaluronic acid or a derivative thereof, which is composed of only the repeating unit (II), with a tetraalkylammonium salt (for example, a tetrabutylammonium (TBA) salt), and reacting the obtained hyaluronic acid salt with an amine in a solvent in the presence of an appropriate condensing agent, the amine being introduced with a steryl group (particularly, a cholesteryl group) represented by the formula: "$HNR^a$-Y-$NR^b$-R, $NHR^a$-Y-$NR^b$-COO-R, $HNR^a$-Y-$NR^b$-COO-R, $HNR^a$-Y-$NR^b$-CO-R, $HNR^a$-Y-$NR^b$-CO-$NR^c$-R , $HNR^a$-Y-COO-R, $HNR^a$-Y-O-COO-R, $HNR^a$-Y-S-R, $HNR^a$-Y-CO-$Y^a$-S-R, $HNR^a$-Y-O-CO-$Y^b$-S-R, $HNR^a$-Y-$NR^b$-CO-$Y^b$-S-R, $HNR^a$-Y-S-S-R, or -Z-$NR^a$-Y-$NR^b$-COO-R (in the formula, $R^a$, $R^b$, $R^c$, Y, $Y^a$, $Y^b$, Z, and R are each defined in the present specification)".

The condensing agent that can be used in the above-described reaction is not particularly limited, and examples

thereof include 4-(4,6-dimethoxy-1,3,5-triazin)-4-methylmorpholium (DMT-MM), N,N'-carbonyl diimidazole (CDI), N,N'-dicyclohexylcarbodiimide (DCC), N-ethoxycarbonyl-2-ethoxy-1,2-dihydroquinoline (EEDQ), 2-benzotriazole-1,1,3,3-tetramethyluronium tetrafluoroborate (TBTU), 3,4-dihydro-3-hydroxy-4-oxo-1,2,3-benzotriazine (HODhbt), benzotriazole-1-oxytris-pyrrolidinyl-phosphonium hexafluorophosphate (PyBOP), benzotriazole-1-yl-oxytris(dimethylamino) phosphonium hexafluorophosphate (BOP), 1-ethyl-3-(3-dimethylaminopropyl)carbodiimide (EDC), and N-hydroxysuccinimide (NHS).

In particular, although not limited, DMT-MM is preferable from the viewpoint that the reaction proceeds with high efficiency even in a mixed solvent of water and an organic solvent. In addition, in a system in which a large number of hydroxy groups coexist by using DMT-MM as a condensing agent, it is possible to carry out the formation of an amide bond by an amino group and a carboxy group with high selectivity while suppressing the formation of an ester bond. By using this condensing agent, for example, it is possible to prevent the alcohol, which is a solvent, from reacting with the carboxy group of the hyaluronic acid moiety, or to prevent the carboxy group and the hydroxy group, which are simultaneously present in the hyaluronic acid moiety, from being bonded to each other in the molecule or between the molecules to form an undesirable crosslink.

Examples of the solvent used in the steryl group introduction reaction include water, DMSO, methanol, ethanol, propanol, butanol, isopropanol, polyhydric alcohol, acetonitrile, DMF, THF, dichloromethane, chloroform, hexane, diethyl ether, ethyl acetate, and a mixed solvent thereof. The polyhydric alcohol may be a dihydric alcohol or a trihydric alcohol. Examples of the dihydric alcohol include ethylene glycol, diethylene glycol, propylene glycol, dipropylene glycol, neopentyl glycol, 1,4-butanediol, and 1,6-hexanediol. Examples of the trihydric alcohol include glycerin and trimethylolpropane.

Alternatively, the hyaluronic acid or a derivative thereof as a raw material may be ion-exchanged with a tetraalkylammonium salt (for example, a tetrabutylammonium (TBA) salt), the hyaluronic acid salt and the spacer moiety may be reacted with each other in a solvent in the presence of an appropriate condensing agent (in this case, a protecting and deprotecting reaction may be carried out as necessary) to convert the carboxy group (-COOH) of the hyaluronic acid or a derivative thereof as a raw material, and then the resulting product may be reacted with an appropriate reagent. Examples of a combination of a group derived from a carboxy group and a reaction reagent are shown below.

-CONR$^a$-Y-NR$^b$H + Hal-R;

-CONR$^a$-Y-NR$^b$H + Hal-COOR;

-CONR$^a$-Y-NR$^b$H + HOCO-R;

-CONR$^a$-Y-NR$^b$H + Hal-CO-R;

-CONR$^a$-Y-NR$^b$-COOH + HNR$^c$-R;

-CONR$^a$-Y-NR$^b$-CO-NR$^c$H + Hal-R;

-CONR$^a$-Y-NR$^b$H + HOCO-NR$^c$-R;

-CONR$^a$-Y-NR$^b$H + Hal-CO-NR°-R;

-CONR$^a$-Y-COOH + HO-R;

-CONR$^a$-Y-OH + Hal-COO-R;

-CONR$^a$-Y-OCOOH + HO-R;

-CONR$^a$-Y-OCOOH + Hal-R;

-CONR$^a$-Y-OCO-Hal + HO-R;

-CONR$^a$-Y-SH + Hal-R;

-CONR$^a$-Y-Hal + HS-R;

-CONR$^a$-Y-CO-Y$^a$-Hal + HS-R;

$-CONR^a-Y-CO-Y^a-SH + Hal-R;$

$-CONR^a-Y-O-CO-CH=CH_2 + HS-R;$

$-CONR^a-Y-NR^b-CO-CH(CH_3)=CH_2 + HS-R;$

$-CONR^a-Y-SH + HS-R;$

$-COZ-OH + HNR^a-Y-NR^b-COO-R;$

$-COZ-NR^a-Y-NR^bH + Hal-COO-R$

(In the formulae, $R^a$, $R^b$, $R^c$, Y, $Y^a$, $Y^b$, and Z are as defined in the present specification, and Hal represents a halogen atom selected from the group consisting of a fluorine atom, a chlorine atom, a bromine atom, and iodine).

Examples of the reaction mode include a dehydrohalogenation reaction, a condensation reaction, a dewater reaction, a nucleophilic addition reaction such as a Michael addition, and an oxidative disulfide formation reaction, which are known reactions, and these reactions can be performed by those skilled in the art by appropriately selecting and finding preferred reaction conditions. In a case where the conjugate or the reactant has a carboxy group, the conjugate or the reactant may react with N-hydroxysuccinimide (hereinafter, also referred to as "NHS") ester.

In addition, a method of preparing a hyaluronic acid derivative in which a spacer having a mercapto group modified with a leaving group is introduced into a terminal by reacting hyaluronic acid of the raw material or a carboxy group of a derivative thereof with 2-aminoethyl 2-pyridyl disulfide, and subjecting the hyaluronic acid derivative to a nucleophilic substitution reaction with thiocollesterol to form a disulfide bond may be used.

Further, a method of preparing a product obtained by introducing a part of the spacer into a carboxy group of hyaluronic acid or a derivative thereof and a product obtained by introducing a part of the spacer into a steryl group, and reacting these products with each other can also be used. Some specific examples have been described above, and further, in a case where -S-S- is inserted into Y, a method of preparing a hyaluronic acid derivative in which a spacer having a mercapto group at a terminal is introduced into a carboxy group of hyaluronic acid and a steryl group in which a spacer having a mercapto group is introduced into a terminal, and oxidatively reacting the hyaluronic acid derivative and the steryl group to form a disulfide bond can also be used. In this case, one mercapto group can react with 2-mercaptopyridine to form a disulfide, and then be substituted with the other mercapto group.

In addition, after the preparation of the hyaluronic acid derivative, other substituents may be further introduced. For example, 0.1% or greater and 99.5% or less and preferably 40% or greater and 65% or less of the carboxy group in the hyaluronic acid derivative which is substantially formed of the repeating unit (I) and the repeating unit (II) is converted into $-CO-X^z$ [where $X^z$ is selected from the group consisting of the following groups: $-NH-(CH_2)_{p1}-O-CO-C(R^{17})=CH_2$; $-NH-(CH_2)_{p1}-O-CO-CH(R^{17})-CH_2-S-CH_2-CH(OH)-CH(OH)-CH_2-SH$; $-NH-(CH_2)_{p1}-SH$; $-NH-(CH_2)_{p1}-NH-CO-C(R^{17})=CH_2$; $-NH-(CH_2)_{p1}-NH-C(=NH)-(CH_2)_3-SH$; $-NH-(CH_2)_{p1}-NH-CO-(CH_2)_r-SH$; $-NH-(CH_2)_{p1}-NH-CO-CH(R^{17})-CH_2-S-CH_2-CH(OH)-CH(OH)-CH_2-SH$; $-NH-(CH_2)_{p1}-NH-CO-CH(NH_2)-CH_2-SH$; $-NH-(CH_2)_{p1}-NH-CO-CH(NH_2)-(CH_2)_2-SH$; $-NH-NH-CO-(CH_2)_4-CO-NH-NH-C(=NH)-(CH_2)_3-SH$; $-NH-(CH_2-CH_2-O)_q-CH_2-CH_2-O-CO-C(R^{17})=CH_2$;$-NH-(CH_2-CH_2-OO)_q-CH_2-CH_2-O-CO-CH(R^{17})-CH_2-S-CH_2-CH(OH)-CH(OH)-CH_2-SH$; $-NH-(CH_2-CH_2-O)_q-CH_2-CH_2-SH$; $-NH-(CH_2-CH_2-O)_q-CH_2-CH_2-NH-CO-C(R^{17})=CH_2$; $-NH-(CH_2-CH_2-O)_q-CH_2-CH_2-NH-C(=NH)-(CH_2)_3-SH$; $-NH-(CH_2-CH_2-O)_q-CH_2-CH_2-NH-CO-(CH_2)_r-SH$; $-NH-(CH_2-CH_2-O)_q-CH_2-CH_2-NH-CO-CH(R^{17})-CH_2-S-CH_2-CH(OH)-CH(OH)-CH_2-SH$; $-NH-(CH_2-CH_2-O)_q-CH_2-CH_2-NH-CO-CH(NH_2)-CH_2-SH$; $-NH-(CH_2-CH_2-O)_q-CH_2-CH_2-NH-CO-CH(NH_2)-(CH_2)_2-SH$; $-NH-CH(CO_2H)-(CH_2)-SH$; $-NH-CH(CO_2H)-(CH_2)_2-SH$; and $-NH-CH(CO_2H)-(CH_2)_2-CONH-CH(CONH-CH_2-CO_2H)-CH_2-SH$ (here, $R^{17}$ represents a hydrogen atom or a $C_{1-6}$ alkyl group, p1 represents an integer of 2 or greater and 10 or less, q represents an integer of 1 or greater and 200 or less, and r represents an integer of 1 or greater and 3 or less) to carry out chemical crosslinking within a molecule or between molecules including other molecules for gelation.

The obtained hyaluronic acid derivative may be dried. Examples of a drying method include air drying, drying in a constant-temperature tank, drying under reduced pressure, hot air circulation drying, and freeze drying. Among these, freeze-drying is preferable. In a case of performing freeze-drying, it is preferable that the hyaluronic acid derivative further contains a cryoprotective agent from the viewpoint of more effectively suppressing an increase in particle diameter of the fine particles formed by the hyaluronic acid derivative.

The cryoprotective agent is not particularly limited as long as the agent is known as "cryoprotective agent" or "freeze-drying protective agent", and examples thereof include disaccharides, sorbitol, dextran, propylene glycol, glycerin, glycerol, polyvinylpyrrolidone, and dimethyl sulfoxide.

The disaccharides are not particularly limited, and examples thereof include sucrose, lactulose, lactose, maltose, trehalose, cellobiose, kojibiose, nigerose, isomaltose, isotrehalose, neotrehalose, sophorose, laminaribiose, gentiobiose,

turanose, maltulose, palatinose, gentiobiose, manno-biose, mellibiose, mellibiulose, neolactose, galactosucrose, scillabiose, neohesperidose, lutinose, lutinulose, vicianose, xylobiose, and primeverose. Among these, sucrose, trehalose, maltose, or lactose is preferable from the viewpoint that these are widely used as a cryoprotective agent. **In** addition, from the viewpoint of the use record as a pharmaceutical additive and more effectively suppressing an increase in the particle diameter of the fine particles formed by the hyaluronic acid derivative during freeze-drying, sucrose is more preferable.
The cryoprotective agent may be added in a solid state or in a state of being dissolved in a solvent such as water.
The amount of the cryoprotective agent to be added is not particularly limited, but is preferably 20 parts by mass or greater with respect to 100 parts by mass of the hyaluronic acid derivative. In a case where the amount of the cryoprotective agent to be added is greater than or equal to the above-described lower limit, an effect of suppressing an increase in particle diameter can be more sufficiently obtained. Meanwhile, the upper limit of the amount of the cryoprotective agent to be added is not particularly limited, but can be, for example, 100,000 parts by mass.
A device used for freeze-drying is not particularly limited, and for example, a commercially available freeze-drying machine can be used. Among these, from the viewpoint of controlling the degree of vacuum, a freeze dryer capable of monitoring the degree of vacuum in the device during freeze-drying is preferable, and from the viewpoint of controlling the product temperature, a shelf type freeze dryer is preferable.

<Method of producing pharmaceutical composition 1>

The pharmaceutical composition 1 of the present embodiment can be produced by the following production method 1 or production method 2 for the pharmaceutical composition 1.
Hereinafter, each production method will be described.

<<Method 1 of producing pharmaceutical composition 1>>

The production method 1 is a method of producing the pharmaceutical composition 1 containing the hyaluronic acid derivative (A) into which a hydrophobic group has been introduced, the association promoter (B), and the active ingredient (C).
The production method 1 includes a step of mixing the hyaluronic acid derivative (A) into which a hydrophobic group has been introduced with the association promoter (B) to obtain an association promoter-containing hyaluronic acid derivative aqueous solution, and a mixing step of mixing the active ingredient (C) with the association promoter-containing hyaluronic acid derivative aqueous solution.

<<Method 2 of producing pharmaceutical composition 1>>

The production method 2 is a method of producing the pharmaceutical composition 1 containing the hyaluronic acid derivative (A) into which a hydrophobic group has been introduced, the association promoter (B), and the active ingredient (C).
The production method 2 includes a step of dispersing the active ingredient (C) in the association promoter (B) to obtain a dispersion liquid (I), a step of preparing a hyaluronic acid derivative aqueous solution or an association promoter-containing hyaluronic acid aqueous solution to obtain an aqueous solution (II), and a step of mixing the dispersion liquid (I) with the aqueous solution (II).
In the production methods 1 and 2, depending on the structure of the drug, the drug may be produced using an appropriate pH or a buffer material as appropriate.
It is preferable that the production method 1 and the production method 2 do not include a step of removing the organic solvent.

<Method of producing pharmaceutical composition 2>

The pharmaceutical composition 2 according to the present embodiment can be produced by the following production method 1 or production method 2 for a pharmaceutical composition 2.
Hereinafter, each production method will be described.

<<Method 1 of producing pharmaceutical composition 2>>

The production method 1 for the pharmaceutical composition 2 is a method of producing the pharmaceutical composition 2 containing the hyaluronic acid derivative (A1) into which a hydrophobic group has been introduced, the solubilizing aid (B1), and the active ingredient (C1).
The production method 1 for the pharmaceutical composition 2 includes a step of mixing the hyaluronic acid

derivative (A1) into which a hydrophobic group has been introduced with the solubilizing aid (B1) to obtain a solubilizing aid-containing hyaluronic acid derivative aqueous solution, and a mixing step of mixing the active ingredient (C1) with the solubilizing aid-containing hyaluronic acid derivative aqueous solution.

<<Method 2 of producing pharmaceutical composition 2>>

The production method 2 for the pharmaceutical composition 2 is a method of producing the pharmaceutical composition 2 containing the hyaluronic acid derivative (A1) into which a hydrophobic group has been introduced, the solubilizing aid (B1), and the active ingredient (C1).

The production method 2 for the pharmaceutical composition 2 includes a step of dispersing the active ingredient (C1) in the solubilizing aid (B1) to obtain a dispersion liquid (I), a step of preparing a hyaluronic acid derivative aqueous solution or a solubilizing aid-containing hyaluronic acid aqueous solution to obtain an aqueous solution (II), and a step of mixing the dispersion liquid (I) with the aqueous solution (II).

In the production method 1 and the production method 2 for the pharmaceutical composition 2, it is preferable that the method does not include a step of removing the organic solvent.

<Administration method>

The target to which the pharmaceutical composition 1 or the pharmaceutical composition 2 of the present embodiment is administered includes animals (monkeys, marmosets, mice, rats, cows, horses, cats, dogs, pigs, sheep, goats, rabbits, and the like) classified as mammals including humans.

The route of administration of the pharmaceutical composition 1 or the pharmaceutical composition 2 of the present embodiment is not particularly limited, and can be appropriately used for a known route of administration at present depending on the intended use, the position of the tissue to be treated, and the like.

Examples of the administration route include subcutaneous administration, intramuscular administration, intravenous administration, intraarterial administration, intrathecal administration, intracerebral administration, intraarticular administration, intraperitoneal administration, intravaginal administration, intravesicular administration, intrarectal administration, intravitreal administration, periorbital administration, intracutaneous administration, intraperitoneal administration, intranasal administration, intrabronchial administration, intrapulmonary administration, transdermal administration, sublingual administration, oral administration, buccal administration, and instillation administration. Among these, subcutaneous administration, intramuscular administration, intravitreal administration, intrathecal administration, and intraarticular administration are preferable.

In addition, the pharmaceutical composition may be used as a powder, a cream, an ointment, or an eye drop for local administration.

Examples of the administration by injection include subcutaneous injection, intramuscular injection, intravenous injection, intra-arterial injection, intrathecal injection, intraarticular injection, intraperitoneal injection, intravitreal injection, periorbital injection, intradermal injection, and intratumoral injection.

In the eye drop, in a case where the hyaluronic acid derivative is used, it is possible to reduce the initial burst of the drug, to suppress aggregation that may occur between drugs during the storage of the preparation and after the administration of the drug, to reduce the variation in drug efficacy, to allow the drug to be sustained-released for a longer period of time by precipitating under physiological conditions and adhering to the corneal epithelium, and to reduce the number of times of eye drop. Further, it is also considered that the uptake of a drug by corneal epithelial cells and the uptake of a drug by conjunctival epithelial cells are promoted. In addition, it is possible to provide a preparation having high moisturizing properties, viscosity, and biocompatibility derived from hyaluronic acid, which can alleviate inflammation, pain, stress, damage, and the like during contact with the preparation, and has high safety. As described above, the hyaluronic acid derivative pharmaceutical composition 1 or the pharmaceutical composition 2, which is excellent in the effect of stabilizing the tear film, the effect of treating corneal epithelial injury, the effect of treating meibomian gland dysfunction, and the effect of suppressing pain, is provided by efficiently bringing out the effect of the drug.

In the pharmaceutical composition 1 or the pharmaceutical composition 2 according to the present embodiment, in a case of being administered parenterally, the dose can be appropriately selected in consideration of the kind of the administration target (including age, gender, and the like), but generally, for example, in a case of a human (assuming a body weight of 60 kg), the amount of the active ingredient (preferably, a protein, a peptide, or a low-molecular-weight compound) per administration can be set to 0.01 μg or greater and 20 mg or less, 0.1 μg or greater and 15 mg or less, or 1 μg or greater and 10 mg or less.

The number of times of administration may be a single administration of the above-described dose or may be a plurality of times of administration of the above-described dose, for example, twice or more times, once a day, once every 2 days, once every 4 days, once a week, once every 2 weeks, once every 3 weeks, once every 4 weeks, once a month, once every 2 months, once every 3 months, or once every half a year. Alternatively, two or more sites may be administered in one

administration.

<<Other embodiments>>

In one embodiment, the present invention provides a method of preventing or treating one or more diseases selected from the group consisting of cancer, infectious disease, immune disease, and chronic disease, the method including administering an effective amount of the pharmaceutical composition 1 or the pharmaceutical composition 2 to a patient or a patient animal.

Examples of the infectious disease include those described in the section of "Infectious disease-derived antigen" in "Antigen" above.

In addition, the term "effective amount" as used herein includes an amount that is effective for prevention or treatment, that is, an amount suitable for preventing or treating the above-described disease.

In one embodiment, the present invention provides a composition for prevention or treatment of one or more diseases selected from the group consisting of cancer, infectious disease, immune disease, inflammatory relaxation, allergic disease, skin disease, hypertension, diabetes, nervous disease, hereditary disease, cardiovascular disease, cerebrovascular disease, respiratory disease, eye disease, ear disease, and bone joint disease, and the composition contains the active ingredient-hyaluronic acid derivative complex.

In one embodiment, the present invention provides the use of the above-described active ingredient-hyaluronic acid derivative complex for producing the pharmaceutical composition 1 or the pharmaceutical composition 2.

Examples

Hereinafter, the present invention will be described in detail with reference to examples, but the present invention is not intended to be limited to the examples.

<Synthesis of hyaluronic acid derivative>

[Synthesis Example 1]

A hyaluronic acid derivative was prepared according to the following step 1-A, step 2-A, and step 3-A.

[Step 1-A]

(Synthesis of cholesteryl 6-aminohexylcarbamate hydrochloride)

A cholesteryl 6-aminohexylcarbamate hydrochloride (Chol hydrochloride) was synthesized by the following step 1-1-A and step 1-2-A in this order.

[Step 1-1-A]

Triethylamine (TEA, 1.05 mL) was added to a solution of cholesteryl chloroformate (3.37 g, 7.5 mmol) in anhydrous dichloromethane (20 mL) under an argon atmosphere, and the mixture was stirred. Under ice cooling, 6-(t-butoxycarbonyl)amino-1-aminohexane (1.12 mL, 5 mmol) was added dropwise thereto, and the mixture was stirred under ice cooling for 30 minutes, heated to room temperature (about 25°C), and stirred overnight. The reaction mixture was washed with ultrapure water and saturated saline, dried over anhydrous magnesium sulfate, and then the solvent was distilled off under reduced pressure. The obtained residue was purified by silica gel column chromatography (eluent:ethyl acetate:n-hexane = 1:4), and the fractions of the target product were combined and the solvent was distilled off under reduced pressure.

[Step 1-2-A]

The obtained residue was dissolved in ethyl acetate (40 mL), and a 4N hydrochloric acid/ethyl acetate solution (40 mL) was added thereto, followed by stirring the solution at room temperature (about 25°C) overnight. The resulting precipitate was collected by centrifugation. The obtained solid was washed four times with ethyl acetate and then dried under reduced pressure, thereby obtaining 1.2 g of cholesteryl 6-aminohexylcarbamate hydrochloride (Chol hydrochloride).

[Step 2-A]

(Preparation of tetrabutylammonium (TBA) salt of hyaluronic acid)

A TBA salt (HA-TBA) of hyaluronic acid was prepared by the following step 2-1-A and step 2-2-A in this order.

[Step 2-1-A]

DOWEX (registered trade name) 50WX-8-400 (manufactured by Sigma-Aldrich Co. LLC) was suspended in ultrapure water, and the resin was washed with ultrapure water about three times by decantation. A 40 mass% tetrabutylammonium hydroxide aqueous solution (TBA-OH) (manufactured by Sigma-Aldrich Co., LLC) was added in an amount of about 1.5 times the molar equivalent amount with respect to the cation exchange capacity of the resin, and the mixture was stirred for 30 minutes. The excess TBA-OH solution was removed by decantation, and the resultant was further washed with an excess of ultrapure water, thereby obtaining a TBA-chlorinated cation exchange resin.

[Step 2-2-A]

A raw material sodium hyaluronate (HA-Na) having a molecular weight of 35,000 (35 kDa) was dissolved in ultrapure water at a concentration of 15 mg/mL. A suspension of the TBA-chlorinated cation exchange resin in [Step 2-1-A] was added in an amount of 5 times the molar equivalent amount of the HA unit (unit molecular weight: 401.3) in terms of the ion exchange capacity of the resin. The solution was stirred for 15 minutes and filtered through a filter having a pore size of 0.45 μm, and the filtrate was freeze-dried, thereby obtaining a TBA salt (HA-TBA) of hyaluronic acid as a white solid.

[Step 3-A]

An anhydrous DMSO solution (10 mg/mL) of the HA-TBA prepared in the [Step 2-2-A] was prepared. Thereafter, the amount of the Chol hydrochloride added was adjusted to 19/100 in terms of molar ratio with respect to the disaccharide repeating unit (HA unit) present in the HA-TBA synthesized in the [Step 1-A]. Next, 4-(4,6-dimethoxy-1,3,5-triazin-2-yl)-4-methylmorpholinium chloride (DMT-MM) was added to the HA unit at a proportion set such that the addition amount thereof reached 24/100 in terms of molar ratio, and the mixture was stirred at room temperature (about 25°C) overnight. The reaction solution was dialyzed (Spectra/Por 7, molecular weight cut-off (MWCO): 3,500) in order of a 0.3 M ammonia acetate/DMSO solution, a 0.15 M NaCl aqueous solution, and ultrapure water. The obtained dialysis solution was freeze-dried to obtain a target substance (HA-$C_6$-Chol) as a white solid.

In the $^1$H-NMR spectrum of the product, a peak derived from an acetyl group of N-acetyl-D-glucosamine ($COCH_3$, 1.6 ppm or greater and 2.0 ppm or less, 3H) and a peak derived from a methyl group in a cholesteryl group ($CH_3$, 0.7 ppm, 3H) were confirmed, and the cholesterol introduction rate was 19%.

[Synthesis Example 2]

A hyaluronic acid derivative was prepared by the following step 1-B, step 2-B, and step 3-B.

[Step 1-B]

(Synthesis of cholesteryl 6-aminohexylcarbamate hydrochloride)

A cholesteryl 6-aminohexylcarbamate hydrochloride (Chol hydrochloride) was synthesized by the following step 1-1-B and step 1-2-B.

[Step 1-1-B]

Triethylamine (TEA, 1.05 mL) was added to a solution of cholesteryl chloroformate (3.37 g, 7.5 mmol) in anhydrous dichloromethane (20 mL) under an argon atmosphere, and the mixture was stirred. Under ice cooling, 6-(t-butoxycarbonyl)amino-1-aminohexane (1.12 mL, 5 mmol) was added dropwise thereto, and the mixture was stirred under ice cooling for 30 minutes, heated to room temperature (about 25°C), and stirred overnight. The reaction mixture was washed with ultrapure water and saturated saline, dried over anhydrous magnesium sulfate, and then the solvent was distilled off under reduced pressure. The obtained residue was purified by silica gel column chromatography (eluent:ethyl acetate:n-hexane = 1:4), and the fractions of the target product were combined and the solvent was distilled off under reduced pressure.

[Step 1-2-B]

The obtained residue was dissolved in ethyl acetate (40 mL), and a 4N hydrochloric acid/ethyl acetate solution (40 mL) was added thereto, followed by stirring the solution at room temperature (about 25°C) overnight. The resulting precipitate was collected by centrifugation. The obtained solid was washed four times with ethyl acetate and then dried under reduced pressure, thereby obtaining 1.2 g of cholesteryl 6-aminohexylcarbamate hydrochloride (Chol hydrochloride).

[Step 2-B]

(Preparation of tetrabutylammonium (TBA) salt of hyaluronic acid)

A TBA (HA-TBA) salt of hyaluronic acid was prepared according to the following step 2-1-B and then the following step 2-2-B.

[Step 2-1-B]

DOWEX (registered trade name) 50WX-8-400 (manufactured by Sigma-Aldrich Co. LLC) was suspended in ultrapure water, and the resin was washed with ultrapure water about three times by decantation. A 40 mass% tetrabutylammonium hydroxide aqueous solution (TBA-OH) (manufactured by Sigma-Aldrich Co., LLC) was added in an amount of about 1.5 times the molar equivalent amount with respect to the cation exchange capacity of the resin, and the mixture was stirred for 30 minutes. The excess TBA-OH solution was removed by decantation, and the resultant was further washed with an excess of ultrapure water, thereby obtaining a TBA-chlorinated cation exchange resin.

[Step 2-2-B]

A raw material sodium hyaluronate (HA-Na) having a molecular weight of 35,000 (35 kDa) was dissolved in ultrapure water at a concentration of 15 mg/mL. A suspension of the TBA chlorinated cation exchange resin in "(1) Step 2-1-B" was added in an amount of 5 times the molar equivalent amount of the HA unit (unit molecular weight: 401.3) in terms of the ion exchange capacity of the resin. The solution was stirred for 15 minutes and filtered through a filter having a pore size of 0.45 μm, and the filtrate was freeze-dried, thereby obtaining a TBA salt (HA-TBA) of hyaluronic acid as a white solid.

[Step 3-B]

A solution of anhydrous DMSO (10 mg/mL) of HA-TBA prepared in [step 2-2-B] was prepared. Thereafter, the amount of the Chol hydrochloride added was adjusted to 31/100 in terms of molar ratio with respect to the disaccharide repeating unit (HA unit) present in the HA-TBA synthesized in [Step 1-B].

Next, 4-(4,6-dimethoxy-1,3,5-triazin-2-yl)-4-methylmorpholinium chloride (DMT-MM) was added to the HA unit at a proportion set such that the addition amount thereof reached 37/100 in terms of molar ratio, and the mixture was stirred overnight at room temperature (about 25°C). The reaction solution was dialyzed (Spectra/Por 7, molecular weight cut-off (MWCO): 3,500) in order of a 0.3 M ammonia acetate/DMSO solution, a 0.15 M NaCl aqueous solution, and ultrapure water.

The obtained dialysis solution was freeze-dried to obtain a target substance (HA-$C_6$-Chol) as a white solid. In the $^1$H-NMR spectrum of the product, a peak derived from an acetyl group of N-acetyl-D-glucosamine ($COCH_3$, 1.6 ppm or more and 2.0 ppm or less, 3H) and a peak derived from a methyl group in a cholesteryl group ($CH_3$, 0.7 ppm, 3H) were confirmed, and the cholesterol introduction rate was 30%.

<Test Example 1>

As Test Example 1, the formulation of cyclosporin (CyA), which is a poorly water-soluble peptide, was carried out.

[Example 1-1]

A preparation of CyA was prepared using a hyaluronic acid derivative (35 k HA-C6-Chol-19%) containing an association promoter (Polysorbate 80). The following operation was performed in an environment at 20°C and room temperature.

The hyaluronic acid derivative (35 k HA-C6-Chol-19%) of the freeze-dried product obtained in Synthesis

Example 1 was dissolved in water for injection by being stirred at 20°C and 12.0 mg/mL for 24 hours. Polysorbate 80 (FUJIFILM Wako Pure Chemical Corporation, product number: 164-21591) was diluted with water for injection at a proportion of 2 mg/mL in another vial.

In addition, 200.0 mg of powder CyA (manufactured by Tokyo Chemical Industry Co., Ltd., product number: C2408) was weighed in another vial, and then 1.0 mL of ethanol was added thereto to uniformly dissolve CyA. Thereafter, 7.2 mL of a 12 mg/mL hyaluronic acid derivative aqueous solution and 1.2 mL of 2 mg/mL Polysorbate 80 were added and mixed, and stirred for 1 hour, 0.046 mL of a 200 mg/mL CyA-containing ethanol solution was added dropwise to the Polysorbate 80-containing hyaluronic acid derivative aqueous solution to carry out compounding of the drug.

Further, the entire CyA was compounded, the concentration was adjusted with water for injection, and then sucrose (manufactured by FUJIFILM Wako Pure Chemical Corporation, for manufacturing only) was added in the form of powder at a proportion of 10% by mass of the sucrose solution, and the mixture was stirred for 3 hours to be homogenized, thereby preparing an association promoter-containing hyaluronic acid derivative pharmaceutical composition. The final solution was visually clear. In addition, no precipitate was observed during refrigerated storage for 2 weeks.

The above-described operation was performed at 20°C.

The final preparation composition is listed in Table 1.

[Example 1-2]

A CyA preparation was prepared using a hyaluronic acid derivative (35 k HA-C6-Chol-19%) containing an association promoter (polyethylene glycol 400).

The following operation was performed in an environment at 20°C and room temperature.

The hyaluronic acid derivative (35 k HA-C6-Chol-19%) of the freeze-dried product obtained in Synthesis Example 1 was dissolved in water for injection by being stirred at 20°C and 12.0 mg/mL for 24 hours. 88.0 mg of powder CyA (Tokyo Chemical Industry Co., Ltd., product number: C2408) was weighed in another vial, and then 10.0 mL of polyethylene glycol 400 (FUJIFILM Wako Pure Chemical Corporation, product number: 161-09065) was added thereto to uniformly dissolve CyA.

Subsequently, 2.0 mL of an 8.8 mg/mL CyA-containing polyethylene glycol 400 solution was added to a vial containing 6 mL of a 12 mg/mL hyaluronic acid derivative aqueous solution while the solution was stirred, and the drug was compounded. Further, the entire CyA was compounded, sucrose (manufactured by FUJIFILM Wako Pure Chemical Corporation, for manufacturing only) in the form of powder was added at a proportion set such that a 10% by mass sucrose solution was obtained, and the mixture was stirred for 3 hours to be homogenized, thereby preparing an association promoter-containing hyaluronic acid derivative pharmaceutical composition. Finally, the solution was visually clear. In addition, no precipitate was observed during refrigerated storage for 2 weeks. The final preparation composition is listed in Table 1.

[Example 1-3]

A CyA preparation was prepared using a hyaluronic acid derivative (35 k HA-C6-Chol-19%) containing an association promoter (polyethylene glycol 400 and polysorbate 80). The following operation was performed in an environment at 20°C and room temperature.

The hyaluronic acid derivative (35 k HA-C6-Chol-19%) of the freeze-dried product obtained in Synthesis Example 1 was dissolved in water for injection by being stirred at 20°C and 12.0 mg/mL for 24 hours. 92.6 mg of powder CyA (Tokyo Chemical Industry Co., Ltd., product number: C2408) was weighed in another vial, and then 10.0 mL of polyethylene glycol 400 (FUJIFILM Wako Pure Chemical Corporation, product number: 161-09065) was added thereto to uniformly dissolve CyA. Subsequently, 1.9 mL of a 9.26 mg/mL CyA-containing polyethylene glycol 400 solution was added to a vial containing 6 mL of a 12 mg/mL hyaluronic acid derivative aqueous solution while the solution was stirred, and the drug was compounded.

In addition, the entire CyA was further compounded, and 0.10 mL of polysorbate 80 at 100 mg/mL was added. Sucrose (manufactured by FUJIFILM Wako Pure Chemical Corporation, for manufacturing only) in the form of powder was added at a proportion set such that a 10% by mass sucrose solution was obtained, and the mixture was stirred for 3 hours to be homogenized, thereby preparing an association promoter-containing hyaluronic acid derivative pharmaceutical composition. Finally, the solution was visually clear. In addition, no precipitate was observed during refrigerated storage for 2 weeks. The final preparation composition is listed in Table 1.

[Comparative Example 1-1]

a CyA preparation was prepared using $\alpha$-cyclodextrin.

The following operation was performed in an environment at 20°C and room temperature.

The powder of CyA was dissolved in a 10 mass% sucrose solution containing α-cyclodextrin (manufactured by Sigma-Aldrich Co., LLC) at a concentration of 10% by mass at a proportion of 0.5 mg/mL, and the solution was adjusted. Finally, the solution was visually clear. In addition, no precipitate was observed during refrigerated storage for 2 weeks. The final preparation composition is listed in Table 1.

[Comparative Example 1-2]

A preparation of CyA was prepared using a hyaluronic acid derivative (35 k HA-C6-Chol-19%).
The following operation was performed in an environment at 20°C and room temperature.
The hyaluronic acid derivative (35 k HA-C6-Chol-19%) of the freeze-dried product obtained in Synthesis Example 1 was dissolved in water for injection by being stirred at 20°C and 12.0 mg/mL for 24 hours.
In addition, 200.0 mg of powder of CyA (Tokyo Chemical Industry Co., Ltd., product number: C2408) was weighed in still another vial, and 1.0 mL of ethanol was added thereto to uniformly dissolve CyA. Thereafter, 0.046 mL of the 200 mg/mL CyA-containing ethanol solution was added dropwise to a 12 mg/mL hyaluronic acid derivative aqueous solution to compound the drug. Further, the entire CyA was compounded, the concentration was adjusted with water for injection, and then sucrose (manufactured by FUJIFILM Wako Pure Chemical Corporation, for manufacturing only) in the form of powder was added at a proportion set such that a 10% by mass sucrose solution was obtained, and the mixture was stirred for 3 hours to be homogenized. Finally, the solution was visually clear.
The above-described operation was performed at 20°C.
The final preparation composition is listed in Table 1.

[Table 1]

| | Item | Name of ingredient | Concentration in preparation [mg/mL] | Parts by mass with respect to 100 parts by mass of hyaluronic acid derivative |
|---|---|---|---|---|
| Example 1-1 | Hyaluronic acid derivative | 35 k HA-C6-Chol-19% | 8.4 | 100 |
| | Active ingredient | CyA | 0.89 | 10.6 |
| | Association promoter | Polysorbate 80 | 0.23 | 2.74 |
| Example 1-2 | Hyaluronic acid derivative | 35 k HA-C6-Chol-19% | 9 | 100 |
| | Active ingredient | CyA | 2.2 | 24.4 |
| | Association promoter | Polyethylene glycol 400 | 281 | 3125 |
| Example 1-3 | Hyaluronic acid derivative | 35 k HA-C6-Chol-19% | 9 | 100 |
| | Active ingredient | CyA | 2.2 | 24.4 |
| | Association promoter 1 | Polysorbate 80 | 1.25 | 13.9 |
| | Association promoter 2 | Polyethylene glycol 400 | 270 | 3000 |
| Comparative Example 1-1 | α-Cyclodextrin | - | 8.4 | 100 |
| | Active ingredient | CyA | 0.89 | 10.6 |
| | Association promoter | - | - | - |
| Comparative Example 1-2 | Hyaluronic acid derivative | 35 k HA-C6-Chol-19% | 8.4 | 100 |
| | Active ingredient | CyA | 0.89 | 10.6 |
| | Association promoter | - | - | - |
| | Solvent | Ethanol | - | - |

<Test Example 2>

A pharmacokinetic test in rats of CyA was carried out.
The solution preparations formed of the hyaluronic acid derivative pharmaceutical compositions prepared in Examples 1-1, 1-2, and 1-3 and Comparative Example 1-2 and the solution preparation of Comparative Example 1-1 were

each subcutaneously administered to normal rats (SD, 6 weeks old, male) at the doses (mg/kg) listed in Table 2 using a 25 G needle.

After the administration, blood was collected from the jugular vein with a heparin-treated syringe over time, and aprotinin was added as a protease inhibitor. The obtained blood was separated into plasma and measured by LC-MS/MS. The changes in the plasma concentration of CyA at the time of administering various CyA preparations and the changes in the plasma concentration of CyA in Comparative Example 1 are shown in FIG. 3. In FIG. 3, Example 2-1 is a case where a solution preparation formed of the hyaluronic acid derivative pharmaceutical composition of Example 1-1 was administered. Examples 2-2 and 2-3 and Comparative Examples 2-1 and 2-2 are cases where each of the solution preparations formed of the hyaluronic acid derivative pharmaceutical compositions of Examples 1-2 and 1-3 and Comparative Example 1-2 and the solution preparation of Comparative Example 1-1 was administered.

In addition, pharmacokinetic parameters (drug half-life ($T_{1/2}$) and mean retention time (MRT), serum drug concentration - (0 to infinity) time area under curve ($AUC_{inf}$)) were analyzed by WinNonlin Ver. 8.3 (manufactured by Pharsight Corporation), and the values thereof are listed in Table 2.

[Table 2]

| | CyA dose | CyA concentration in preparation | $T_{1/2}$ | MRT | $AUC_{inf}$ |
|---|---|---|---|---|---|
| | [mg/kg] | [mg/mL] | [h] | [h] | [hr·day/mL] |
| Example 2-1 | 5 | 0.89 | 64.5 | 109 | 8282 |
| Example 2-2 | 10 | 2.2 | 117 | 115 | 14680 |
| Example 2-3 | 10 | 2.2 | 87 | 121 | 11372 |
| Comparative Example 2-1 | 1 | 0.2 | 20.1 | 27 | 5830 |
| Comparative Example 2-2 | 5 | 0.89 | 50.6 | 81 | 5331 |

In the present specification, the sustained-release property is evaluated by the mean residual time (MRT), and in a case where the MRT of the association promoter-containing hyaluronic acid derivative pharmaceutical composition is longer than the MRT of the hyaluronic acid derivative pharmaceutical composition, it is evaluated that the sustained-release property is exhibited.

More specifically, in a case where Expression (A) is satisfied, it is evaluated that "the concentration of the active ingredient can be maintained for a long period of time in vivo".

(MRT of association promoter-containing hyaluronic acid derivative pharmaceutical composition)/ (MRT of hyaluronic acid derivative pharmaceutical composition) $\geq$ 1. 1 ... Expression (A) Expression (A)

In Examples 2-1, 2-2, and 2-3, which were preparations containing an association promoter of a hyaluronic acid derivative, the half-life of the concentration in the blood was long and the MRT was long as compared with the preparation of Comparative Example 2-2, which did not contain an association promoter. In a case where the sustained-release property was evaluated by Expression (A), the results of Example 2-1, Example 2-2, and Example 2-3 were 1.35, 1.42, and 1.49 as compared with Comparative Example 2-2. That is, it was found that the drug remained in the blood for a long period of time. As described above, it was confirmed that the hyaluronic acid derivative composition of the present invention had excellent longer-term sustained-release properties.

<Test Example 3>

The degree of association promotion was evaluated by GPC.

[Example 3-1]

The degree of association promotion of the hyaluronic acid derivative by GPC was evaluated for Example 1-1 prepared in Test Example 1.

[Evaluation of degree of association promotion of hyaluronic acid derivative: area ratio A2/A1]

FIG. 1 is a chromatogram of the hyaluronic acid derivative used in Synthesis Example 1.

The hyaluronic derivative was stirred and dissolved in water for injection for 24 hours at a proportion at which the

concentration of the hyaluronic acid derivative was 1 mg/mL, and the solution was subjected to GPC measurement. In this case, the area value surrounded by the chromatogram and the baseline was defined as A1.

FIG. 2 is a chromatogram of the hyaluronic acid derivative composition of Example 1-1.

The measurement was performed by diluting the hyaluronic acid derivative with water for injection at a proportion set such that the concentration of the hyaluronic acid derivative was 1 mg/mL. In this case, the area value surrounded by the chromatogram and the baseline was defined as A2.

(GPC measurement conditions)

Device: HLC8320-GPC (manufactured by Tosoh Corporation)
Column: G4000SWXL (manufactured by Tosoh Corporation, particle diameter: 8 μm, inner diameter: 7.8 mm, length: 30 cm, product number: 8542)
Eluent: 10 mM phosphate buffer solution (pH of 7.4)
Flow rate: 1 mL/min
Injection volume: 50 μL
Detector: RI (differential refractometer)
Temperature: 30°C

[Examples 3-2 and 3-3 and Comparative Examples 3-1 and 3-2]

In Examples 1-2 and 1-3 and Comparative Example 1-2, the measurement was performed by diluting the hyaluronic acid derivative with water for injection at a proportion set such that the concentration of the hyaluronic acid derivative was 1 mg/mL in the same manner as in Example 1-1. In Comparative Example 2-1, the solution was subjected to GPC measurement without dilution. In this case, A2, which was an area value surrounded by each chromatogram and the baseline, was calculated, and the value of the area ratio A2/A1 with respect to A1 is listed in Table 3.

[Comparative Examples 3-3 to 3-6]

An aqueous solution was prepared at a proportion at which the concentration of the additive alone was the same as that in Examples 3-1 to 3-3 and Comparative Example 3-2, and the aqueous solution was subjected to GPC measurement in the same manner as described above.

[Table 3]

| | Additive | Content of association promoter in units of parts by mass with respect to 100 parts by mass of hyaluronic acid derivative | Degree of association promotion A2/A1 [-] |
|---|---|---|---|
| Example 3-1 | Polysorbate 80 | 2.74 | 1.55 |
| Example 3-2 | Polyethylene glycol 400 | 3125 | 1.83 |
| Example 3-3 | Polyethylene glycol 400 | 3000 | 2.12 |
| | Polysorbate 80 | 13.9 | |
| Comparative Example 3-1 | - | - | No peaks |
| Comparative Example 3-2 | Ethanol | 41.8 | 1.06 |
| Comparative Example 3-3 | Polysorbate 80 | - | No peaks |
| Comparative Example 3-4 | Polyethylene glycol 400 | - | No peaks |
| Comparative Example 3-5 | Polyethylene glycol 400 | - | No peaks |
| | Polysorbate 80 | - | No peaks |
| Comparative Example 3-6 | Ethanol | - | No peaks |

As shown in the results described above, it was suggested that the hyaluronic acid derivatives of Examples 3-1 to

3-3 were promoted to associate with polysorbate 80 or polyethylene glycol 400. Meanwhile, it was confirmed that in ethanol, the area value of the peak of the hyaluronic acid derivative in GPC hardly changed regardless of the presence or absence of the addition of ethanol, and the association of the hyaluronic acid derivative was not promoted.

<Test Example 4>

The association promoter was evaluated by GPC.

[Examples 4-1, 4-2, and 4-3 and Comparative Examples 4-1 and 4-2]

A hyaluronic acid derivative aqueous solution was prepared as listed in Table 4 at the proportion at which the composition was obtained except that CyA as an active ingredient and sucrose as an isotonic agent were not contained, in the same manner as in Test Example 1. Each of the adjusted samples was diluted with water for injection at a proportion set such that the concentration of the hyaluronic acid derivative reached 1 mg/mL and subjected to GPC measurement.

[Table 4]

| | Association promoter | Content of association promoter in units of parts by mass with respect to 100 parts by mass of hyaluronic acid derivative | Degree of association promotion A2/A1 [-] |
|---|---|---|---|
| Example 4-1 | Polysorbate 80 | 2.74 | 1.32 |
| Example 4-2 | PEG400 | 3125 | 1.52 |
| Example 4-3 | PEG400 | 3000 | 1.91 |
| | Polysorbate 80 | 13.9 | |
| Comparative Example 4-1 | - | - | No peaks |
| Comparative Example 4-2 | Ethanol | 41.8 | 1.03 |

<Test Example 5>

The precipitation behavior at the physiological saline concentration in vitro was confirmed.

[Examples 5-1 to 5-3 and Comparative Examples 5-1 and 5-2]

150 μL of each preparation obtained in Example 1 was sampled in a 1.5 mL microtube. Thereafter, a concentrated buffer solution (40 mM PB (pH of 7.4), 600 mM NaCl) was added at a proportion at which the final buffer solution composition was formed of 10 mM PB (pH of 7.4) and 150 mM NaCl, and the hyaluronic acid derivative was precipitated. After incubation at 37°C for 20 minutes, centrifugation was carried out at 2,000 G for 5 minutes, 50 μL of the supernatant was sampled, the supernatant was diluted two times with a HP-β-CD aqueous solution (300 mM), the diluted solution was incubated for 1 hour, and 350 μL of the HP-β-CD aqueous solution (10 mM) was added to the diluted solution for dilution, and the solution was subjected to GPC measurement. The residual rate of the hyaluronic acid derivative in the solution to the initial use amount of the hyaluronic acid derivative was calculated from the peak area of the detected hyaluronic acid derivative, and the proportion (precipitation rate) of the precipitated hyaluronic acid derivative was calculated. The precipitation evaluation method and the results of the precipitation evaluation method described in [Physical Properties of Pharmaceutical Composition] (Test Conditions) and the preparation of the precipitated sample match each other.

(GPC measurement conditions)

Device: HLC8420-GPC (manufactured by Tosoh Corporation)
Column: G4000SWXL (manufactured by Tosoh Corporation, particle diameter: 8 μm, inner diameter: 7.8 mm, length: 30 cm, product number: 8542)
Eluent: 10 mM HP-β-CD/phosphate buffer solution (pH of 7.4)

Flow rate: 1 mL/min
Injection volume: 50 μL
Detector: RI (differential refractometer)
Temperature: 30°C

[Table 5]

| | Sample | Item | Name of ingredient | Concentration in preparation [mg/mL] | Precipitation rate [%] |
|---|---|---|---|---|---|
| Example 5-1 | Example 1-1 | Hyaluronic acid derivative | 35 k HA-C6-Chol-19% | 8.4 | 96.30% |
| | | Active ingredient | CyA | 0.89 | |
| | | Association promoter | Polysorbate 80 | 0.23 | |
| Example 5-2 | Example 1-2 | Hyaluronic acid derivative | 35 k HA-C6-Chol-19% | 9 | 98.00% |
| | | Active ingredient | CyA | 2.2 | |
| | | Association promoter | Polyethylene glycol 400 | 281 | |
| Example 5-3 | Example 1-3 | Hyaluronic acid derivative | 35 k HA-C6-Chol-19% | 9 | 97.20% |
| | | Active ingredient | CyA | 2.2 | |
| | | Association promoter 1 | Polysorbate 80 | 1.25 | |
| | | Association promoter 2 | Polyethylene glycol 400 | 270 | |
| Comparative Example 5-1 | Comparative Example 1-1 | α-Cyclodextrin | - | 8.4 | Precipitate was not visually observed |
| | | Active ingredient | CyA | 0.89 | |
| | | Association promoter | - | - | |
| Comparative Example 5-2 | Comparative Example 1-2 | Hyaluronic acid derivative | 35 k HA-C6-Chol-19% | 8.4 | 90.60% |
| | | Active ingredient | CyA | 0.89 | |
| | | Association promoter | - | - | |
| | | Solvent | Ethanol | | |

As shown in the results, it was suggested that the hyaluronic acid derivatives of Examples 5-1 to 5-3 not only promote the association by polysorbate 80 or polyethylene glycol 400, but also improve the precipitation performance at the physiological saline concentration in vitro.

<Test Example 6>

The kind of the association promoter was examined.

A solution obtained by stirring and dissolving the hyaluronic acid derivative (35 k HA-C6-Chol-19%) of the freeze-dried product obtained in Synthesis Example 1 in water for injection at 20°C for 24 hours at 12.0 mg/mL was used in Examples 6-1 to 6-11 and Comparative Examples 6-1 to 6-6.

[Example 6-1]

The hyaluronic acid derivative aqueous solution dissolved as described above was diluted with water for injection at a proportion of 2 mg/mL. In addition, adjustment was made such that polyethylene glycol 300 was diluted with water for injection at a proportion set such that the concentration was 10 mg/mL. The above-described hyaluronic acid derivative aqueous solution and the polyethylene glycol 300 aqueous solution were mixed at a volume ratio of 1:1, and adjustment was made to obtain the composition listed in Table 6. Thereafter, the solution was incubated at 20°C for 24 hours and then subjected to GPC measurement.

(GPC measurement conditions)

Device: HLC8420-GPC (manufactured by Tosoh Corporation)
Column: G4000SWXL (manufactured by Tosoh Corporation, particle diameter: 8 μm, inner diameter: 7.8 mm, length: 30 cm, product number: 8542)
Eluent: 10 mM phosphate buffer solution (pH of 7.4)
Flow rate: 1 mL/min
Injection volume: 50 μL
Detector: RI (differential refractometer)
Temperature: 30°C

[Examples 6-2 to 6-11 and Comparative Examples 6-1 to 6-6]

A solution was prepared at the proportion adjusted to obtain the composition of Table 6 by the same method as in Example 6-1, and subjected to GPC measurement.

[Table 6]

| | Association promoter | Content of association promoter in units of parts by mass with respect to 100 parts by mass of hyaluronic acid derivative | Degree of association promotion A2/A1 [-] |
|---|---|---|---|
| Example 6-1 | Polyethylene glycol 300 | 500 | 2.34 |
| Example 6-2 | Polyethylene glycol 400 | 500 | 2.34 |
| Example 6-3 | Polyethylene glycol 400 | 2815 | 1.52 |
| Example 6-4 | Polyethylene glycol 400 | 4223 | 1.78 |
| Example 6-5 | Polysorbate 80 | 2.74 | 1.47 |
| Example 6-6 | Polysorbate 80 | 13 | 1.91 |
| Example 6-7 | Polysorbate 80 | 50 | 2.59 |
| Example 6-8 | Poloxamer 188 | 50 | 1.84 |
| Example 6-9 | Poloxamer 188 | 100 | 2.21 |
| Example 6-10 | Cremophor EL | 26.6 | 1.89 |
| Example 6-11 | Cremophor EL | 100 | 2.37 |
| Comparative Example 6-1 | Tetrahydrofuran | 1500 | 1.13 |
| Comparative Example 6-2 | Ethanol | 1500 | 1.12 |
| Comparative Example 6-3 | Ethylene glycol | 1500 | 1.16 |
| Comparative Example 6-4 | Tetrahydrofuran | 62.5 | 1.12 |
| Comparative Example 6-5 | Ethanol | 62.5 | 1.12 |
| Comparative Example 6-6 | Ethylene glycol | 62.5 | 1.15 |

As shown in the result, it was suggested that the hyaluronic acid derivatives of Examples 6-1 to 6-11 were promoted to associate with polyethylene glycol 300, poloxamer 188, and Cremophor EL other than polysorbate 80 and polyethylene glycol 400. Meanwhile, in Comparative Examples 6-1 to 6-4, it was confirmed that the area value of the peak of the hyaluronic acid derivative in GPC was almost unchanged regardless of the presence or absence of the addition of ethanol or the like, and the association of the hyaluronic acid derivative was not promoted in tetrahydrofuran (having one ether structure and having 4 carbon atoms), ethanol (having no ether structure and having 2 carbon atoms), and ethylene glycol (having no ether structure and having 2 carbon atoms). Here, similarly to polysorbate 80 or polyethylene glycol 400, no GPC peak of the association promoter alone was detected at the peak position of the hyaluronic acid derivative for poloxamer 188 or Cremophor EL.

<Test Example 7>

A hyaluronic acid derivative pharmaceutical composition was produced.

A solution obtained by stirring and dissolving the hyaluronic acid derivative (35 k HA-C6-Chol-19%) of the freeze-dried product obtained in Synthesis Example 1 in water for injection at 20°C for 24 hours at 12.0 mg/mL was used in Examples 7-1 to 7-10 and Comparative Examples 7-1 to 7-6.

[Example 7-1]

A CyA preparation was prepared using an association promoter (Polysorbate 80)-containing hyaluronic acid derivative (35 k HA-C6-Chol-19%).

The following operation was performed in an environment at 20°C and room temperature.

1.8 mg of powder of CyA (Tokyo Chemical Industry Co., Ltd., product number: C2408) was weighed in a vial, and then water for injection was added to another vial so that the concentration of polysorbate 80 was 0.5 mg/mL, and diluted. The weighed CyA was suspended in 18 μL of a polysorbate 80 aqueous solution having a concentration of 0.5 mg/mL. Subsequently, 450 μL of a 12 mg/mL hyaluronic acid derivative aqueous solution was added to the CyA-containing polysorbate 80 aqueous solution while the solution was stirred, and 432 μL of water for injection was added thereto so that the composition at the time of formulation was set such that the concentration of CyA was 2 mg/mL, the concentration of the hyaluronic acid derivative was 6 mg/mL, and the concentration of polysorbate 80 was 0.01 mg/mL, thereby carrying out the compounding of the drug. The drug was stirred at 20°C for 24 hours, the precipitate was filtered through a 0.45 μm sterile filtration filter, the precipitated CyA was removed, and the drug concentration in the preparation was quantified by HPLC measurement described below. The final composition is listed in Table 7.

[Examples 7-2 to 7-10]

An association promoter-containing hyaluronic acid derivative pharmaceutical composition was produced by the same method as in Example 7-1 such that the concentration of the association promoter in the final composition was set to the concentration listed in Table 7. In addition, the CyA concentration in the preparation after being filtered through a 0.45 μm sterile filtration filter was also quantified by HPLC in the same manner as in Example 7-1, and the results are collectively listed in Table 7.

[HPLC quantification conditions: reverse phase chromatography analysis conditions]

Column: Inert Sustain C18 particle diameter 5 μm × inner diameter 4.6 mm × length 150 mm
Column temperature: 40°C
Mobile phase: eluent A 0.1% TFA/acetonitrile
Eluent B: 0.1% TFA/water
Ratio between mobile phases: eluent A/eluent B = 9/1
Flow rate: 1 mL/min
Injection volume: 30 μL
Detector: UV (210 nm)

[Conditions for evaluating precipitate]

The precipitation behavior of each pharmaceutical composition prepared in Test Example 7 at a physiological saline concentration in vitro was evaluated by the following method.

[Preparation of precipitated sample]

200 μL of a concentrated buffer solution (40 mM PB, 150 mM NaCl aqueous solution) was put into a microtube (1.5 mL), and 600 μL of a preparation formed of a hyaluronic acid derivative pharmaceutical composition was put into the microtube. Thereafter, the mixture is mixed with a voltex for 30 seconds and incubated at 37°C for 20 min, and then the precipitate is sedimented by a centrifuge (2,000 G, 5 min). Subsequently, the hyaluronic acid derivative in the supernatant is subjected to GPC measurement.

[Preparation of blank sample]

200 μL of water for injection is put into a microtube (1.5 mL), and 600 μL of a preparation formed of the hyaluronic acid derivative pharmaceutical composition (the theoretical concentration of the hyaluronic acid derivative is set to Xm g/mL, X > 1.33) is put into the microtube. Thereafter, the mixture is mixed with a voltex for 30 seconds, incubated at 37°C for 20 minutes, and then subjected to a centrifuge (2,000 G, 5 minutes). Subsequently, the supernatant is diluted with water for injection such that the concentration of the hyaluronic acid derivative in the supernatant is 1 mg/mL, and the diluted solution is subjected to GPC measurement.

[GPC measurement conditions]

Device: HLC8420-GPC (manufactured by Tosoh Corporation)
Column: G4000SWXL (manufactured by Tosoh Corporation, particle diameter: 8 μm, inner diameter: 7.8 mm, length: 30 cm, product number: 8542)
Eluent: 10 mM phosphate buffer solution (pH of 7.4)
Flow rate: 1 mL/min
Injection volume: 50 μL
Detector: RI
Temperature: 30°C

[Confirmation of precipitate]

In the present specification, the hyaluronic acid derivative pharmaceutical composition in which the precipitation rate represented by the following equation is 20% or greater is precipitated at a physiological saline concentration.

Precipitation rate (%) = {1 - (area value of hyaluronic acid derivative of precipitated sample) ÷ (area value of hyaluronic acid derivative of blank sample) × (0.75X)} × 100

[Table 7]

| | Association promoter | Concentration of association promoter in preparation | CyA concentration in preparation | Substantial amount of active ingredient (C) (CyA) in units of parts by mass with respect to 100 parts by mass of hyaluronic acid derivative | Content of association promoter in units of parts by mass with respect to 100 parts by mass of hyaluronic acid derivative | Precipitation rate |
|---|---|---|---|---|---|---|
| | | [mg/mL] | [mg/mL] | Parts by mass | Parts by mass | [%] |
| Example 7-1 | Polysorbate 80 | 0.01 | 1.17 | 19.6 | 0.167 | >95% |
| Example 7-2 | | 0.10 | 1.21 | 20.1 | 1.67 | >95% |
| Example 7-3 | | 12.00 | 1.25 | 20.8 | 200 | >95% |
| Example 7-4 | Polysorbate 80 | 0.001 | 1.19 | 19.8 | 0.0167 | >95% |
| Example 7-5 | | 0.10 | 1.00 | 16.7 | 1.67 | >95% |
| Example 7-6 | | 12.00 | 1.22 | 20.3 | 200 | >95% |
| Example 7-7 | Poloxamer 188 | 0.01 | 1.26 | 19.6 | 0.167 | >95% |
| Example 7-8 | | 0.10 | 1.24 | 20.1 | 1.67 | >95% |
| Example 7-9 | | 12.00 | 1.10 | 20.8 | 200 | >95% |
| Example 7-10 | Cremophor EL | 0.001 | 1.22 | 20.4 | 0.0167 | >95% |
| Comparative Example 7-1 | Polysorbate 80 | 0.01 | 0.00254 | - | - | Precipitate was not visually observed |
| Comparative Example 7-2 | | 0.10 | 0.00288 | - | - | Precipitate was not visually observed |
| Comparative Example 7-3 | Polysorbate 80 | 0.01 | 0.00260 | - | - | Precipitate was not visually observed |
| Comparative Example 7-4 | | 0.10 | 0.00119 | - | - | Precipitate was not visually observed |
| Comparative Example 7-5 | Poloxamer 188 | 0.01 | 0.00073 | - | - | Precipitate was not visually observed |
| Comparative Example 7-6 | | 0.10 | 0.00160 | - | - | Precipitate was not visually observed |

As shown in the results, it was shown that the poorly water-soluble active ingredient can be solubilized at a high concentration without using an organic solvent from the powder, only by adding a small amount of various association promoters such as polysorbate 80, polysorbate 20, poloxamer 188, and Cremophor EL. Further, it was suggested that the precipitation performance was also improved by the association promoter. As described above, it is expected that a pharmaceutical composition in which the association of the hyaluronic acid derivative is promoted by the association promoter, the amount of the active ingredient is high, and the long-term sustained-release can be obtained.

<Test Example 8>

The kind of the hyaluronic acid derivatives was examined.
A solution obtained by stirring and dissolving 2.0 mg/mL of the hyaluronic acid derivative (10 k HA-C6-Chol-30%) of the freeze-dried product obtained in Synthesis Example 2 in water for injection at 20°C for 24 hours was used in Examples 8-1 to 8-11 and Comparative Examples 8-1 to 8-6.

[Example 8-1]

Adjustment was made such that Polysorbate 80 was diluted with water for injection at a proportion set such that the concentration was 10 mg/mL. A 6 mL clean vial was prepared, 400 μL of the above-described hyaluronic acid derivative aqueous solution and 10 μL of a polysorbate 80 aqueous solution were added thereto, the mixture was mixed with a voltex for 30 seconds, and 390 μL of water for injection was added thereto to adjust the total volume to 800 μL. Adjustment was made at the proportion set such that the composition listed in Table 8 was obtained. Thereafter, the mixture was incubated at 20°C for 24 hours and then subjected to GPC measurement.

(GPC measurement conditions)

Device: HLC8420-GPC (manufactured by Tosoh Corporation)

Column: G4000SWXL (manufactured by Tosoh Corporation, particle diameter: 8 μm, inner diameter: 7.8 mm, length: 30 cm, product number: 8542)

Eluent: 10 mM phosphate buffer solution (pH of 7.4)

Flow rate: 1 mL/min

Injection volume: 50 μL

Detector: RI (differential refractometer)

Temperature: 30°C

[Examples 8-2 to 8-4 and Comparative Examples 8-1 to 8-6]

A solution was prepared in the same manner as in Example 8-1 at the proportion set such that the composition in Table 8 was obtained, and subjected to GPC measurement.

[Table 8]

| | Association promoter | Content of association promoter in units of parts by mass with respect to 100 parts by mass of hyaluronic acid derivative | Degree of association promotion A2/A1 [-] |
|---|---|---|---|
| Example 8-1 | Polysorbate 80 | 13 | 1.43 |
| Example 8-2 | Polysorbate 80 | 100 | 1.50 |
| Example 8-3 | Polysorbate 20 | 100 | 1.34 |
| Example 8-4 | Cremophor EL | 50 | 1.45 |

(continued)

| | Association promoter | Content of association promoter in units of parts by mass with respect to 100 parts by mass of hyaluronic acid derivative | Degree of association promotion A2/A1 [-] |
|---|---|---|---|
| Comparative Example 8-1 | Tetrahydrofuran | 111 | 1.03 |
| Comparative Example 8-2 | Tetrahydrofuran | 222 | 1.04 |
| Comparative Example 8-3 | Ethanol | 99 | 1.06 |
| Comparative Example 8-4 | Ethanol | 197 | 0.99 |
| Comparative Example 8-5 | Ethanol | 493 | 1.02 |
| Comparative Example 8-6 | Acetonitrile | 98 | 1.04 |

As shown in the result, it was suggested that the hyaluronic acid derivatives of Examples 8-1 to 8-4 were associated by polysorbate 80, polysorbate 20, and Cremophor EL. Meanwhile, in Comparative Examples 8-1 to 8-6, it was confirmed that the area value of the peak of the hyaluronic acid derivative in GPC was almost unchanged regardless of the presence or absence of the addition of tetrahydrofuran or the like, and the association of the hyaluronic acid derivative was not promoted in tetrahydrofuran (having one ether structure and having 4 carbon atoms), ethanol (having no ether structure and having 2 carbon atoms), and acetonitrile (having no ether structure and having 2 carbon atoms). Therefore, it is expected that even in the case of a hyaluronic acid derivative (10 k HA-C6-Chol-30%) having a low molecular weight and a high introduction rate of hydrophobic groups, the association of the hyaluronic acid derivative is promoted by the association promoter, and a pharmaceutical composition having a high amount of the active ingredient and capable of long-term sustained-release is obtained.

<Test Example 9>

A hyaluronic acid derivative pharmaceutical composition formed of human growth hormone (protein) was prepared.

[Example 9-1]

A solution obtained by stirring and dissolving the hyaluronic acid derivative (35 k HA-C6-Chol-19%) of the freeze-dried product obtained in Synthesis Example 1 in water for injection at 20°C for 24 hours at 4.0 mg/mL was used in Examples 9-1 and 9-2 and Comparative Example 9-1.

[Example 9-1 and Example 9-2]

The association promoter and water for injection were added to the above-described hyaluronic acid derivative aqueous solution.

In addition, a powder of human growth hormone (hGH: Genotropin (registered trademark) for injection) was dissolved in water for injection in another vial to have a concentration of 2 mg/mL, and after the preparation, 250 μL of the solution was added to the association promoter-containing hyaluronic acid derivative aqueous solution, and the solution was adjusted to have a total amount of 1 mL. Thereafter, the mixture was incubated at 37°C for 24 hours to promote compounding with hGH. The composition of the final preparation was adjusted to the concentration listed in Table 9.

[Comparative Example 9-1]

Tetrahydrofuran (THF) and water for injection were added to the above-described hyaluronic acid derivative aqueous solution prepared at a concentration of 4.0 mg/mL. In addition, 250 μL of a 2.0 mg/mL hGH aqueous solution was added to the THF-containing hyaluronic acid derivative aqueous solution, and the total amount was adjusted to 1 mL. Thereafter, the mixture was incubated at 37°C for 24 hours to promote compounding with hGH. Similarly to Example 9-1, the composition of the final preparation was adjusted set such that the concentration was as listed in Table 9.

In all of Examples 9-1 and 9-2 and Comparative Example 9-1, the concentration of the hyaluronic acid derivative was adjusted to 1.0 mg/mL, and the concentration of hGH was adjusted to 0.5 mg/mL. After 24 hours, a clear solution was obtained in Examples 9-1 and 9-2. Here, in only Comparative Example 9-1, the solution was cloudy after incubation at

37°C for 24 hours, and it was difficult to acquire a composite solution of the hyaluronic acid derivative and hGH.

[Table 9]

| | Association promoter | Content of association promoter in units of parts by mass with respect to 100 parts by mass of hyaluronic acid derivative | Substantial amount of active ingredient (C) (hGH) in units of parts by mass with respect to 100 parts by mass of hyaluronic acid derivative |
|---|---|---|---|
| Example 9-1 | Polyethylene glycol 400 | 33750 | 49.0 |
| Example 9-2 | Polyethylene glycol 400 | 11250 | 44.6 |
| Comparative Example 9-1 | Tetrahydrofuran | 26767 | - |

Subsequently, the compounding ratio of hGH in Examples 9-1 and 9-2 was evaluated by GPC shown below.

[GPC measurement conditions]

Device: HLC8420-GPC (manufactured by Tosoh Corporation)

Column: G4000SWXL (manufactured by Tosoh Corporation, particle diameter: 8 μm, inner diameter: 7.8 mm, length: 30 cm, product number: 8542)

Eluent: 10 mM phosphate buffer solution (pH of 7.4)

Flow rate: 1 mL/min

Injection volume: 50 μL

Detector: UV (280 nm)

Temperature: 30°C

Samples of hGH alone having concentrations of 0.50 mg/mL and 0.25 mg/mL were prepared and subjected to GPC measurement. At this time, a peak of hGH single body was confirmed at 11.5 minutes. In a case where the hGH single body was compounded and incorporated into the hyaluronic acid derivative composition, the peak area value of the hGH single body at 11.5 minutes was reduced. The concentration of hGH contained in the hyaluronic acid derivative composition was calculated as the hGH compounding ratio based on the reduction rate of the peak area. It was also confirmed that the peak area value derived from the hyaluronic acid derivative was increased in the hyaluronic acid derivative composition containing hGH. Table 9 lists the substantial amount of the active ingredient (C) (hGH) in units of parts by mass with respect to 100 parts by mass of the hyaluronic acid derivative, based on the hGH compounding ratio calculated above.

As shown in the results, the hyaluronic acid derivative composition containing polyethylene glycol 400 as an association promoter was efficiently compounded with hGH, which is a protein pharmaceutical product. The long-term sustained-release function is expected not only for the poorly soluble drug but also for the water-soluble drug. In addition, it is speculated that long-term sustained-release can be achieved for many modalities by application to the protein pharmaceutical product other than a peptide pharmaceutical product such as CyA.

Meanwhile, in the hGH-hyaluronic acid derivative pharmaceutical composition prepared in Comparative Example 9-1, a cloudy sample was obtained, probably because THF inhibited the compounding. It was presumed that hGH was not successfully compounded with the hyaluronic acid derivative.

As described above, it is expected that a protein-hyaluronic acid derivative pharmaceutical composition, in which the compounding with hGH is efficiently carried out in the presence of the association promoter and which is capable of long-term sustained-release in vivo, can be acquired.

<Test Example 10>

[Synthesis Example 3]

The degree of association promotion of the hyaluronic acid derivative (10 k HA-C6-Chol-40%) of the freeze-dried product obtained by the same method as in Synthesis Example 2 with reference to Test Example 6 was evaluated by GPC.

(GPC measurement conditions)

Device: HLC8420-GPC (manufactured by Tosoh Corporation)
Column: G4000SWXL (manufactured by Tosoh Corporation, particle diameter: 8 μm, inner diameter: 7.8 mm, length: 30 cm, product number: 8542)
Eluent: 10 mM phosphate buffer solution (pH of 7.4)
Flow rate: 1 mL/min
Injection volume: 50 μL
Detector: RI (differential refractometer)
Temperature: 30°C

An area ratio A2/A1 used for evaluating the degree of association promotion of the hyaluronic acid derivative was calculated by the same method as in Test Example 3.

10 mM PB (phosphate buffer solution, pH 7.4) was added to the hyaluronic acid derivative (10 k HA-C6-Chol-40%) obtained in Synthesis Example 3 at a proportion set such that the concentration was 1.0 mg/mL, and the mixture was stirred overnight to completely dissolve the hyaluronic acid derivative. An area value calculated by the GPC evaluation of this sample was defined as A1.

[Example 10-1]

A solution obtained by stirring and dissolving the hyaluronic acid derivative (10 k HA-C6-Chol-40%) obtained in Synthesis Example 3 in water for injection at 20°C for 24 hours at 36.0 mg/mL was used in Examples 10-1 to 10-9. In addition, polysorbate 80 was diluted with water for injection at a proportion set such that the concentration was 10 mg/mL in another vial, thereby adjusting the concentration. 600 μL of the above-described hyaluronic acid derivative aqueous solution was added to a 6 ml sterilized vial, 10 μL of a 10 mg/mL polysorbate 80 aqueous solution was added thereto while being stirred, the solution was mixed, and finally 390 μL of water for injection was added thereto. Thereafter, the solution was incubated at 20°C for 24 hours, diluted with 10 mM PB (phosphate buffer solution, pH 7.4) at a proportion at which the concentration of the hyaluronic acid derivative was 1 mg/mL, and then subjected to GPC measurement.

[Example 10-2]

First, a 100 mM PB phosphate buffer solution (pH of 7.4) was prepared using a phosphate buffer powder (manufactured by FUJIFILM Wako Pure Chemical Corporation: 167-14491 for Biochemistry).

Subsequently, the following solution adjustment was carried out using the solution prepared in Example 10-1. 600 μL of the above-described hyaluronic acid derivative aqueous solution was added to a 6 ml sterilized vial, 50 μL of a 10 mg/mL polysorbate 80 aqueous solution was added thereto and mixed with the mixture while being stirred, 100 μL of a 100 mM PB phosphate buffer solution was added thereto, and finally 250 μL of water for injection was added thereto, thereby preparing an hyaluronic acid derivative aqueous solution at a proportion at which the final concentration was 10 mM PB. Thereafter, the solution was incubated at 20°C for 24 hours, diluted with 10 mM PB (phosphate buffer solution, pH 7.4) at a proportion at which the concentration of the hyaluronic acid derivative was 1 mg/mL, and then subjected to GPC measurement.

[Example 10-3]

The following solution adjustment was carried out using the solution prepared in Example 10-1. 600 μL of the above-described hyaluronic acid derivative aqueous solution was added to a 6 ml sterilized vial, 50 μL of a 10 mg/mL polysorbate 80 aqueous solution was added thereto and mixed with the mixture while being stirred, and finally 350 μL of water for injection was added thereto to prepare a hyaluronic acid derivative aqueous solution. Thereafter, the solution was incubated at 20°C for 24 hours, diluted with 10 mM PB (phosphate buffer solution, pH 7.4) at a proportion at which the

concentration of the hyaluronic acid derivative was 1 mg/mL, and then subjected to GPC measurement.

[Example 10-4]

Subsequently, the following solution adjustment was carried out using the solutions prepared in Example 10-1 and Example 10-2. 600 μL of the above-described hyaluronic acid derivative aqueous solution was added to a 6 ml sterilized vial, 100 μL of polyethylene glycol 400 (manufactured by Nacalai Tesque, Inc.) was added thereto and mixed with the mixture while being stirred, 100 μL of a 100 mM PB phosphate buffer solution was added thereto, and finally, 200 μL of water for injection was added thereto, thereby preparing an hyaluronic acid derivative aqueous solution at a proportion set such that the final concentration was 10 mM PB. Thereafter, the solution was incubated at 20°C for 24 hours, diluted with 10 mM PB (phosphate buffer solution, pH 7.4) at a proportion at which the concentration of the hyaluronic acid derivative was 1 mg/mL, and then subjected to GPC measurement.

[Example 10-5]

Subsequently, the following solution adjustment was carried out using the solution prepared in Example 10-1. 600 μL of the above-described hyaluronic acid derivative aqueous solution was added to a 6 ml sterilized vial, 100 μL of polyethylene glycol 400 (manufactured by Nacalai Tesque, Inc.) was added thereto and mixed with the mixture while being stirred, and finally 300 μL of water for injection was added thereto to prepare an hyaluronic acid derivative aqueous solution. Thereafter, the solution was incubated at 20°C for 24 hours, diluted with 10 mM PB (phosphate buffer solution, pH 7.4) at a proportion at which the concentration of the hyaluronic acid derivative was 1 mg/mL, and then subjected to GPC measurement.

[Example 10-6]

Subsequently, the following solution adjustment was carried out using the solution prepared in Example 10-1. 600 μL of the above-described hyaluronic acid derivative aqueous solution was added to a 6 ml sterilized vial, 300 μL of polyethylene glycol 400 (manufactured by Nacalai Tesque, Inc.) was added thereto and mixed with the mixture while being stirred, and finally 100 μL of water for injection was added thereto to prepare an hyaluronic acid derivative aqueous solution. Thereafter, the solution was incubated at 20°C for 24 hours, diluted with 10 mM PB (phosphate buffer solution, pH 7.4) at a proportion at which the concentration of the hyaluronic acid derivative was 1 mg/mL, and then subjected to GPC measurement.

[Example 10-7]

First, Poloxamer 338 (manufactured by Sigma-Aldrich Co., LLC) was dissolved by adding water for injection at a proportion set such that the concentration reached 150 mg/mL. Subsequently, the following solution adjustment was carried out using the solution prepared in Example 10-1. 600 μL of the above-described hyaluronic acid derivative aqueous solution was added to a 6 ml sterilized vial, 100 μL of a 150 mg/mL aqueous solution of Poloxamer 338 was added thereto and mixed with the mixture while being stirred, and finally 300 μL of water for injection was added thereto to prepare an hyaluronic acid derivative aqueous solution. Thereafter, the solution was incubated at 20°C for 24 hours, diluted with 10 mM PB (phosphate buffer solution, pH 7.4) at a proportion at which the concentration of the hyaluronic acid derivative was 1 mg/mL, and then subjected to GPC measurement.

[Example 10-8]

The following solution adjustment was carried out using the solutions prepared in Example 10-1, Example 10-2, and Example 10-7. 600 μL of the above-described hyaluronic acid derivative aqueous solution was added to a 6 ml sterilized vial, 300 μL of an aqueous solution of Poloxamer 338 was added thereto and mixed with the mixture while being stirred, and 100 μL of a 100 mM PB phosphate buffer solution was added thereto, thereby preparing an hyaluronic acid derivative aqueous solution at a proportion at which the final concentration was 10 mM PB. Thereafter, the solution was incubated at 20°C for 24 hours, diluted with 10 mM PB (phosphate buffer solution, pH 7.4) at a proportion at which the concentration of the hyaluronic acid derivative was 1 mg/mL, and then subjected to GPC measurement.

[Example 10-9]

The following solution adjustment was carried out using the solutions prepared in Example 10-1 and Example 10-7. 600 μL of the above-described hyaluronic acid derivative aqueous solution was added to a 6 ml sterilized vial, 300 μL

of a 150 mg/mL aqueous solution of Poloxamer 338 was added thereto and mixed with the mixture while being stirred, and finally 100 μL of water for injection was added thereto to prepare a hyaluronic acid derivative aqueous solution. Thereafter, the solution was incubated at 20°C for 24 hours, diluted with 10 mM PB (phosphate buffer solution, pH 7.4) at a proportion at which the concentration of the hyaluronic acid derivative was 1 mg/mL, and then subjected to GPC measurement.

[Example 10-10]

First, water for injection was added to Chol-PEG600 (manufactured by Avanti Polar Lipids, Inc.) at a proportion set such that the concentration was 50 mg/mL, and the Chol-PEG600 was dissolved. Subsequently, the solution obtained by stirring and dissolving the hyaluronic acid derivative (10 k HA-C6-Chol-40%) obtained in Synthesis Example 3 in water for injection at 20°C for 24 hours at 10.0 mg/mL was used in Examples 10-10 to 10-12. 750 μL of the above-described hyaluronic acid derivative aqueous solution was added to a 6 ml sterilized vial, 2.8 μL of a 50 mg/mL Chol-PEG600 aqueous solution was added thereto and mixed with the mixture while being stirred, and finally 747.2 μL of water for injection was added thereto to prepare a hyaluronic acid derivative aqueous solution. Thereafter, the solution was incubated at 20°C for 24 hours, diluted with 10 mM PB (phosphate buffer solution, pH 7.4) at a proportion at which the concentration of the hyaluronic acid derivative was 1 mg/mL, and then subjected to GPC measurement.

[Example 10-11]

The following solution adjustment was carried out using the solution prepared in Example 10-10. 750 μL of the above-described hyaluronic acid derivative aqueous solution was added to a 6 ml sterilized vial, 11.2 μL of a 50 mg/mL Chol-PEG600 aqueous solution was added thereto and mixed with the mixture while being stirred, and finally 738.8 μL of water for injection was added thereto, thereby preparing an hyaluronic acid derivative aqueous solution. Thereafter, the solution was incubated at 20°C for 24 hours, diluted with 10 mM PB (phosphate buffer solution, pH 7.4) at a proportion at which the concentration of the hyaluronic acid derivative was 1 mg/mL, and then subjected to GPC measurement.

[Example 10-12]

The following solution adjustment was carried out using the solution prepared in Example 10-10. 750 μL of the above-described hyaluronic acid derivative aqueous solution was added to a 6 ml sterilized vial, 28 μL of a 50 mg/mL Chol-PEG600 aqueous solution was added thereto and mixed with the mixture while being stirred, and finally 722 μL of water for injection was added thereto to prepare an hyaluronic acid derivative aqueous solution. Thereafter, the solution was incubated at 20°C for 24 hours, diluted with 10 mM PB (phosphate buffer solution, pH 7.4) at a proportion at which the concentration of the hyaluronic acid derivative was 1 mg/mL, and then subjected to GPC measurement.

In the same manner as in Test Example 3, each of chromatograms of Examples 10-1 to 10-12 and A2 which is an area value surrounded by the baseline was calculated, and the value of the area ratio A2/A1 with respect to A1 is listed in Table 10.

[Table 10]

| | Association promoter | Content of association promoter in units of parts by mass with respect to 100 parts by mass of hyaluronic acid derivative | Degree of association promotion A2/A1 [-] |
|---|---|---|---|
| Example 10-1 | Polysorbate 80 | 0.5 | 1.26 |
| Example 10-2 | Polysorbate 80 | 2.3 | 1.41 |
| Example 10-3 | Polysorbate 80 | 2.3 | 1.35 |
| Example 10-4 | Polyethylene glycol 400 | 523 | 1.41 |
| Example 10-5 | Polyethylene glycol 400 | 523 | 1.27 |
| Example 10-6 | Polyethylene glycol 400 | 1308 | 1.81 |
| Example 10-7 | Poloxamer 338 | 69 | 1.42 |
| Example 10-8 | Poloxamer 338 | 243 | 1.81 |
| Example 10-9 | Poloxamer 338 | 243 | 1.74 |
| Example 10-10 | Chol-PEG600 | 1.8 | 1.29 |
| Example 10-11 | Chol-PEG600 | 7.4 | 1.36 |

(continued)

| | Association promoter | Content of association promoter in units of parts by mass with respect to 100 parts by mass of hyaluronic acid derivative | Degree of association promotion A2/A1 [-] |
|---|---|---|---|
| Example 10-12 | Chol-PEG600 | 18.6 | 1.41 |

As shown in the results, it was suggested that the hyaluronic acid derivatives of Examples 10-1 to 10-12 were associated by polysorbate 80, polyethylene glycol 400, Poloxamer 338, and Chol-PEG600. Here, similarly to polysorbate 80 or polyethylene glycol 400, no GPC peak of the association promoter alone was detected at the peak position of the hyaluronic acid derivative for poloxamer 338 or Chol-PEG600.

<Test Example 11>

As Test Example 11, the formulation of cyclosporin (CyA), which is a poorly water-soluble peptide, was carried out.

[Example 11-1]

A CyA preparation was prepared using an association promoter (Polysorbate 80)-containing hyaluronic acid derivative (35 k HA-C6-Chol-19%).

The following operation was performed in an environment at 20°C and room temperature.

A 10% sucrose aqueous solution was added to the hyaluronic acid derivative (35 k HA-C6-Chol-19%) of the freeze-dried product obtained in Synthesis Example 1 at a proportion set such that the concentration was 12.0 mg/mL, and the hyaluronic acid derivative was dissolved.

Thereafter, the solution was stirred at 20°C for 24 hours to be dissolved. As the 10% sucrose solution, a solution obtained by sufficiently dissolving a sucrose powder (manufactured by FUJIFILM Wako Pure Chemical Corporation, for manufacturing only) in water for injection in advance and then sterilizing and filtering the solution through a 0.22 μm filter (membrane material: PTFE) was used. 10.0 mg of CyA powder (Tokyo Chemical Industry Co., Ltd., product number: C2408) was weighed in another vial, and then 0.50 mL of a polysorbate 80 aqueous solution dissolved in water for injection at a concentration of 100 mg/mL was added thereto, and the mixture was stirred with a stirring bar to disperse CyA.

Subsequently, 3.75 mL of a 12 mg/mL hyaluronic acid derivative aqueous solution was added to the vial containing the above-described CyA suspension while being stirred, 0.75 mL of a 10% sucrose aqueous solution was further added thereto, and then the drug was compounded. The solution of the association promoter-containing hyaluronic acid derivative pharmaceutical composition in which the entire CyA was compounded was visually clear. In addition, no precipitate was observed during refrigerated storage for 2 weeks. Table 11 lists the final preparation composition.

[Comparative Example 11-1]

a CyA preparation was prepared using α-cyclodextrin.

The following operation was performed in an environment at 20°C and room temperature. First, 2.0 g of sucrose and 2.0 g of α-Cyclodextrin (manufactured by Tokyo Chemical Industry Co., Ltd.) were weighed in a beaker, and about 15 mL of water for injection was added thereto for dissolution. The solution was transferred to a measuring cylinder and diluted to 20 mL. Subsequently, the solution was filtered through a 0.45 μm filter to obtain a 10% sucrose/10% α-Cyclodextrin aqueous solution. 2.0 mg of Cyclosporin A (manufactured by Tokyo Chemical Industry Co., Ltd.) was weighed in a conical tube, and 10 mL of a 10% sucrose/10% α-Cyclodextrin aqueous solution was added thereto and stirred for dissolution. Finally, the solution was filtered through a 0.2 μm filter to obtain a dosing solution. Table 11 lists the final preparation composition.

[Comparative Example 11-2]

A preparation of CyA was prepared using a hyaluronic acid derivative (35 k HA-C6-Chol-19%).

The following operation was performed in an environment at 20°C and room temperature.

The hyaluronic acid derivative (35 k HA-C6-Chol-19%) of the freeze-dried product obtained in Synthesis Example 1 was dissolved in water for injection by being stirred at 20°C and 12.0 mg/mL for 24 hours.

In addition, 200.0 mg of powder of CyA (Tokyo Chemical Industry Co., Ltd., product number: C2408) was weighed in still another vial, and 1.0 mL of ethanol was added thereto to uniformly dissolve CyA. Thereafter, 0.0225 mL of a 200

mg/mL CyA-containing ethanol solution was added dropwise to 3.75 mL of a 12 mg/mL hyaluronic acid derivative aqueous solution, and 1.2275 mL of a 10% sucrose aqueous solution was added thereto to carry out the compounding of the drug. Thereafter, the mixture was slowly stirred at room temperature for 12 hours or longer to be homogenized. The solution was visually clear, and it was confirmed that the powder was completely dissolved, and the solution was used as a dosing solution. Table 11 lists the final preparation composition.

[Table 11]

| | Item | Name of ingredient | Concentration in preparation [mg/mL] | Parts by mass with respect to 100 parts by mass of hyaluronic acid derivative |
|---|---|---|---|---|
| Example 11-1 | Hyaluronic acid derivative | 35 k HA-C6-Chol-19% | 9 | 100 |
| | Active ingredient | CyA | 2.0 | 22.2 |
| | Association promoter | Polysorbate 80 | 10 | 111 |
| Comparative Example 11-1 | α-Cyclodextrin | - | 100 | - |
| | Active ingredient | CyA | 0.2 | - |
| | Association promoter | - | - | - |
| Comparative Example 11-2 | Hyaluronic acid derivative | 35 k HA-C6-Chol-19% | 9 | 100 |
| | Active ingredient | CyA | 0.9 | 10 |
| | Association promoter | - | - | - |
| | Solvent | Ethanol | | |

<Test Example 12>

A pharmacokinetic test in rats of CyA was carried out.

The solution preparations formed of the hyaluronic acid derivative pharmaceutical compositions prepared in Example 11-1 and Comparative Example 11-2 and the solution preparation of Comparative Example 11-1 were each subcutaneously administered to normal rats (SD, 6-week-old, male) at a dose (mg/kg) listed in Table 12 using a 25 G needle.

After the administration, blood was collected from the subclavian vein over time. An equal amount of a 0.1% $ZnSO_4$ aqueous solution was added to whole blood, and then the blood was subjected to a hemolysis treatment, and then the blood was pretreated using a Micro Volume QueCHeRS Kit. The supernatant after centrifugation was filtered through a filter and measured by LC-MS/MS. The changes in the blood concentration of CyA at the time of administering various CyA preparations and the changes in the blood concentration of CyA in Comparative Example 1 are shown in FIG. 4. In FIG. 4, Example 12-1 was a case where a solution preparation formed of the hyaluronic acid derivative pharmaceutical composition of Example 11-1 was administered. Comparative Example 12-2 was a case where the solution preparation formed of the hyaluronic acid derivative pharmaceutical composition of Comparative Example 11-2 was administered, and Comparative Example 12-1 was a case where the solution preparation of Comparative Example 11-1 was administered.

In addition, an actually measured value was used as the maximum plasma concentration ($C_{max}$). The area under the curve of blood concentration - time ($AUC_{0\to\infty}$) and the area under the curve of blood concentration - 1st moment ($AUMC_{0\to\infty}$) were calculated using the linear trapezoidal method for the area up to the detectable measurement time, and using the calculated disappearance rate constant ($k_e$) approximated from three points before the detection limit for the area from the time after the measurement time to infinity. Finally, the average retention time (MRT) was determined from the following equation, and the value thereof is listed in Table 12.

$$MRT = AUMC_{0\to\infty} / AUC_{0\to\infty}$$

[Table 12]

| | CyA dose | CyA concentration in preparation | Cmax/Dose | MRT |
|---|---|---|---|---|
| | [mg/kg] | [mg/mL] | [ng/mL/mg] | [h] |
| Example 12-1 | 10 | 2 | 17.9 | 101.7 |
| Comparative Example 12-1 | 1 | 0.2 | 117 | 27 |
| Comparative Example 12-2 | 5 | 0.9 | 57 | 40.9 |

In Example 12-1 which was a preparation containing an association promoter of a hyaluronic acid derivative, the value of Cmax/Dose, which is an index of the initial release abnormality, was small and the MRT was long as compared with the preparation of Comparative Example 12-2 which did not contain an association promoter. As a result of evaluating the sustained-release properties by Expression (A), the sustained-release properties of Example 12-1 were 2.49 as compared with Comparative Example 12-2. That is, it was found that the drug remained in the blood for a long period of time. As described above, it was confirmed that the hyaluronic acid derivative composition of the present invention had excellent longer-term sustained-release properties.

<Test Example 13>

A hyaluronic acid derivative pharmaceutical composition was produced.

A solution obtained by stirring and dissolving the hyaluronic acid derivative (35 k HA-C6-Chol-19%) of the freeze-dried product obtained in Synthesis Example 1 in water for injection at 12.0 mg/mL at 20°C for 24 hours was used in Examples 13-1 and 13-2 and Comparative Example 13-1.

The following operation was performed in an environment at 20°C and room temperature.

[Example 13-1]

First, polysorbate 80 (manufactured by Thermo Fisher Scientific Inc.) was dissolved by adding water for injection at a proportion set such that the concentration was 10 mg/mL. Subsequently, glycerin (manufactured by Nacalai Tesque, Inc., product code: 17045-94) was dissolved in water for injection at a proportion set such that the concentration was 170 mg/mL, and the solution was adjusted. Further, 1 mL of a 0.5 mol/L-EDTA solution (pH of 8.0) (manufactured by NACALAI TESQUE, INC., product code: 06894-14) was added to 28.240 mL of water for injection, and a 5.0 mg/mL EDTA buffer solution was adjusted.

Here, 416.7 μL of the above-described hyaluronic acid derivative aqueous solution was added to a 6 ml sterilized vial, 100 μL of a 10 mg/mL polysorbate 80 aqueous solution was added thereto while the solution was stirred, and then 318.3 μL of water for injection was added thereto and mixed with the mixture. Finally, 15 μL of a 5.0 mg/mL EDTA buffer solution was added thereto to prepare a hyaluronic acid derivative aqueous solution. At this time, the EDTA concentration was 0.075 mg/mL, the glycerin concentration was 25.5 mg/mL, the polysorbate 80 concentration was 1 mg/mL, and the concentration of the hyaluronic acid derivative was 5 mg/mL. Thereafter, the solution was stirred at 20°C for 24 hours and diluted with water for injection at a proportion at which the concentration of the hyaluronic acid derivative was 1 mg/mL, and the diluted solution was subjected to GPC measurement. A hyaluronic acid derivative solution having the same composition as that of the solution containing no association promoter was adjusted, the obtained area value was defined as A1, and the degree of association promotion A2/A1 by the association promoter was calculated and listed in Table 13.

Next, a CyA preparation was prepared using a hyaluronic acid derivative (35 k HA-C6-Chol-19%) containing an association promoter (Polysorbate 80).

First, 2.5 mg of CyA was weighed in a 6 mL sterilized vial. Thereafter, 450 μL of the hyaluronic acid derivative solution prepared in Example 13-1 was added thereto, and the mixture was stirred at 20°C for 24 hours. Undissolved CyA powder was observed, and the operation proceeded to the next step in a saturated state. The CyA-containing hyaluronic acid derivative pharmaceutical composition prepared above was sterilized and filtered through a 0.22 μm PES filter having a diameter of 13 mmφ, and the precipitated drug that could not be enclosed in the hyaluronic acid derivative was removed, and the concentration of CyA in the obtained filtrate was quantified by reverse phase HPLC. The experiment was carried out with n = 2, and the concentrations were 1.50 mg/mL and 1.41 mg/mL. The obtained average value of the CyA concentration in the preparation is collectively listed in Table 13. In addition, whether or not precipitation occurred was verified by the same method as in Test Example 7. The results are also collectively listed in Table 13.

[Example 13-2]

An association promoter-containing hyaluronic acid derivative aqueous solution was adjusted in the same manner as in Example 13-1 except that the polysorbate concentration was 3 mg/mL and all the other concentrations were the same.

At this time, the EDTA concentration was 0.075 mg/mL, the glycerin concentration was 25.5 mg/mL, the polysorbate 80 concentration was 3 mg/mL, and the concentration of the hyaluronic acid derivative was 5 mg/mL. Thereafter, the solution was stirred at 20°C for 24 hours and diluted with water for injection at a proportion at which the concentration of the hyaluronic acid derivative was 1 mg/mL, and the diluted solution was subjected to GPC measurement. A hyaluronic acid derivative solution having the same composition as that of Example 13-1 except that it did not contain an association promoter was adjusted, the obtained area value was defined as A1, and the degree of association promotion by the association promoter was calculated in the same manner as in Example 13-1, and the results are listed in Table 13.

Next, a CyA preparation was prepared using a hyaluronic acid derivative (35 k HA-C6-Chol-19%) containing an association promoter (Polysorbate 80).

First, 2.5 mg of CyA was weighed in a 6 mL sterilized vial. Thereafter, 450 μL of the hyaluronic acid derivative solution prepared in Example 13-2 was added thereto, and the mixture was stirred at 20°C for 24 hours. Undissolved CyA powder was observed, and the operation proceeded to the next step in a saturated state. The CyA-containing hyaluronic acid derivative pharmaceutical composition prepared above was sterilized and filtered through a 0.22 μm PES filter having a diameter of 13 mmφ, and the precipitated drug that could not be enclosed in the hyaluronic acid derivative was removed, and the concentration of CyA in the obtained filtrate was quantified by reverse phase HPLC. This experiment was carried out with n = 2, and the concentrations were 1.89 mg/mL and 1.87 mg/mL. The obtained average value of the CyA concentration in the preparation is collectively listed in Table 13. In addition, whether or not precipitation occurred was verified by the same method as in Test Example 7. The results are also collectively listed in Table 13.

[Comparative Example 13-1]

A hyaluronic acid derivative aqueous solution was adjusted in the same manner as in Example 13-1 except that the polysorbate concentration was 0 mg/mL and all the other concentrations were the same.

At this time, the EDTA concentration was 0.075 mg/mL, the glycerin concentration was 25.5 mg/mL, the polysorbate 80 concentration was 0 mg/mL, and the concentration of the hyaluronic acid derivative was 5 mg/mL. Thereafter, the solution was stirred at 20°C for 24 hours and diluted with water for injection at a proportion at which the concentration of the hyaluronic acid derivative was 1 mg/mL, and the diluted solution was subjected to GPC measurement.

Next, a CyA preparation was prepared using a hyaluronic acid derivative (35 k HA-C6-Chol-19%).

First, 2.5 mg of CyA was weighed in a 6 mL sterilized vial. Thereafter, 450 μL of the hyaluronic acid derivative solution prepared in Comparative Example 13-1 was added thereto, and the mixture was stirred at 20°C for 24 hours. Undissolved CyA powder was observed, and the operation proceeded to the next step in a saturated state. The CyA-containing hyaluronic acid derivative pharmaceutical composition prepared above was attempted to be sterilized and filtered through a 0.22 μm PES filter having a diameter of 13 mmφ, but filtration was difficult.

Here, the GPC peak of the association promoter alone was also verified under the condition that the hyaluronic acid derivative was not contained, but the peak derived from the association promoter was not detected at all at the peak position of the hyaluronic acid derivative. The final composition is listed in Table 13.

[Table 13]

| | Association promoter | Content of association promoter in units of parts by mass with respect to 100 parts by mass of hyaluronic acid derivative | Substantial amount of active ingredient (C) (CyA) in units of parts by mass with respect to 100 parts by mass of hyaluronic acid derivative | Degree of association promotion A2/A1 [-] | Precipitation at physiological saline concentration |
|---|---|---|---|---|---|
| Example 13-1 | Polysorbate 8 | 200 | 29.1 | 2.10 | Precipitated |
| Example 13-2 | Polysorbate 8 | 600 | 37.6 | 1.90 | Precipitated |

(continued)

| | Association promoter | Content of association promoter in units of parts by mass with respect to 100 parts by mass of hyaluronic acid derivative | Substantial amount of active ingredient (C) (CyA) in units of parts by mass with respect to 100 parts by mass of hyaluronic acid derivative | Degree of association promotion A2/A1 [-] | Precipitation at physiological saline concentration |
|---|---|---|---|---|---|
| Comparative Example 13-1 | Polysorbate 8 | 0 | - | - | - |

As a result, it was shown that polysorbate 80 promotes the association of the hyaluronic acid derivative even in the glycerin-containing EDTA solution used as an isotonic agent (A2/A1 = 1.90 to 2.10). In addition, the results suggest that the sterilization filtration properties of the hyaluronic acid derivative pharmaceutical composition were improved by association promotion.

Furthermore, it was confirmed that the amount of CyA, which is an active ingredient, is 29.1 or 37.6 parts by mass with respect to 100 parts by mass of the hyaluronic acid derivative, and the CyA can be solubilized at a high concentration, and thus a longer sustained-release is expected by eye drop, intramuscular or subcutaneous administration, or the like.

<Test Example 14>

The degree of association promotion of another association promoter was evaluated by GPC in the same manner as in Test Example 3.

[Example 14-1]

The hyaluronic acid derivative (35 k HA-C6-Chol-19%) of the freeze-dried product obtained in Synthesis Example 1 was dissolved in water for injection by being stirred at 20°C and 12.0 mg/mL for 24 hours. 416.7 μL of the 12.0 mg/mL hyaluronic acid derivative aqueous solution was added to a 1.5 mL Eppendorf tube, and 50 μL of polyvinyl alcohol (degree of polymerization: 500, manufactured by Nacalai Tesque, Inc., product number: 11738-62) dissolved in water for injection at a concentration of 100 mg/mL was added thereto. Thereafter, water for injection was added thereto for adjustment at a proportion set such that the total amount thereof was 1,000 μL (Table 14). Thereafter, the solution was incubated at 20°C for 2 hours, diluted 5 times with water for injection at a proportion set such that the concentration of the hyaluronic acid derivative reached 1 mg/mL, and subjected to GPC measurement.

[Example 14-2]

The hyaluronic acid derivative (10 k HA-C6-Chol-40%) of the freeze-dried product obtained in Synthesis Example 3 was dissolved in water for injection by stirring the solution at 20°C for 24 hours at 62.6 mg/mL. 79.9 μL of the 62.6 mg/mL hyaluronic acid derivative aqueous solution was added to a 1.5 mL Eppendorf tube, and 50 μL of polyvinyl alcohol (degree of polymerization: 500, manufactured by Nacalai Tesque, Inc., product number: 11738-62) dissolved in water for injection at a concentration of 100 mg/mL was added thereto. Thereafter, water for injection was added thereto for adjustment at a proportion set such that the total amount thereof was 1,000 μL (Table 14). Thereafter, the solution was incubated at 20°C for 2 hours, diluted 5 times with water for injection at a proportion set such that the concentration of the hyaluronic acid derivative reached 1 mg/mL, and subjected to GPC measurement.

[Example 14-3]

The hyaluronic acid derivative (10 k HA-C6-Chol-30%) of the freeze-dried product obtained in Synthesis Example 2 was dissolved in water for injection by stirring the solution at 20°C for 24 hours at 40.0 mg/mL. 125.0 μL of the 40.0 mg/mL hyaluronic acid derivative aqueous solution was added to a 1.5 mL Eppendorf tube, and 50 μL of polyvinyl alcohol (degree of polymerization: 500, manufactured by Nacalai Tesque, Inc., product number: 11738-62) dissolved in water for injection at a concentration of 100 mg/mL was added thereto. Thereafter, water for injection was added thereto for adjustment at a proportion set such that the total amount thereof was 1,000 μL (Table 14). Thereafter, the solution was incubated at 20°C for 2 hours, diluted 5 times with water for injection at a proportion set such that the

concentration of the hyaluronic acid derivative reached 1 mg/mL, and subjected to GPC measurement.

Here, the GPC peak of the association promoter alone under the condition that the hyaluronic acid derivative was not contained was also verified, but the peak derived from PVA, which is the association promoter, was not detected at all at the peak position of the hyaluronic acid derivative.

[Examples 14-4 to 14-6]

The hyaluronic acid derivative (35 k HA-C6-Chol-19%) of the freeze-dried product obtained in Synthesis Example 1 was dissolved in water for injection by being stirred at 20°C and 12.0 mg/mL for 24 hours. Further, the association promoter-containing hyaluronic acid derivative aqueous solution was adjusted to have a proportion set to the concentration listed in Table 14 using the 10 mg/mL polysorbate 80 aqueous solution, the 170 mg/mL glycerol aqueous solution, the water for injection, and the 5.0 mg/mL EDTA solution, which were prepared in Example 13-1.

Thereafter, the solution was incubated at 20°C for 24 hours and then subjected to GPC measurement.

[Example 14-7 and Example 14-8]

The hyaluronic acid derivative (10 k HA-C6-Chol-40%) of the freeze-dried product obtained in Synthesis Example 3 was dissolved in water for injection by stirring the solution at 20°C for 24 hours at 62.6 mg/mL. Further, the association promoter-containing hyaluronic acid derivative aqueous solution was adjusted to have a proportion set to the concentration listed in Table 14 using the 10 mg/mL polysorbate 80 aqueous solution, the 170 mg/mL glycerol aqueous solution, the water for injection, and the 5.0 mg/mL EDTA solution, which were prepared in Example 13-1.

Thereafter, the solution was incubated at 20°C for 24 hours and then subjected to GPC measurement.

[Examples 14-9 to 14-11]

The hyaluronic acid derivative (10 k HA-C6-Chol-30%) of the freeze-dried product obtained in Synthesis Example 2 was dissolved in water for injection by stirring the solution at 20°C for 24 hours at 40.0 mg/mL. Further, the association promoter-containing hyaluronic acid derivative aqueous solution was adjusted to have a proportion set to the concentration listed in Table 14 using the 10 mg/mL polysorbate 80 aqueous solution, the 170 mg/mL glycerol aqueous solution, the water for injection, and the 5.0 mg/mL EDTA solution, which were prepared in Example 13-1.

Thereafter, the solution was incubated at 20°C for 24 hours and then subjected to GPC measurement.

[Examples 14-4 to 14-11]

In any case, the sample was diluted 5 times with water for injection at a proportion at which the concentration of the hyaluronic acid derivative was 1 mg/mL, and then subjected to GPC measurement.

Here, the GPC peak of the association promoter alone was also verified under the condition that the hyaluronic acid derivative was not contained, but the peak derived from Polysorbate 80, which is the association promoter, was not detected at all at the peak position of the hyaluronic acid derivative. Table 14 lists the final composition and the degree of association promotion.

[Table 14]

| | Hyaluronic acid derivative | Association promoter | Concentration of each ingredient in association promoter-containing hyaluronic acid derivative aqueous solution [mg/mL] | Content of association promoter in units of parts by mass with respect to 100 parts by mass of hyaluronic acid derivative | Degree of association promotion A2/Al [-] |
|---|---|---|---|---|---|
| Example 14-1 | 35 k HA-C6-Chol-19% | PVA | PVA(5 mg/mL) Hyaluronic acid derivative (5 mg/mL) | 100.0 | 1.76 |
| Example 14-2 | 10 k HA-C6-Chol-40% | PVA | PVA(5 mg/mL) Hyaluronic acid derivative (5 mg/mL) | 100.0 | 1.52 |
| Example 14-3 | 10 k HA-C6-Chol-30% | PVA | PVA(5 mg/mL) Hyaluronic acid derivative (5 mg/mL) | 100.0 | 3.38 |

(continued)

| | Hyaluronic acid derivative | Association promoter | Concentration of each ingredient in association promoter-containing hyaluronic acid derivative aqueous solution [mg/mL] | Content of association promoter in units of parts by mass with respect to 100 parts by mass of hyaluronic acid derivative | Degree of association promotion A2/Al [-] |
|---|---|---|---|---|---|
| Example 14-4 | 35 k HA-C6-Chol-19% | Polysorbate 80 | Hyaluronic acid derivative (5.0 mg/mL)<br>Polysorbate 80 (0.1 mg/mL)<br>Glycerol (25.5 mg/ml)<br>EDTA (7.5 mg/mL) | 2.0 | 1.87 |
| Example 14-5 | 35 k HA-C6-Chol-19% | Polysorbate 80 | Hyaluronic acid derivative (5.0 mg/mL)<br>Polysorbate 80 (1.0 mg/mL)<br>Glycerol (25.5 mg/ml)<br>EDTA (7.5 mg/mL) | 20.0 | 2.10 |
| Example 14-6 | 35 k HA-C6-Chol-19% | Polysorbate 80 | Hyaluronic acid derivative (5.0 mg/mL)<br>Polysorbate 80 (3.0 mg/mL)<br>Glycerol (25.5 mg/ml)<br>EDTA (7.5 mg/mL) | 60.0 | 1.90 |
| Example 14-7 | 10 k HA-C6-Chol-40% | Polysorbate 80 | Hyaluronic acid derivative (5.0 mg/mL)<br>Polysorbate 80 (1.0 mg/mL)<br>Glycerol (25.5 mg/ml)<br>EDTA (7.5 mg/mL) | 20.0 | 1.45 |
| Example 14-8 | 10 k HA-C6-Chol-40% | Polysorbate 80 | Hyaluronic acid derivative (5.0 mg/mL)<br>Polysorbate 80 (3.0 mg/mL)<br>Glycerol (25.5 mg/ml)<br>EDTA (7.5 mg/mL) | 60.0 | 2.14 |
| Example 14-9 | 10 k HA-C6-Chol-30% | Polysorbate 80 | Hyaluronic acid derivative (5.0 mg/mL)<br>Polysorbate 80 (0.1 mg/mL)<br>Glycerol (25.5 mg/ml)<br>EDTA (7.5 mg/mL) | 2.0 | 1.21 |
| Example 14-10 | 10 k HA-C6-Chol-30% | Polysorbate 80 | Hyaluronic acid derivative (5.0 mg/mL)<br>Polysorbate 80 (1.0 mg/mL)<br>Glycerol (25.5 mg/ml)<br>EDTA (7.5 mg/mL) | 20.0 | 2.56 |

(continued)

| | Hyaluronic acid derivative | Association promoter | Concentration of each ingredient in association promoter-containing hyaluronic acid derivative aqueous solution [mg/mL] | Content of association promoter in units of parts by mass with respect to 100 parts by mass of hyaluronic acid derivative | Degree of association promotion A2/Al [-] |
|---|---|---|---|---|---|
| Example 14-11 | 10 k HA-C6-Chol-30% | Polysorbate 80 | Hyaluronic acid derivative (5.0 mg/mL)<br>Polysorbate 80 (3.0 mg/mL)<br>Glycerol (25.5 mg/ml)<br>EDTA (7.5 mg/mL) | 60.0 | 4.11 |

As shown in the results, it was also confirmed that the association was promoted by PVA due to the difference in molecular weight of the hyaluronic acid derivative and the difference in the cholesteryl group introduction rate in Examples 14-1 to 14-3. Therefore, it is expected that the enlargement of the hydrophobic portion in the nanoparticles will impart a function of increasing the solubilization amount of the poorly soluble drug and extending the sustained-release period after administration in vivo.

In addition, as shown in the results of Examples 14-4 to 14-11, it was found that, even in a case where the hyaluronic acid derivative was a hyaluronic acid derivative having a different molecular weight and a different cholesteryl group introduction rate, and another additive such as glycerol or EDTA was contained, the association promotion was not inhibited. JP6271672B2 describes that EDTA can play a role of a protease inhibitor and can be incorporated as a stabilizer of API, and Japanese Patent No. 4758893 describes that the efficacy of antibacterial and preservative is improved by blending glycerol. It has been shown that even in the presence of such an antibacterial and preservative agent or an API stabilizer, the association promoter properly interacts with the hyaluronic acid derivative to have a function of promoting the association of the hyaluronic acid derivative. In various other buffer solutions, antibacterial and preservative agents, and stabilizers, an effect of promoting association is expected, and it is expected that the solubilization amount of poorly soluble drugs is increased and the function of extending the sustained-release period after administration in vivo is imparted due to the enlargement of the hydrophobic portion in the nanoparticles.

<Test Example 15>

A preparation of a hyaluronic acid derivative pharmaceutical composition using semaglutide (peptide) was carried out.

[Example 15-1]

The hyaluronic acid derivative (35 k HA-C6-Chol-19%) of the freeze-dried product obtained in Synthesis Example 1 was dissolved in water for injection by being stirred at 20°C and 12.0 mg/mL for 24 hours. Thereafter, the solution was dissolved in water for injection at a proportion set such that the concentration of glycerol was 170 mg/mL in another vial. Further, the solution was diluted with water for injection at a proportion set such that the EDTA concentration was 5 mg/mL, using a 0.5 mol/l-EDTA solution (pH of 8.0) (manufactured by NACALAI TESQUE, INC.) in another vial. In addition, adjustment was made such that polysorbate 80 was diluted with water for injection at a proportion set such that the concentration thereof was 10 mg/mL in still another vial. Using the solution prepared above, an association promoter-containing hyaluronic acid derivative aqueous solution was adjusted.

Specifically, 416.7 μL of a 12.0 mg/mL hyaluronic acid derivative (35 k HA-C6-Chol-19%) aqueous solution was added, 100 μL of a 10 mg/mL polysorbate 80 aqueous solution was added while being stirred with a stirrer, and 150 μL of a 170 mg/mL glycerol solution was added. Thereafter, 318.3 μL of water for injection and 15 μL of a 5 mg/mL EDTA solution were added thereto, and the mixture was stirred overnight. In addition, a powder of semaglutide (manufactured by FunaKoshi Co., Ltd., product code: AG-CP3-0032) was dissolved in a 10 mM phosphate buffer solution with an Eppendorf tube to a concentration of 1 mg/mL, the solution was prepared, and 150 μL of the association promoter-containing hyaluronic acid derivative aqueous solution and 150 μL of a 1 mg/mL semaglutide solution were mixed with each other, and then incubated at 20°C for 1 hour to promote the compounding with semaglutide. The composition of the final preparation was adjusted to the concentration listed in Table 15.

[Example 15-2]

The hyaluronic acid derivative (10 k HA-C6-Chol-30%) of the freeze-dried product obtained in Synthesis Example 2 was dissolved in water for injection by stirring the solution at 20°C for 24 hours at 40.0 mg/mL. Thereafter, the solution was dissolved in water for injection at a proportion set such that the concentration of glycerol was 170 mg/mL in another vial. Further, the solution was diluted with water for injection at a proportion set such that the EDTA concentration was 5 mg/mL, using a 0.5 mol/l-EDTA solution (pH of 8.0) (manufactured by NACALAI TESQUE, INC.) in another vial. In addition, adjustment was made such that polysorbate 80 was diluted with water for injection at a proportion set such that the concentration thereof was 10 mg/mL in still another vial. Using the solution prepared above, an association promoter-containing hyaluronic acid derivative aqueous solution was adjusted.

Specifically, 125 μL of a 40.0 mg/mL hyaluronic acid derivative 10 k HA-C6-Chol-30%) aqueous solution was added, 100 μL of a 10 mg/mL polysorbate 80 aqueous solution was added thereto while being stirred with a stirrer, and 150 μL of a 170 mg/mL glycerol solution was added thereto. Thereafter, 610 μL of water for injection and 15 μL of a 5 mg/mL EDTA solution were added thereto, and the mixture was stirred overnight. In addition, a powder of semaglutide (manufactured by FunaKoshi Co., Ltd., product code: AG-CP3-0032) was dissolved in a 10 mM phosphate buffer solution with an Eppendorf tube to a concentration of 1 mg/mL, the solution was prepared, and 150 μL of the association promoter-containing hyaluronic acid derivative aqueous solution and 150 μL of a 1 mg/mL semaglutide solution were mixed with each other, and then incubated at 20°C for 1 hour to promote the compounding with semaglutide. The composition of the final preparation was adjusted to the concentration listed in Table 15.

In all of Examples 15-1 and 15-2, the concentration of the hyaluronic acid derivative was adjusted to 2.5 mg/mL, and the concentration of semaglutide was adjusted to 0.5 mg/mL. In Examples 15-1 and 15-2, the semaglutide compounding ratio was evaluated by the following GPC.

[GPC measurement conditions]

Device: HLC8320-GPC (manufactured by Tosoh Corporation)
Column: G3000SWXL (manufactured by Tosoh Corporation, particle diameter: 8 μm, inner diameter: 7.8 mm, length: 30 cm, product number: 8542)
Eluent: 10 mM phosphate buffer solution (pH of 7.4)
Flow rate: 1 mL/min
Injection volume: 50 μL
Detector: UV (220 nm)
Temperature: 30°C

Samples of semaglutide alone having concentrations of 0.50 mg/mL and 0.25 mg/mL were prepared and subjected to GPC measurement. At this time, a peak of the semaglutide single body was confirmed at 9.0 minutes. In a case where the compound was incorporated into the hyaluronic acid derivative composition, the peak area value of the semaglutide single body at 9.0 minutes was reduced. The concentration of semaglutide contained in the hyaluronic acid derivative composition was calculated as the semaglutide compounding ratio based on the reduction rate of the peak area. The substantial amount of the active ingredient (C) (semaglutide) in units of parts by mass with respect to 100 parts by mass of the hyaluronic acid derivative was listed in Table 15 based on the semaglutide compounding ratio calculated above.

As shown in the results, the hyaluronic acid derivative composition containing polysorbate 80 as an association promoter was efficiently compounded with semaglutide, which is a peptide drug. JP6271672B2 describes that EDTA can play a role of a protease inhibitor and can be incorporated as a stabilizer of API, but it suggests that even in the presence of such a stabilizer, the association promoter can be enclosed in the hyaluronic acid derivative while interacting properly. As shown in the results, it was speculated that long-term sustained-release was possible for many modalities by applying the long-chain peptide pharmaceutical product in addition to the cyclic peptide pharmaceutical product such as CyA. Further, it is considered that other stabilizers, preservatives such as m-cresol and phenol, D-mannitol, propylene glycol, or the like as another isotonic agent can also be utilized.

As described above, it is expected that a long-chain peptide-hyaluronic acid derivative pharmaceutical composition, in which the compounding with semaglutide is efficiently carried out in the presence of the association promoter and which is capable of long-term sustained-release in vivo, can be acquired.

[Table 15]

| | Association promoter | Preparation composition (concentration [mg/mL]) | Content of association promoter in units of parts by mass with respect to 100 parts by mass of hyaluronic acid derivative | Substantial amount of active ingredient (C) (semaglutide) in units of parts by mass with respect to 100 parts by mass of hyaluronic acid derivative |
|---|---|---|---|---|
| Example 15-1 | Polysorbate 80 | Hyaluronic acid derivative (2.5 mg/mL)<br>Polysorbate 80 (0.5 mg/mL)<br>Glycerol (12.8 mg/ml)<br>EDTA (3.75 mg/mL) | 20.0 | 5.0 |
| Example 15-2 | Polysorbate 80 | Hyaluronic acid derivative (2.5 mg/mL)<br>Polysorbate 80 (0.5 mg/mL)<br>Glycerol (12.8 mg/ml)<br>EDTA (3.75 mg/mL) | 20.0 | 0.3 |

<Synthesis of hyaluronic acid derivative>

[Synthesis Example 1B]

A hyaluronic acid derivative was prepared according to the following step 1B to step 3B.

[Step 1B]

(Synthesis of cholesteryl 6-aminohexylcarbamate hydrochloride)

Cholesteryl 6-aminohexylcarbamate hydrochloride (Chol hydrochloride) was synthesized according to the following step 1B-1 and then step 1B-2.

(Step 1B-1)

Triethylamine (TEA, 1.05 mL) was added to a solution of cholesteryl chloroformate (3.37 g, 7.5 mmol) in anhydrous dichloromethane (20 mL) under an argon atmosphere, and the mixture was stirred. Under ice cooling, 6-(t-butoxycarbonyl)amino-1-aminohexane (1.12 mL, 5 mmol) was added dropwise thereto, and the mixture was stirred under ice cooling for 30 minutes, heated to room temperature (about 25°C), and stirred overnight. The reaction mixture was washed with ultrapure water and saturated saline, dried over anhydrous magnesium sulfate, and then the solvent was distilled off under reduced pressure. The obtained residue was purified by silica gel column chromatography (eluent:ethyl acetate:n-hexane = 1:4), and the fractions of the target product were combined and the solvent was distilled off under reduced pressure.

(Step 1B-2)

The obtained residue was dissolved in ethyl acetate (40 mL), and a 4N hydrochloric acid/ethyl acetate solution (40 mL) was added thereto, followed by stirring the solution at room temperature (about 25°C) overnight. The resulting precipitate was collected by centrifugation. The obtained solid was washed four times with ethyl acetate and then dried under reduced pressure, thereby obtaining 1.2 g of cholesteryl 6-aminohexylcarbamate hydrochloride (Chol hydrochloride).

[Step 2B]

(Preparation of tetrabutylammonium (TBA) salt of hyaluronic acid)

A TBA salt (HA-TBA) of hyaluronic acid was prepared according to the following step 2B-1 and then the following step 2B-2.

(Step 2B-1)

DOWEX (registered trade name) 50WX-8-400 (manufactured by Sigma-Aldrich Co. LLC) was suspended in ultrapure water, and the resin was washed with ultrapure water about three times by decantation. A 40 mass% tetrabutylammonium hydroxide aqueous solution (TBA-OH) (manufactured by Sigma-Aldrich Co., LLC) was added in an amount of about 1.5 times the molar equivalent amount with respect to the cation exchange capacity of the resin, and the mixture was stirred for 30 minutes. The excess TBA-OH solution was removed by decantation, and the resultant was further washed with an excess of ultrapure water, thereby obtaining a TBA-chlorinated cation exchange resin.

(Step 2B-2)

A raw material sodium hyaluronate (HA-Na) having a molecular weight of 10,000 (10 kDa) was dissolved in ultrapure water at a concentration of 15 mg/mL. The suspension of the TBA chlorinated cation exchange resin in "Step 2B-1" was added in an amount of 5 times the molar equivalent amount of the resin in terms of the ion exchange capacity with respect to the molar amount of the HA unit (unit molecular weight: 401.3). The solution was stirred for 15 minutes and filtered through a filter having a pore size of 0.45 μm, and the filtrate was freeze-dried, thereby obtaining a TBA salt (HA-TBA) of hyaluronic acid as a white solid.

[Step 3B]

An anhydrous DMSO solution (10 mg/mL) of HA-TBA prepared in "Step 2B-2" was prepared. Then, the amount of the Chol hydrochloride added was adjusted so that the molar ratio thereof to the disaccharide repeating unit (HA unit) present in the HA-TBA synthesized in the "Step 1B" was 44/100. Next, 4-(4,6-dimethoxy-1,3,5-triazin-2-yl)-4-methylmorpholinium chloride (DMT-MM) was added to the HA unit so that the addition amount thereof was 48/100 in terms of molar ratio, and the mixture was stirred overnight at room temperature (about 25°C). The reaction solution was dialyzed (Spectra/Por 7, molecular weight cut-off (MWCO): 3,500) in order of a 0.3 M ammonia acetate/DMSO solution, a 0.15 M NaCl aqueous solution, and ultrapure water. The obtained dialysis solution was freeze-dried to obtain a target substance (HA-$C_6$-Chol) as a white solid. In the $^1$H-NMR spectrum of the product, a peak derived from an acetyl group of N-acetyl-D-glucosamine ($COCH_3$, 1.6 ppm or more and 2.0 ppm or less, 3H) and a peak derived from a methyl group in a cholesteryl group ($CH_3$, 0.7 ppm, 3H) were confirmed, and the cholesterol introduction rate was 44%.

<Test Example 1B>

[Example 1B-1]

Cyclosporin (CyA), which is a poorly water-soluble peptide, was formulated from powder using a hyaluronic acid derivative (10 k HA-C6-Chol-44%).

[Step of obtaining aqueous solution (II)]

The hyaluronic acid derivative (10 k HA-C6-Chol-44%) of the freeze-dried product obtained in Synthesis Example 1B was dissolved in water for injection at 36.0 mg/mL. As a result, an aqueous solution (II), which is a hyaluronic acid derivative aqueous solution, was obtained.

[Step of obtaining dispersion liquid (I)]

Polysorbate 80 was diluted with water for injection at a proportion of 10 mg/mL in another vial.
4.0 mg of powder CyA (manufactured by Tokyo Chemical Industry Co., Ltd., product number: C2408) was weighed in another vial, and then 80 μL of 10 mg/mL polysorbate 80 was added thereto. In this manner, a CyA dispersion

liquid in which CyA was dispersed in polysorbate 80 was obtained as the dispersion liquid (I).

[Mixing step]

0.30 mL of a hyaluronic acid derivative aqueous solution which was an aqueous solution (II) was added thereto while being stirred in a vial with a stirring bar, and then the CyA dispersion liquid was added at a proportion set such that the liquid amount was 0.650 mL. Thereafter, the mixture was stirred for 24 hours to solubilize the drug. Table 16 lists the final preparation composition.

[Table 16]

| Item | Name of ingredient | Theoretical concentration in preparation [mg/mL] |
|---|---|---|
| Hyaluronic acid derivative | 10 k HA-C6-Chol-44% | 16.6 |
| Poorly water-soluble drug | CyA | 6.2 |
| Solubilizing aid | Polysorbate 80 | 1.23 |

Thereafter, the solution was filtered through a 0.45 μm sterile filtration filter, the precipitated CyA was removed, and the drug concentration in the preparation was quantified by HPLC measurement described below. The concentration of CyA in the filtered preparation is listed in Table 17.

[HPLC quantification conditions: reverse phase chromatography analysis conditions]

Column: Inert Sustain C18 particle diameter 5 μm × inner diameter 4.6 mm × length 150 mm
Column temperature: 40°C
Mobile phase: eluent A 0.1% TFA/acetonitrile
Eluent B: 0.1% TFA/water
Ratio between mobile phases: eluent A: eluent B = 9:1
Flow rate: 1 mL/min
Injection volume: 30 μL
Detector: UV (210 nm)

[Examples 1B-2 to 1B-11]

The formulation of CyA was carried out using the hyaluronic acid derivative and the solubilizing aid according to the same procedure as in Example 1B-1 except that the kind and the addition amount of the solubilizing aid were changed. The theoretical concentration of the drug in the preparation calculated from the added amount and the actual drug concentration in the preparation quantified by HPLC measurement are listed in Tables 17 and 18 for each preparation.

[Comparative Example 1B-1]

The hyaluronic acid derivative (10 k HA-C6-Chol-44%) of the freeze-dried product obtained in Synthesis Example 1B was dissolved in water for injection at 36.0 mg/mL.

4.0 mg of the powder of CyA was weighed in another vial, and then 0.30 mL of the above-described 36.0 mg/mL hyaluronic acid derivative aqueous solution was added thereto. A stirring bar was placed in a vial, and water for injection was added thereto so that the liquid amount was 0.650 mL. Thereafter, the mixture was stirred for 24 hours to solubilize the drug. Subsequently, the solution was filtered through a 0.45 μm sterile filtration filter, the precipitated CyA was removed, and the drug concentration in the preparation was quantified by HPLC measurement in the same manner as in Example 1B-1. It was confirmed that all the filtrates were transparent.

Table 4 lists the theoretical concentration of the drug in the preparation calculated from the added amount and the results of the actual drug concentration in the preparation quantified by HPLC measurement.

[Comparative Example 1B-2]

The hyaluronic acid derivative (10 k HA-C6-Chol-44%) of the freeze-dried product obtained in Synthesis Example 1B was dissolved in water for injection at 36.0 mg/mL.

4.0 mg of the powder of CyA was weighed in another vial, and then 0.1783 mL of the 36.0 mg/mL of the hyaluronic acid derivative aqueous solution was added thereto. A stirring bar was placed in a vial, and water for injection was added thereto so that the liquid amount was 0.650 mL. Thereafter, the mixture was stirred for 24 hours to solubilize the drug. Subsequently, the solution was filtered through a 0.45 μm sterile filtration filter, the precipitated CyA was removed, and the drug concentration in the preparation was quantified by HPLC measurement in the same manner as in Example 1B-1. It was confirmed that all the filtrates were transparent.

Table 19 lists the theoretical concentration of the drug in the preparation calculated from the added amount and the actual drug concentration in the preparation quantified by HPLC measurement.

[Comparative Example 1B-3]

A raw material sodium hyaluronate (HA-Na) having a molecular weight of 10,000 (10 kDa) used in Synthesis Example 1B was dissolved in water for injection at 55 mg/mL. Thereafter, polysorbate 20 and sodium hyaluronate were diluted with water for injection as appropriate so that the concentrations thereof were as listed in Table 4, thereby preparing an aqueous solution.

4.0 mg of the powder of CyA was weighed in another vial, and then 0.65 mL of the HA-Na aqueous solution containing polysorbate 20 was added thereto. A stirring bar was placed in a vial and stirred for 24 hours to solubilize the drug. Subsequently, the solution was filtered through a 0.45 μm sterile filtration filter, the precipitated CyA was removed, and the drug concentration in the preparation was quantified by HPLC measurement. Table 19 lists the theoretical concentration of the drug in the preparation calculated from the added amount and the actual drug concentration in the preparation quantified by HPLC measurement.

[Comparative Example 1B-4]

Polysorbate 80 was diluted with water for injection at a proportion of 100 mg/mL.

4.0 mg of the powder of CyA was weighed in another vial, and then 0.65 mL of the above-described 100.0 mg/mL polysorbate 80 was added thereto. A stirring bar was placed in a vial and stirred for 24 hours to solubilize the drug. Subsequently, the solution was filtered through a 0.45 μm sterile filtration filter, the precipitated CyA was removed, and the drug concentration in the preparation was quantified by HPLC measurement in the same manner as in Example 1B-1.

Table 19 lists the theoretical concentration of the drug in the preparation calculated from the added amount and the actual drug concentration in the preparation quantified by HPLC measurement.

[Comparative Examples 1B-5 to 1B-12]

The concentration of the solubilizing aid was set to the concentration listed in the tables below, and the formulation was carried out and the drug concentration was quantified by the same procedure as in Comparative Example 1B-4 or Example 1B-1 except for the other conditions. The results are listed in Tables 19 and 20.

As Span 83 used in Comparative Example 1B-9, a reagent (manufactured by Tokyo Chemical Industry Co., Ltd.) was used, and as sucrose stearic acid ester used in Comparative Examples 1B-10 to 1B-12, a reagent (manufactured by Mitsubishi Chemical Corporation) was used.

[Table 17]

| Preparation | Item | Name of ingredient | Theoretical concentration in preparation [mg/mL] | Concentration of drug (CyA) in preparation [mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Example 1B-1 | Hyaluronic acid derivative | 10 k HA-C6-Chol-44% | *16.6* | 3.96 | 20 or more | 64 or more | 7.41 | 23.9 |
| | Poorly water-soluble drug | CyA | 6.2 | | | | | |
| | Solubilizing aid | Polysorbate 80 | 1.23 | | | | | |
| Example 1B-2 | Hyaluronic acid derivative | 10 k HA-C6-Chol-44% | 16.6 | 4.28 | *20 or more* | *64 or more* | 14.82 | 25.8 |
| | Poorly water-soluble drug | CyA | *6.2* | | | | | |
| | Solubilizing aid | Polysorbate 80 | 2.46 | | | | | |
| Example 1B-3 | Hyaluronic acid derivative | 10 k HA-C6-Chol-44% | 16.6 | 4.2 | 20 or more | 64 or more | 19.76 | 25.3 |
| | Poorly water-soluble drug | CyA | 6.2 | | | | | |
| | Solubilizing aid | Polysorbate 80 | 3.28 | | | | | |

(continued)

| Preparation | Item | Name of ingredient | Theoretical concentration in preparation [mg/mL] | Concentration of drug (CyA) in preparation [mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Example 1B-4 | Hyaluronic acid derivative | 10 k HA-C6-Chol-44% | 16.6 | 4.35 | 20 or more | 64 or more | 29.64 | 26.2 |
| | Poorly water-soluble drug | CyA | 6.2 | | | | | |
| | Solubilizing aid | Polysorbate 80 | 4.92 | | | | | |
| Example 1B-5 | Hyaluronic acid derivative | 10 k HA-C6-Chol-44% | *16.6* | 3.77 | 20 or more | 57 or more | 14.82 | 22.7 |
| | Poorly water-soluble drug | CyA | 6.2 | | | | | |
| | Solubilizing aid | Polysorbate 80 | 2.46 | | | | | |

[Table 18]

| Preparation | Item | Name of ingredient | Theoretical concentration in preparation [mg/mL] | Concentration of drug (CyA) in preparation [mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Example 1B-6 | Hyaluronic acid derivative | 10 k HA-C6-Chol-44% | 16.6 | 3.85 | 20 or more | 57 or more | 19.76 | 23.2 |
| | Poorly water-soluble drug | CyA | 6.2 | | | | | |
| | Solubilizing aid | Polysorbate 20 | 3.28 | | | | | |
| Example 1B-7 | Hyaluronic acid derivative | 10 k HA-C6-Chol-44% | 16.6 | 4.1 | 20 or more | 57 or more | 29.64 | 24.7 |
| | Poorly water-soluble drug | CyA | 6.2 | | | | | |
| | Solubilizing aid | Polysorbate 20 | 4.92 | | | | | |
| Example 1B-8 | Hyaluronic acid derivative | 10 k HA-C6-Chol-44% | 16.6 | 4.15 | 98 or more | 225 or more | 7.41 | 25 |
| | Poorly water-soluble drug | CyA | 6.2 | | | | | |
| | Solubilizing aid | Poloxamer 188 | 1.23 | | | | | |

(continued)

| Preparation | Item | Name of ingredient | Theoretical concentration in preparation [mg/mL] | Concentration of drug (CyA) in preparation [mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Example 1B-9 | Hyaluronic acid derivative | 10 k HA-C6-Chol-44% | 16.6 | 4.24 | 98 or more | 225 or more | 14.82 | 25.5 |
| | Poorly water-soluble drug | CyA | 6.2 | | | | | |
| | Solubilizing aid | Poloxamer 188 | 2.46 | | | | | |
| Example 1B-10 | Hyaluronic acid derivative | 10 k HA-C6-Chol-44% | 16.6 | 3.87 | 98 or more | 225 or more | 19.76 | 23.3 |
| | Poorly water-soluble drug | CyA | 6.2 | | | | | |
| | Solubilizing aid | Poloxamer 188 | 3.28 | | | | | |
| Example 1B-11 | Hyaluronic acid derivative | 10 k HA-C6-Chol-44% | 16.6 | 4.01 | 98 or more | 225 or more | 29.64 | 24.2 |
| | Poorly water-soluble drug | CyA | 6.2 | | | | | |
| | Solubilizing aid | Poloxamer 188 | 4.92 | | | | | |

[Table 19]

| Preparation | Item | Name of ingredient | Theoretical concentration in preparation [mg/mL] | Concentration of drug (CyA) in preparation [mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Comparative Example 1B-1 | Hyaluronic acid derivative | 10 k HA-C6-Chol-44% | 9.88 | 0.68 | 0 | 0 | 0 | 6.9 |
| | Poorly water-soluble drug | CyA | 6.2 | | | | | |
| | Solubilizing aid | - | 0 | | | | | |
| Comparative Example 1B-2 | Hyaluronic acid derivative | 10 k HA-C6-Chol-44% | 36 | 3.57 | 0 | 0 | 0 | 9.9 |
| | Poorly water-soluble drug | CyA | 6.2 | | | | | |
| | Solubilizing aid | - | 0 | | | | | |
| Comparative Example 1B-3 | Hyaluronic acid derivative | 10 kDa HA-Na | 50 | 0.053 | 20 or more | 57 or more | 9.8 | 0.106 |
| | Poorly water-soluble drug | CyA | 6.2 | | | | | |
| | Solubilizing aid | Polysorbate 20 | 4.92 | | | | | |

(continued)

| Preparation | Item | Name of ingredient | Theoretical concentration in preparation [mg/mL] | Concentration of drug (CyA) in preparation [mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Comparative Example 1B-4 | Hyaluronic acid derivative | | 0 | 1.45 | 20 or more | 64 or more | - | - |
| | Poorly water-soluble drug | CyA | 6.2 | | | | | |
| | Solubilizing aid | Polysorbate 80 | 100 | | | | | |
| Comparative Example 1B-5 | Hyaluronic acid derivative | | 0 | 0.097 | 20 or more | 57 or more | - | - |
| | Poorly water-soluble drug | CyA | 6.2 | | | | | |
| | Solubilizing aid | Polysorbate 20 | 100 | | | | | |
| Comparative Example 1B-6 | Hyaluronic acid derivative | | 0 | 0.13 | 35 | 127 | - | - |
| | Poorly water-soluble drug | CyA | 6.2 | | | | | |
| | Solubilizing aid | Cremophor EL | 100 | | | | | |

[Table 20]

| Preparation | Item | Name of ingredient | Theoretical concentration in preparation [mg/mL] | Concentration of drug (CyA) in preparation mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Comparative Example 1B-7 | Hyaluronic acid derivative | - | 0 | 0.011 | 98 or more | 225 or more | - | - |
| | Poorly water-soluble drug | CyA | 6.2 | | | | | |
| | Solubilizing aid | Poloxamer 188 | 10 | | | | | |
| Comparative Example 1B-8 | Hyaluronic acid derivative | - | 0 | 0.82 | 20 or more | 64 or more | - | - |
| | Poorly water-soluble drug | CyA | 6.2 | | | | | |
| | Solubilizing aid | Polysorbate 80 | 10 | | | | | |
| Comparative Example 1B-9 | Hyaluronic acid derivative | 10 k HA-C6-Chol-44% | 16.6 | - | 1 | 24 | 24.1 | - |
| | Poorly water-soluble drug | CyA | 6.2 | | | | | |
| | Solubilizing aid | Span 83 | 4 | | | | | |

(continued)

| Preparation | Item | Name of ingredient | Theoretical concentration in preparation [mg/mL] | Concentration of drug (CyA) in preparation mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Comparative Example 1B-10 | Hyaluronic acid derivative | 10 k HA-C6-Chol-44% | 16.6 | - | 3 | 12 or more | 24.1 | - |
| | Poorly water-soluble drug | CyA | 6.2 | | | | | |
| | Solubilizing aid | Sucrose stearic acid ester S-270 | 4 | | | | | |
| Comparative Example 1B-11 | Hyaluronic acid derivative | 10 k HA-C6-Chol-44% | 16.6 | - | 3 | 12 or more | 24.1 | - |
| | Poorly water-soluble drug | CyA | 6.2 | | | | | |
| | Solubilizing aid | Sucrose stearic acid ester S-990 | 4 | | | | | |
| Comparative Example 1B-12 | Hyaluronic acid derivative | 10 k HA-C6-Chol-44% | 16.6 | - | 3 | 12 or more | 24.1 | - |
| | Poorly water-soluble drug | CyA | 6.2 | | | | | |
| | Solubilizing aid | Sucrose stearic acid ester S-1570 | 4 | | | | | |

As listed in Tables 17 and 18, it is presumed that in a case of formulating a poorly water-soluble drug, the drug can be solubilized without using an organic solvent, the amount of solubilizing aid to be used such as polysorbate 80 and Cremophor EL having high toxicity in the related art can be reduced, the hydrophobic interaction between the steryl groups of the hyaluronic acid derivative is promoted by the solubilizing aid, and as a result, the powder drug can be solubilized at a high concentration by increasing the amount of the hydrophobic drug carried due to the expansion of the hydrophobic moiety of the hyaluronic acid derivative, and the solubilizing aid inside the hyaluronic acid derivative composition works as a hydrophobic moiety having high mobility, not only the hydrophobic moiety that is chemically bonded to the hyaluronic acid polymer. As a result, in general, the amount of the poorly water-soluble drug to be solubilized increases in proportion to the amount of the solubilizing aid to be added. However, in the present invention, a high-concentration preparation of an active ingredient can be prepared even though the total mass of the solubilizing aid is reduced.
In Comparative Examples 1B-1 and 1B-2, the concentration of CyA in the preparation was 0.68 mg/mL at a concentration of 9.88 mg/mL of the hyaluronic acid derivative. Further, even in a case where the hyaluronic acid derivative was concentrated to 36 mg/mL and formulated, only a slight improvement effect was observed, and the concentration of CyA in the preparation was 3.57 mg/mL.
In Comparative Example 1B-3, no synergistic effect of the solubilizing aid was observed in the hyaluronic acid in which the hydrophobic group was not modified.
Comparative Examples 1B-4 to 1B-8 show the solubilizing ability of the solubilizing aid alone, but in a case of comparison with the concentration of the drug to which the solubilizing ability of the the hyaluronic acid derivative alone was added, the examples of the present invention can significantly improve the solubility of the poorly water-soluble drug.
Furthermore, as a result of intensive examination on the structure of the solubilizing aid, it was found that all of Comparative Examples 1B-9 to 1B-12 were solubilizing aids satisfying the requirement of having 4 or more carbon atoms, but having only one or three ether structures. In any of these cases, the drug and the solubilizing aid could not be stably dissolved in the hyaluronic acid derivative, and filtration was almost impossible.
As shown in the results, it can be seen that a composition formed of a solubilizing aid containing at least four or more of a hyaluronic acid derivative and an ether structure (R-O-R) and having 4 or more carbon atoms can significantly improve the solubilizing ability of a poorly water-soluble drug even in a case where the organic solvent is not used at all for the powder and the concentration of the solubilizing aid is reduced, and can provide a simple formulation method and a pharmaceutical composition having low toxicity.

<Test Example 2B>

[Examples 2B-1 to 2B-12]

The addition amount of the solubilizing aid was verified in detail.
The hyaluronic acid derivative (10 k HA-C6-Chol-44%) of the freeze-dried product obtained in Synthesis Example 1B was dissolved in water for injection so that the concentration was 5 mg/mL. 1.0 mg of the powder CyA was weighed and 0.2 mL of the solution was added to another vial so that the concentration of the solubilizing aid in the final composition in the preparation was the concentration listed in Table 21, and the mixture was stirred with a stirring bar.
Subsequently, 0.8 mL of the 5 mg/mL of the hyaluronic acid derivative aqueous solution was added thereto while being stirred, and the mixture was stirred for 24 hours to solubilize the drug. Subsequently, the solution was filtered through a 0.45 μm sterile filtration filter, the precipitated CyA was removed, and the drug concentration in the preparation was quantified by HPLC measurement in the same manner as in Example 1B-1.
Table 21 lists the theoretical concentration of the drug in the preparation calculated from the added amount and the actual drug concentration in the preparation quantified by HPLC measurement.

[Comparative Example 2B-1]

The hyaluronic acid derivative (10 k HA-C6-Chol-44%) of the freeze-dried product obtained in Synthesis Example 1B was dissolved in water for injection so that the concentration was 5 mg/mL. 1.0 mg of the powder of CyA was weighed and put into another vial, 0.1 mL of a sodium stearoyl lactylate aqueous solution having a concentration of 10 mg/mL was added thereto such that the concentration of the solubilizing aid in the final composition in the preparation was the concentration listed in Table 21, and the mixture was stirred with a stirring bar.
Subsequently, 0.8 mL of the 5 mg/mL hyaluronic acid derivative aqueous solution was added thereto while being stirred, 0.1 mL of water for injection was added thereto, and the mixture was stirred for 24 hours to solubilize the drug. Subsequently, the solution was filtered through a 0.45 μm sterile filtration filter, the precipitated CyA was removed, and the drug concentration in the preparation was quantified by HPLC measurement in the same manner as in Example 1B-1.
Table 21 lists the theoretical concentration of the drug in the preparation calculated from the added amount and the actual drug concentration in the preparation quantified by HPLC measurement.

[Comparative Examples 2B-2 to 2B-6]

1.0 mg of the powder CyA was weighed in a vial, 0.2 mL of a solubilizing aid was added thereto so that the concentration of the solubilizing aid in the final composition of the preparation was the concentration listed in Table 21, and the mixture was stirred with a stirring bar. Subsequently, 0.8 mL of water for injection was added thereto while being stirred, and the mixture was stirred for 24 hours to carry out solubilization of the drug only with the solubilizing aid. Subsequently, the solution was filtered through a 0.45 μm sterile filtration filter, the precipitated CyA was removed, and the drug concentration in the preparation was quantified by HPLC measurement in the same manner as in Example 1B-1.
Table 21 lists the theoretical concentration of the drug in the preparation calculated from the added amount and the actual drug concentration in the preparation quantified by HPLC measurement.

[Table 21]

| Preparation | Name of ingredient | Concentration of solubilizing aid in preparation [mg/mL] | Concentration of theoretical drug (CyA) in preparation [mg/mL] | Quantified concentration of drug (CyA) in preparation [mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Example 2B-1 | Polysorbate 80 | 0.0001 | 1 | 0.99 | 20 or more | 64 or more | 0.003 | 24.76 |
| Example 2B-2 | | 0.001 | 1 | 1.05 | | | 0.025 | 26.32 |
| Example 2B-3 | | 0.01 | 1 | 0.97 | | | 0.25 | 24.32 |
| Example 2B-4 | | 0.1 | 1 | 1.08 | | | 2.5 | 27.11 |
| Example 2B-5 | Polysorbate 20 | 0.0001 | 1 | 1.01 | 20 or more | 57 or more | 0.003 | 25.24 |
| Example 2B-6 | | 0.001 | 1 | 0.94 | | | 0.025 | 23.54 |
| Example 2B-7 | | 0.01 | 1 | 0.91 | | | 0.25 | 22.69 |
| Example 2B-8 | | 0.1 | 1 | 1 | | | 2.5 | 26.2 |
| Example 2B-9 | Poloxamer 188 | 0.0001 | 1 | 1.05 | 98 or more | 225 or more | 0.003 | 26.15 |
| Example 2B-10 | | 0.001 | 1 | 1 | | | 0.025 | 24.88 |
| Example 2B-11 | | 0.01 | 1 | 1 | | | 0.25 | 25.01 |
| Example 2B-12 | | 0.1 | 1 | 1.01 | | | 2.5 | 25.04 |
| Example 2B-13 | HP-α-CD | 0.1 | 1 | 1.02 | 12 or more | 36 or more | 2.5 | 25.5 |

(continued)

| Preparation | Name of ingredient | Concentration of solubilizing aid in preparation [mg/mL] | Concentration of theoretical drug (CyA) in preparation [mg/mL] | Quantified concentration of drug (CyA) in preparation [mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Comparative Example 2B-1 | Sodium stearoyl lactate | 1 | 1 | 0.82 | 0 | 21 | 25 | 20.8 |
| Comparative Example 2B-2 | Polysorbate 80 | 0.1 | 1 | 0.003 | 20 or more | 64 or more | - | - |
| Comparative Example 2B-3 | Polysorbate 20 | 0.1 | 1 | 0.001 | 20 or more | 57 or more | - | - |
| Comparative Example 2B-4 | Poloxamer 188 | 0.1 | 1 | 0.002 | 98 or more | 225 or more | - | - |
| Comparative Example 2B-5 | Cremophor EL | 0.1 | 1 | 0.004 | 35 or more | 127 or more | - | - |
| Comparative Example 2B-6 | HP-α-CD | 0.1 | 1 | 0.004 | 12 or more | 36 or more | - | - |

As shown in the results, in the solubilizing aid containing at least four or more ether structures (R-O-R) and having 4 or more carbon atoms, the drug concentration in the preparation was improved as in Examples 2B-1 to 2B-13. Meanwhile, in sodium stearoyl lactylate, which is one of the solubilizing aids that do not contain at least four or more ether structures (R-O-R), the solubilization effect was hardly recognized.

In addition, in Comparative Examples 2B-2 to 2B-6, it was confirmed that the solubilizing aid itself had almost no solubilizing ability. As shown in the results, it was found that the solubilizing ability could be significantly improved by adding a small amount of the solubilizing aid to the hyaluronic acid derivative.

As listed in Table 21, the solubilizing ability of the solubilizing aid is improved without using an organic solvent from the powder only by adding 0.003 parts by mass or greater of the hyaluronic acid derivative with respect to 100 parts by mass of the hyaluronic acid derivative.

<Test Example 3B>

[Examples 3B-1 to 3B-14]

Next, the available solubilizing aid was verified in detail.

Specifically, it was verified whether or not polyethylene glycol 300 and polyethylene glycol 400, which have at least 5 or more ether structures (R-O-R) and have 12 or more carbon atoms, can be used as solubilizing aids.

The hyaluronic acid derivative (10 k HA-C6-Chol-44%) of the freeze-dried product obtained in Synthesis Example 1B was dissolved in water for injection to a concentration of 8 mg/mL. 1.6 mg of the powder CyA was weighed in another vial, and the solubilizing aid and the water for injection were added thereto so that the total volume was 0.5 mL, and the mixture was stirred with a stirring bar. In this case, the concentration of the solubilizing aid in the finally obtained hyaluronic acid derivative pharmaceutical composition was adjusted as listed in Table 7.

Subsequently, 0.5 mL of the above-described 8 mg/mL of the hyaluronic acid derivative aqueous solution was added thereto while stirring, and the mixture was stirred for 24 hours to solubilize the drug. Subsequently, the solution was filtered through a 0.45 μm sterile filtration filter, the precipitated CyA was removed, and the drug concentration in the preparation was quantified by HPLC measurement in the same manner as in Example 1B-1.

Table 22 shows the theoretical concentration of the drug in the preparation calculated from the added amount and the results of the actual drug concentration in the preparation quantified by HPLC measurement.

[Comparative Examples 3B-1 to 3B-6]

1.0 mg of the powder CyA was weighed in a vial, and the solubilizing aid and water for injection were added thereto so that the total volume was 0.5 mL, and the mixture was stirred with a stirring bar. In this case, the concentration of the solubilizing aid in the finally obtained pharmaceutical composition containing the solubilizing aid was adjusted as listed in Table 22.

Subsequently, 0.5 mL of water for injection was added thereto while stirring, and the mixture was stirred for 24 hours to carry out solubilization of the drug only with the solubilizing aid. Subsequently, the solution was filtered through a 0.45 μm sterile filtration filter, the precipitated CyA was removed, and the drug concentration in the preparation was quantified by HPLC measurement in the same manner as in Example 1B-1.

Table 22 shows the theoretical concentration of the drug in the preparation calculated from the added amount and the results of the actual drug concentration in the preparation quantified by HPLC measurement.

[Table 22]

| Preparation | Name of ingredient | Concentration of solubilizing aid in preparation [mg/mL] | Concentration of theoretical drug (CyA) in preparation [mg/mL] | Quantified concentration of drug (CyA) in preparation [mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Example 3B-1 | Polyethylene glycol 300 | 565 | 1.6 | 1.38 | 5 or more | 12 or more | 34.5 | 14125 |
| Example 3B-2 | | 452 | | 1.4 | | | 35 | 11300 |
| Example 3B-3 | | 339 | | 1.44 | | | 36 | 8475 |
| Example 3B-4 | | 226 | | 1.2 | | | 30 | 5620 |
| Example 3B-5 | | 113 | | 1.3 | | | 32.5 | 2825 |
| Example 3B-6 | | 11.3 | | 1.06 | | | 26.5 | 282.5 |
| Example 3B-7 | | 1 | | 1.05 | | | 26.3 | 25 |
| Example 3B-8 | Polyethylene glycol 400 | 565 | | 1.65 | 7 or more | 16 or more | 41.3 | 14125 |
| Example 3B-9 | | 452 | | 1.37 | | | 34.3 | 11300 |
| Example 3B-10 | | 339 | | 1.25 | | | 31.3 | 8475 |
| Example 3B-11 | | 226 | | 1.05 | | | 26.3 | 5620 |
| Example 3B-12 | | 113 | | 1.1 | | | 27.5 | 2825 |
| Example 3B-13 | | 11.3 | | 1.06 | | | 26.5 | 282.5 |

(continued)

| Preparation | Name of ingredient | Concentration of solubilizing aid in preparation [mg/mL] | Concentration of theoretical drug (CyA) in preparation [mg/mL] | Quantified concentration of drug (CyA) in preparation [mg/mL] | Number of ether structures in solubilizing aid | Number of carbon atoms in solubilizing aid | Substantial amount of solubilizing aid in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Example 3B-14 | | 1 | | 1.15 | | | 28.8 | 25 |
| Comparative Example 3B-1 | | 565 | 1 | 0.2 | | | - | - |
| Comparative Example 3B-2 | Polyethylene glycol 300 | 452 | 1 | 009 | 5 or more | 12 or more | - | - |
| Comparative Example 3B-3 | | 339 | 1 | 0.05 | | | - | - |
| Comparative Example 3B-4 | | 565 | 1 | 0.17 | | | - | - |
| Comparative Example 3B-5 | Polyethylene glycol 400 | 452 | 1 | 0.08 | 7 or more | 16 or more | - | - |
| Comparative Example 3B-6 | | 339 | 1 | 0.05 | | | - | - |

As shown in the results, even in polyethylene glycol 300 or polyethylene glycol 400, the drug concentration in the preparation was improved as in Examples 3B-1 to 3B-14 in the solubilizing aid having at least four or more ether structures (R-O-R) and having 4 or more carbon atoms. The active ingredient could be solubilized at a higher concentration than the sum of the solubilization amounts of the single hyaluronic acid derivative and the single solubilizing aid, and a synergistic effect was exhibited.

<Test Example 4B>

[Example 4B-1]

Next, detailed verification of the method of producing a hyaluronic acid derivative pharmaceutical composition was carried out.

The hyaluronic acid derivative (10 k HA-C6-Chol-44%) of the freeze-dried product obtained in Synthesis Example 1B was dissolved in water for injection at 36.0 mg/mL. Polysorbate 80 was diluted with water for injection to 10 mg/mL in another vial.

In addition, 4.0 mg of the powder of CyA was weighed and added to another vial, and then 160 μL of 10 mg/mL of polysorbate 80 was added thereto. After addition of 0.30 mL of the above-described 36.0 mg/mL hyaluronic acid derivative aqueous solution while stirring the solution with a stirring bar in a vial, water for injection was added so that the liquid amount was 0.650 mL. Thereafter, the mixture was stirred for 24 hours to solubilize the drug. The final preparation composition is listed in Table 23. Subsequently, the solution was filtered through a 0.45 μm sterile filtration filter, the precipitated CyA was removed, and the drug concentration in the preparation was quantified by HPLC measurement in the same manner as in Example 1B-1.

Table 23 lists the theoretical concentration of the drug in the preparation calculated from the added amount and the results of the actual drug concentration in the preparation quantified by HPLC measurement.

[Example 4B-2]

The hyaluronic acid derivative (10 k HA-C6-Chol-44%) of the freeze-dried product obtained in Synthesis Example 1B was dissolved in water for injection at 36.0 mg/mL. Polysorbate 80 was diluted with water for injection to 10 mg/mL in another vial.

Thereafter, 320 μL of 10 mg/mL polysorbate 80 was added to another vial, and 0.60 mL of the above-described 36.0 mg/mL hyaluronic acid derivative aqueous solution was added thereto while being stirred in the vial containing a stirring bar, and then water for injection was added thereto so that the liquid amount was 1.30 mL. In addition, 4.0 mg of the powder CyA was weighed in another vial, 0.65 mL of the solubilizing aid-containing hyaluronic acid derivative was added thereto, and the mixture was stirred for 24 hours to carry out the solubilization of the drug. Table 20 lists the final preparation composition. Subsequently, the solution was filtered through a 0.45 μm sterile filtration filter, the precipitated CyA was removed, and the drug concentration in the preparation was quantified by HPLC measurement in the same manner as in Example 1B-1.

Table 23 lists the theoretical concentration of the drug in the preparation calculated from the added amount and the results of the actual drug concentration in the preparation quantified by HPLC measurement.

[Table 23]

| Preparation | Item | Name of ingredient | Theoretical concentration in preparation [mg/mL] | Concentration of drug (CyA) in preparation [mg/mL] | Substantial amount of poorly water-soluble drug in units of parts by mass with respect to 100 parts by mass of hyaluronic acid derivative | Substantial amount of solubilizing aid in units of parts by mass with respect to 100 parts by mass of hyaluronic acid derivative |
|---|---|---|---|---|---|---|
| Example 4B-1 | Hyaluronic acid derivative | 10 k HA-C6-Chol-44% | 16.6 | 4.28 | 25.8 | 14.82 |
| | Poorly water-soluble drug | CyA | 6.2 | | | |
| | Solubilizing aid | Polysorbate 80 | 2.46 | | | |
| Example 4B-2 | Hyaluronic acid derivative | 10 k HA-C6-Chol-44% | 16.6 | 5.38 | 32.4 | 14.82 |
| | Poorly water-soluble drug | CyA | 6.2 | | | |
| | Solubilizing aid | Polysorbate 80 | 2.46 | | | |

It has been found that a hyaluronic acid derivative pharmaceutical composition, which is excellent in increasing the solubility of the poorly water-soluble active ingredient in water from the powder, can be obtained by any method of a formulation method of preparing a preparation by mixing a step (I) of dispersing the active ingredient in a solubilizing aid and a step (II) of preparing the hyaluronic acid derivative aqueous solution or the solubilizing aid-containing hyaluronic acid aqueous solution, and a formulation method of preparing a formulation by mixing the active ingredient with the solubilizing aid-containing hyaluronic acid derivative aqueous solution. In addition, the present production method is not significantly dependent on the mixing method even in a case where the solubilizing aid is other than polysorbate 80, and thus high solubilizing ability is expected.

<Test Example 5B>

[Example 5B-1]

Next, the type of poorly water-soluble drug was verified.

The hyaluronic acid derivative (10 k HA-C6-Chol-44%) of the freeze-dried product obtained in Synthesis Example 1B was dissolved in water for injection at a proportion set such that the concentration was 36.0 mg/mL.

In addition, 1.1 mg of a powder of paclitaxel (manufactured by Tokyo Chemical Industry Co., Ltd.) was weighed and then 0.40 mL of the above-described 36.0 mg/mL hyaluronic acid derivative aqueous solution was added to the vial. While stirring the solution with a stirring bar in a vial, 0.40 mL of water for injection was added, and then 200 μL of polysorbate 80 at 10 mg/mL was added. Thereafter, the mixture was stirred for 24 hours to solubilize the drug. The final preparation composition is listed in Table 24. Subsequently, the solution was filtered through a 0.22 μm sterile filtration filter, the precipitated paclitaxel was removed, and the drug concentration in the preparation was quantified by HPLC measurement in the same manner as in Example 1B-1.

Table 24 lists the theoretical concentration of the drug in the preparation calculated from the added amount and the actual drug concentration in the preparation quantified by HPLC measurement.

[Example 5B-2]

A preparation was carried out in the same manner as in Example 5B-1 except that the poorly water-soluble drug was changed from paclitaxel to fluticasone propionate ester (manufactured by Tokyo Chemical Industry Co., Ltd.).

[Comparative Example 5B-1]

A preparation was prepared according to the same procedure as in Example 5B-1 except that the solubilizing aid was not added.

[Comparative Example 5B-2]

A preparation was prepared according to the same procedure as in Example 5B-2 except that the solubilizing aid was not added.

[Comparative Example 5B-3]

A preparation was prepared according to the same procedure as in Example 5B-1 except that the hyaluronic acid derivative was not added.

[Comparative Example 5B-4]

A preparation was prepared according to the same procedure as in Example 5B-2 except that the hyaluronic acid derivative was not added.

For Examples 5B-1 and 5B-2 and Comparative Examples 5B-1 to 5B-4, Table 24 lists the theoretical concentration of the drug in the preparation calculated from the added amount and the results of the actual drug concentration in the preparation quantified by HPLC measurement.

[Table 24]

| Preparation | Item | Name of ingredient | Theoretical concentration in preparation [mg/mL] | Concentration of drug in preparation [mg/mL] | Number of ether structures in solubilizing aid | Number of carbon atoms in solubilizing aid | Substantial amount of solubilizing aid (B) in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Example 5B-1 | Hyaluronic acid derivative | 10 k HA-C6-hol-44% | 14.4 | 0.233 | 20 or more | 64 or more | 13.9 | 1.62 |
| | Poorly water-soluble drug | Paclitaxel | 1.1 | | | | | |
| | Solubilizing aid | Polysorbate 80 | 2.0 | | | | | |
| Example 5B-2 | Hyaluronic acid derivative | 10 k HA-C6-hol-44% | 14.4 | 0.41 | 20 or more | 64 or more | 6.94 | 2.85 |
| | Poorly water-soluble drug | Fluticasone propionate ester | 2.0 | | | | | |
| | Solubilizing aid | Polysorbate 80 | 1.0 | | | | | |
| Comparative Example 5B-1 | Hyaluronic acid derivative | 10 k HA-C6-hol-44% | 14.4 | 0.096 | 0 | 0 | 0 | 0.67 |
| | Poorly water-soluble drug | Paclitaxel | 1.1 | | | | | |
| | Solubilizing aid | - | 0 | | | | | |

(continued)

| Preparation | Item | Name of ingredient | Theoretical concentration in preparation [mg/mL] | Concentration of drug in preparation [mg/mL] | Number of ether structures in solubilizing aid | Number of carbon atoms in solubilizing aid | Substantial amount of solubilizing aid (B) in units of parts by mass with respect to 100 parts by mass of hyaluronic acid derivative | Substantial amount of poorly water-soluble drug in units of parts by mass with respect to 100 parts by mass of hyaluronic acid derivative |
|---|---|---|---|---|---|---|---|---|
| Comparative Example 5B-2 | Hyaluronic acid derivative | 10 k HA-C6-hol-44% | 14.4 | 0.23 | 0 | 0 | 0 | 1.6 |
| | Poorly water-soluble drug | Fluticasone propionate ester | 2.0 | | | | | |
| | Solubilizing aid | - | 0 | | | | | |
| Comparative Example 5B-3 | Hyaluronic acid derivative | 10 k HA-C6-hol-44% | 0 | 0 (lower limit of detection) | 20 or more | 64 or more | - | 0 |
| | Poorly water-soluble drug | Paclitaxel | 1.1 | | | | | |
| | Solubilizing aid | Polysorbate 80 | 2.0 | | | | | |
| Comparative Example 5B-4 | Hyaluronic acid derivative | 10 k HA-C6-hol-44% | 0 | 0 (lower limit of detection) | 20 or more | 64 or more | - | 0 |
| | Poorly water-soluble drug | Fluticasone propionate ester | 2.0 | | | | | |
| | Solubilizing aid | Polysorbate 80 | 1.0 | | | | | |

As shown in the results, in the solubilizing aid containing at least four or more ether structures (R-O-R) and having 4 or more carbon atoms, the drug concentration in the preparation was improved as in Examples 5B-1 and 5B-2. Meanwhile, in Comparative Examples 5B-3 and 5B-4, it was confirmed that the solubilizing ability of the poorly water-soluble drug was not almost exhibited in a case where the solubilizing aid was used alone. As shown in the results, it can be seen that even in the case of low-molecular-weight poorly water-soluble drugs, the solubilizing ability can be significantly improved by adding a small amount of the solubilizing aid to the hyaluronic acid derivative.

[Example 6B-1]

The hyaluronic acid derivative (10 k HA-C6-Chol-44%) of the freeze-dried product obtained in Synthesis Example 1B was dissolved in water for injection at 36.0 mg/mL. Cholesterol-PEG600 (C1145-250MG, manufactured by Sigma-Aldrich Co., LLC) was diluted with water for injection to 10 mg/mL in another vial.

Thereafter, 400 μL of 10 mg/mL of cholesterol-PEG600 was added to another vial, and 0.60 mL of the above-described 36.0 mg/mL of the hyaluronic acid derivative aqueous solution was added thereto while being stirred with a stirring bar in the vial. In addition, 5.0 mg of powder temsirolimus (manufactured by Tokyo Chemical Industry Co., Ltd., product number: T3574) was weighed and 0.5 mL of the solubilizing aid-containing hyaluronic acid derivative was added to another vial, and the mixture was stirred for 24 hours to carry out drug solubilization. The final preparation composition is listed in Table X1. Subsequently, the solution was filtered through a 0.45 μm sterile filtration filter, the precipitated temsirolimus was removed, and the drug concentration in the preparation was quantified by HPLC measurement in the same manner as in Example 1B-1.

Table 25 shows the theoretical concentration of the drug in the preparation calculated from the added amount and the actual drug concentration in the preparation quantified by HPLC measurement.

[Comparative Example 6B-1]

The hyaluronic acid derivative (10 k HA-C6-Chol-44%) of the freeze-dried product obtained in Synthesis Example 1B was dissolved in water for injection at 36.0 mg/mL.

5.0 mg of temsirolimus (manufactured by Tokyo Chemical Industry Co., Ltd., product number: T3574) was weighed in another vial, and then 0.30 mL of the above-described 36.0 mg/mL hyaluronic acid derivative aqueous solution was added thereto. A stirring bar was placed in a vial, and water for injection was added thereto so that the liquid amount was 0.50 mL. Thereafter, the mixture was stirred for 24 hours to solubilize the drug. Subsequently, the solution was filtered through a 0.45 μm sterile filtration filter, the precipitated temsirolimus was removed, and the drug concentration in the preparation was quantified by HPLC measurement in the same manner as in Example 1B-1. It was confirmed that all the filtrates were transparent.

Table 26 lists the theoretical concentration of the drug in the preparation calculated from the added amount and the actual drug concentration in the preparation quantified by HPLC measurement.

[Comparative Example 6B-2]

5.0 mg of temsirolimus (product number: T3574, manufactured by Tokyo Chemical Industry Co., Ltd.) was weighed in a vial, then a stirring bar was put in the vial, 200 μL of 10 mg/mL cholesterol-PEG600 was added, and water for injection was added so that the liquid amount was 0.50 mL. Thereafter, the mixture was stirred for 24 hours to solubilize the drug. Subsequently, the solution was filtered through a 0.45 μm sterile filtration filter, the precipitated temsirolimus was removed, and the drug concentration in the preparation was quantified by HPLC measurement in the same manner as in Example 1B-1. It was confirmed that all the filtrates were transparent.

Table 25 shows the theoretical concentration of the drug in the preparation calculated from the added amount and the actual drug concentration in the preparation quantified by HPLC measurement.

[Table 25]

| Preparation | Item | Name of ingredient | Theoretical concentration in preparation [mg/mL] | Concentration of drug (temsirolimus) in preparation [mg/mL] | Substantial amount of poorly water-soluble drug in units of parts by mass with respect to 100 parts by mass of hyaluronic acid derivative | Substantial amount of solubilizing aid in units of parts by mass with respect to 100 parts by mass of hyaluronic acid derivative |
|---|---|---|---|---|---|---|
| Example 6B-1 | Hyaluronic acid derivative | 10 k HA-C6-Chol-44% | 21.6 | 7.29 | 33.8 | 18.5 |
| | Poorly water-soluble drug | Temsirolimus | 10.0 | | | |
| | Solubilizing aid | Cholesterol-PEG600 | 4.0 | | | |
| Comparative Example 6B-1 | Hyaluronic acid derivative | 10 k HA-C6-Chol-44% | 21.6 | 0.57 | 2.96 | 0.0 |
| | Poorly water-soluble drug | Temsirolimus | 10.0 | | | |
| | Solubilizing aid | Cholesterol-PEG600 | 0 | | | |
| Comparative Example 6B-2 | Hyaluronic acid derivative | 10 k HA-C6-Chol-44% | 0 | N.D. | - | - |
| | Poorly water-soluble drug | Temsirolimus | 10.0 | | | |
| | Solubilizing aid | Cholesterol-PEG600 | 4.0 | | | |

As shown in the results, even in the case of cholesterol-PEG600, the drug concentration in the preparation was improved as in Example 6B-1 in the case of the solubilizing aid having at least four or more ether structures (R-O-R) and having 4 or more carbon atoms. The active ingredient could be solubilized at a higher concentration than the sum of the solubilization amounts of the single hyaluronic acid derivative and the single solubilizing aid, and a synergistic effect was exhibited.

[Example 7B-1]

The hyaluronic acid derivative (10 k HA-C6-Chol-44%) of the freeze-dried product obtained in Synthesis Example 1B was dissolved in water for injection at 36.0 mg/mL. Cholesterol-PEG600 (C1145-250MG, manufactured by Sigma-Aldrich Co., LLC) was diluted with water for injection to 10 mg/mL in another vial.

Thereafter, 400 μL of 10 mg/mL of cholesterol-PEG600 was added to another vial, and 0.60 mL of the above-described 36.0 mg/mL of the hyaluronic acid derivative aqueous solution was added thereto while being stirred with a stirring bar in the vial. In addition, 4.0 mg of the powder of CyA was weighed and 5.0 mg thereof was weighed, 0.5 mL of the

solubilizing aid-containing hyaluronic acid derivative was added thereto, and the mixture was stirred for 24 hours to carry out the solubilization of the drug. The final preparation composition is listed in Table Y1. Subsequently, the solution was filtered through a 0.45 μm sterile filtration filter, the precipitated CyA was removed, and the drug concentration in the preparation was quantified by HPLC measurement in the same manner as in Example 1B-1.

Table 26 lists the theoretical concentration of the drug in the preparation calculated from the added amount and the actual drug concentration in the preparation quantified by HPLC measurement.

[Example 7B-2]

The hyaluronic acid derivative (10 k HA-C6-Chol-44%) of the freeze-dried product obtained in Synthesis Example 1B was dissolved in water for injection at 36.0 mg/mL. Cholesterol-PEG600 (C1145-250MG, manufactured by Sigma-Aldrich Co., LLC) was diluted with water for injection to 10 mg/mL in another vial.

Thereafter, 10.0 mg of the powder of CyA was weighed, 0.60 mL of the 36.0 mg/mL hyaluronic acid derivative aqueous solution was added thereto, 40 μL of a 10 mg/mL cholesterol-PEG600 aqueous solution was added thereto while stirring the mixture with a stirring bar in a vial, and 360 μL of water for injection was added thereto. Thereafter, the mixture was stirred for 24 hours to solubilize the drug. The final preparation composition is listed in Table 26. Subsequently, the solution was filtered through a 0.45 μm sterile filtration filter, the precipitated CyA was removed, and the drug concentration in the preparation was quantified by HPLC measurement in the same manner as in Example 1B-1.

Table 26 lists the theoretical concentration of the drug in the preparation calculated from the added amount and the actual drug concentration in the preparation quantified by HPLC measurement.

[Example 7B-3]

The hyaluronic acid derivative (10 k HA-C6-Chol-44%) of the freeze-dried product obtained in Synthesis Example 1B was dissolved in water for injection at 36.0 mg/mL. HP-β-CD (manufactured by Tokyo Chemical Industry Co., Ltd.: H0979) was diluted with water for injection to 10 mg/mL in another vial.

Thereafter, 10.0 mg of the powder of CyA was weighed, 0.60 mL of the 36.0 mg/mL hyaluronic acid derivative aqueous solution was subsequently added thereto, and 272.2 μL of a 10 mg/mL HP-β-CD aqueous solution was added thereto while stirring the solution with a stirring bar in the vial. Thereafter, 127.8 μL of water for injection was added thereto, and the mixture was stirred for 24 hours to solubilize the drug. The final preparation composition is listed in Table Y1. Subsequently, the solution was filtered through a 0.45 μm sterile filtration filter, the precipitated CyA was removed, and the drug concentration in the preparation was quantified by HPLC measurement in the same manner as in Example 1B-1.

Table 26 lists the theoretical concentration of the drug in the preparation calculated from the added amount and the actual drug concentration in the preparation quantified by HPLC measurement.

[Comparative Example 7B-1]

The hyaluronic acid derivative (10 k HA-C6-Chol-44%) of the freeze-dried product obtained in Synthesis Example 1B was dissolved in water for injection at 36.0 mg/mL.

10.0 mg of the powder of CyA was weighed in another vial, and then 0.60 mL of the above-described 36.0 mg/mL hyaluronic acid derivative aqueous solution was added thereto. A stirring bar was put in a vial, and water for injection was added thereto so that the liquid amount was 1.00 mL. Thereafter, the mixture was stirred for 24 hours to solubilize the drug. Subsequently, the solution was filtered through a 0.45 μm sterile filtration filter, the precipitated CyA was removed, and the drug concentration in the preparation was quantified by HPLC measurement in the same manner as in Example 1B-1. It was confirmed that all the filtrates were transparent.

Table 26 lists the theoretical concentration of the drug in the preparation calculated from the added amount and the actual drug concentration in the preparation quantified by HPLC measurement.

[Comparative Example 7B-2]

10.0 mg of the powder CyA was weighed into a vial, a stirring bar was then put into the vial, 400 μL of cholesterol-PEG600 of 10 mg/mL was added thereto, and water for injection was added thereto so that the liquid amount was 1.0 mL. Thereafter, the mixture was stirred for 24 hours to solubilize the drug. Subsequently, the solution was filtered through a 0.45 μm sterile filtration filter, the precipitated CyA was removed, and the drug concentration in the preparation was quantified by HPLC measurement in the same manner as in Example 1B-1. It was confirmed that all the filtrates were transparent.

Table 26 lists the theoretical concentration of the drug in the preparation calculated from the added amount and the actual drug concentration in the preparation quantified by HPLC measurement.

[Table 26]

| Preparation | Item | Name of ingredient | Theoretical concentration in preparation [mg/mL] | Concentration of drug (temsirolimus) in preparation [mg/mL] | Substantial amount of poorly water-soluble in units of parts by mass with respect to 100 parts by mass of hyaluronic acid derivative | Substantial amount of solubilizing aid in units of parts by mass with respect to 100 parts by mass of hyaluronic acid derivative |
|---|---|---|---|---|---|---|
| Example 7B-1 | Hyaluronic acid derivative | 10 k HA-C6-Chol-44% | 21.6 | 4.86 | 22.5 | 1.9 |
| | Poorly water-soluble drug | CyA | 10.0 | | | |
| | Solubilizing aid | Cholesterol-PEG600 | 0.4 | | | |
| Example 7B-2 | Hyaluronic acid derivative | 10 k HA-C6-Chol-44% | 21.6 | 4.53 | 21.0 | 18.5 |
| | Poorly water-soluble drug | CyA | 10.0 | | | |
| | Solubilizing aid | Cholesterol-PEG600 | 4.0 | | | |
| Example 7B-3 | Hyaluronic acid derivative | 10 k HA-C6-Chol-44% | 21.6 | 1.57 | 7.3 | 18.5 |
| | Poorly water-soluble drug | CyA | 10.0 | | | |
| | Solubilizing aid | HP-β-cyclodextrin | 4.0 | | | |
| Comparative Example 7B-1 | Hyaluronic acid derivative | 10 k HA-C6-Chol-44% | 21.6 | 0.64 | 2.96 | 0.0 |
| | Poorly water-soluble drug | CyA | 10.0 | | | |
| | Solubilizing aid | - | 0 | | | |

(continued)

| Preparation | Item | Name of ingredient | Theoretical concentration in preparation [mg/mL] | Concentration of drug (temsirolimus) in preparation [mg/mL] | Substantial amount of poorly water-soluble in units of parts by mass with respect to 100 parts by mass of hyaluronic acid derivative | Substantial amount of solubilizing aid in units of parts by mass with respect to 100 parts by mass of hyaluronic acid derivative |
|---|---|---|---|---|---|---|
| Comparative Example 7B-2 | Hyaluronic acid derivative | 10 k HA-C6-Chol-44% | 0 | N.D. | - | - |
| | Poorly water-soluble drug | CyA | 10.0 | | | |
| | Solubilizing aid | Cholesterol-PEG600 | 4.0 | | | |
| Comparative Example 7B-3 | Hyaluronic acid derivative | 10 k HA-C6-Chol-44% | 0 | N.D. | - | - |
| | Poorly water-soluble drug | CyA | 10.0 | | | |
| | Solubilizing aid | HP-β-cyclodextrin | 2.7 | | | |

As shown in the results, even in the case of cholesterol-PEG600 or HP-β-CD, the drug concentration in the preparation was improved as in Examples 7B-1 to 7B-3 in the case of the solubilizing aid having at least four or more ether structures (R-O-R) and having 4 or more carbon atoms. The active ingredient could be solubilized at a higher concentration than the sum of the solubilization amounts of the single hyaluronic acid derivative and the single solubilizing aid, and a synergistic effect was exhibited.

INDUSTRIAL APPLICABILITY

With the hyaluronic acid derivative pharmaceutical composition according to the present embodiment, it is possible to obtain a pharmaceutical composition using a hyaluronic acid derivative, which is excellent in solubilizing a large amount of a drug from a powder, maximizing the solubilization amount, controlling a release rate of an active ingredient from a subcutaneously gelated hyaluronic acid derivative composition, and sustained-releasing the active ingredient in a sustained manner for a long period of time.

## Claims

**1.** A hyaluronic acid derivative pharmaceutical composition comprising:

a hyaluronic acid derivative (A) into which a hydrophobic group has been introduced;
an association promoter (B); and
an active ingredient (C).

**2.** The hyaluronic acid derivative pharmaceutical composition according to Claim 1,

wherein the association promoter (B) has at least four or more ether structures (R-O-R) and has 4 or more carbon atoms.

3. The hyaluronic acid derivative pharmaceutical composition according to Claim 1 or 2, wherein the association promoter (B) is one or more selected from the group consisting of polysorbate 80, polysorbate 20, poloxamer, oxyethylene castor oil, polyethylene glycol 300, polyethylene glycol 400, polyethylene glycol 4000, fatty acid sorbitan ester, tocopheryl polyethylene glycol succinate, and polyvinyl alcohol.

4. The hyaluronic acid derivative pharmaceutical composition according to Claim 1 or 2, wherein a precipitate is generated at a physiological saline concentration.

5. The hyaluronic acid derivative pharmaceutical composition according to Claim 1 or 2, wherein the active ingredient (C) is at least one selected from a protein or a poorly water-soluble drug.

6. The hyaluronic acid derivative pharmaceutical composition according to Claim 5, wherein the poorly water-soluble drug has a solubility of 1 mg/mL or less in water.

7. The hyaluronic acid derivative pharmaceutical composition according to Claim 5, wherein the poorly water-soluble drug has a molecular weight of 200 or greater.

8. The hyaluronic acid derivative pharmaceutical composition according to Claim 5, wherein the poorly water-soluble drug is a poorly water-soluble peptide.

9. The hyaluronic acid derivative pharmaceutical composition according to Claim 8, wherein in the poorly water-soluble peptide, at least one nitrogen atom constituting an amide bond has a methyl group.

10. The hyaluronic acid derivative pharmaceutical composition according to Claim 8, wherein the poorly water-soluble peptide includes at least one or more selected from the group consisting of a cyclic peptide and a long-chain peptide.

11. The hyaluronic acid derivative pharmaceutical composition according to Claim 8, wherein the poorly water-soluble peptide is a cyclic peptide.

12. The hyaluronic acid derivative pharmaceutical composition according to Claim 1 or 2, wherein an amount of the active ingredient (C) is 10 parts by mass or greater and 100 parts by mass or less with respect to 100 parts by mass of the hyaluronic acid derivative (A) into which the hydrophobic group has been introduced.

13. The hyaluronic acid derivative pharmaceutical composition according to Claim 1 or 2,

wherein the hyaluronic acid derivative (A) into which the hydrophobic group has been introduced has one or more repeating units represented by General Formula (I),

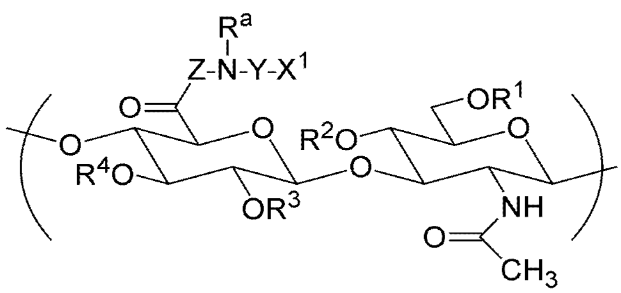

(I)

(in the formula, $R^1$, $R^2$, $R^3$, and $R^4$ each independently represent a group selected from the group consisting of a hydrogen atom, $C_{1-6}$ alkyl, formyl, and $C_{1-6}$ alkylcarbonyl,
Z represents a direct bond or a peptide linker formed of 2 or more and 30 or less of any amino acid residues,
$X^1$ represents a group selected from the group consisting of a group represented by $-NR^b-R$, $-NR^b-COO-R$, $-NR^b-CO-R$, $-NR^b-CO-NR^c-R$, $-COO-R$, $-O-COO-R$, $-S-R$, $-CO-Y^a-S-R$, $-O-CO-Y^b-S-R$, $-NR^b-CO-Y^b-S-R$, and $-S-S-R$,

$R^a$, $R^b$, and $R^c$ each independently represent a group selected from the group consisting of a hydrogen atom, $C_{1-20}$ alkyl, amino $C_{2-20}$ alkyl, and hydroxy $C_{2-20}$ alkyl,
a group selected from the group consisting of -O- and -$NR^f$- may be inserted into an alkyl moiety of each of $R^a$, $R^b$, and $R^c$,
$R^f$ represents a group selected from the group consisting of a hydrogen atom, $C_{1-12}$ alkyl, amino $C_{2-12}$ alkyl, and hydroxy $C_{2-12}$ alkyl,
a group selected from the group consisting of -O- and -NH- may be inserted into an alkyl moiety of $R^f$,
R represents a steryl group,
Y represents $C_{2-30}$ alkylene or $-(CH_2CH_2O)_m-CH_2CH_2-$,
where a group selected from the group consisting of -O-, -$NR^g$-, and -S-S- may be inserted into alkylene of Y,
$R^g$ represents a group selected from the group consisting of a hydrogen atom, $C_{1-20}$ alkyl, amino $C_{2-20}$ alkyl, and hydroxy $C_{2-20}$ alkyl,
a group selected from the group consisting of -O- and -NH- may be inserted into an alkyl moiety of $R^g$,
$Y^a$ represents $C_{1-5}$ alkylene,
$Y^b$ represents $C_{2-8}$ alkylene or $C_{2-8}$ alkenylene, and
m represents an integer of 1 or greater and 100 or less).

**14.** The hyaluronic acid derivative pharmaceutical composition according to Claim 13, wherein the steryl group is a cholesteryl group.

**15.** The hyaluronic acid derivative pharmaceutical composition according to Claim 13, wherein an introduction rate of the steryl group into the hyaluronic acid derivative is 7% or greater and less than 35%.

**16.** The hyaluronic acid derivative pharmaceutical composition according to Claim 1 or 2, wherein in the hyaluronic acid derivative pharmaceutical composition, no precipitate is visually observed.

**17.** The hyaluronic acid derivative pharmaceutical composition according to Claim 1 or 2, wherein the hyaluronic acid derivative pharmaceutical composition is filter-sterilizable.

**18.** A method of producing a hyaluronic acid derivative pharmaceutical composition including a hyaluronic acid derivative (A) into which a hydrophobic group has been introduced, an association promoter (B), and an active ingredient (C), the method comprising:

a step of mixing the hyaluronic acid derivative (A) into which a hydrophobic group has been introduced with the association promoter (B) to obtain an association promoter-containing hyaluronic acid derivative aqueous solution; and
a mixing step of mixing the active ingredient (C) with the association promoter-containing hyaluronic acid derivative aqueous solution.

**19.** A method of producing a hyaluronic acid derivative pharmaceutical composition including a hyaluronic acid derivative (A) into which a hydrophobic group has been introduced, an association promoter (B), and an active ingredient (C), the method comprising:

a step of dispersing the active ingredient (C) in the association promoter (B) to obtain a dispersion liquid (I);
a step of preparing a hyaluronic acid derivative aqueous solution or an association promoter-containing hyaluronic acid aqueous solution to obtain an aqueous solution (II); and
a step of mixing the dispersion liquid (I) with the aqueous solution (II).

**20.** The method of producing a hyaluronic acid derivative pharmaceutical composition according to Claim 18 or 19, wherein the method does not include a step of removing an organic solvent.

**21.** A hyaluronic acid derivative pharmaceutical composition comprising:

a hyaluronic acid derivative (A) into which a hydrophobic group has been introduced;
a solubilizing aid (B); and
an active ingredient (C),
wherein the solubilizing aid (B) has at least four or more ether structures (R-OR) and 4 or more carbon atoms, and an amount of the solubilizing aid (B) is 0.0001 parts by mass or greater and 15,000 parts by mass or less with

respect to 100 parts by mass of the hyaluronic acid derivative (A) into which a hydrophobic group has been introduced.

**22.** The hyaluronic acid derivative pharmaceutical composition according to Claim 21,
wherein the solubilizing aid (B) is one or more selected from the group consisting of a nonionic surfactant, polyethylene glycol having a molecular weight of 190 g/moL or greater and 4,000 g/moL or less, and a cyclodextrin derivative.

**23.** The hyaluronic acid derivative pharmaceutical composition according to Claim 21 or 22,
wherein the solubilizing aid (B) is one or more selected from the group consisting of polysorbate 80, polysorbate 65, polysorbate 60, polysorbate 40, polysorbate 20, poloxamer, polyoxyethylene hydrogenated castor oil, cyclodextrin derivative, polyethylene glycol 300, polyethylene glycol 400, polyethylene glycol 4000, and tocopheryl polyethylene glycol succinate.

**24.** The hyaluronic acid derivative pharmaceutical composition according to Claim 21 or 22,
wherein the solubilizing aid (B) is a nonionic surfactant, and an amount of the nonionic surfactant is 0.0001 parts by mass or greater and 150 parts by mass or less with respect to 100 parts by mass of the hyaluronic acid derivative (A) into which the hydrophobic group has been introduced.

**25.** The hyaluronic acid derivative pharmaceutical composition according to Claim 21 or 22,

wherein the solubilizing aid (B) is polyethylene glycol having a molecular weight of 190 g/moL or greater and 4,000 g/moL or less, and
an amount of the polyethylene glycol is 25 parts by mass or greater and 15,000 parts by mass or less with respect to 100 parts by mass of the hyaluronic acid derivative (A) into which the hydrophobic group has been introduced.

**26.** The hyaluronic acid derivative pharmaceutical composition according to Claim 21 or 22,
wherein the active ingredient (C) is a poorly water-soluble drug having a solubility of 1 mg/mL or less in water.

**27.** The hyaluronic acid derivative pharmaceutical composition according to Claim 26,
wherein the poorly water-soluble drug has a molecular weight of 200 or greater.

**28.** The hyaluronic acid derivative pharmaceutical composition according to Claim 26,
wherein the poorly water-soluble drug is a poorly water-soluble peptide.

**29.** The hyaluronic acid derivative pharmaceutical composition according to Claim 28,
wherein, in the poorly water-soluble peptide, at least one nitrogen atom constituting an amide bond has a methyl group.

**30.** The hyaluronic acid derivative pharmaceutical composition according to Claim 28,
wherein the poorly water-soluble peptide includes at least one or more selected from the group consisting of a cyclic peptide and a long-chain peptide.

**31.** The hyaluronic acid derivative pharmaceutical composition according to Claim 28,
wherein the poorly water-soluble peptide is a cyclic peptide.

**32.** The hyaluronic acid derivative pharmaceutical composition according to Claim 26,
wherein an amount of the poorly water-soluble drug to be blended is 21 parts by mass or greater and less than 100 parts by mass with respect to 100 parts by mass of the hyaluronic acid derivative (A) into which the hydrophobic group has been introduced.

**33.** The hyaluronic acid derivative pharmaceutical composition according to Claim 21 or 22,

wherein the hyaluronic acid derivative has one or more repeating units represented by General Formula (I),

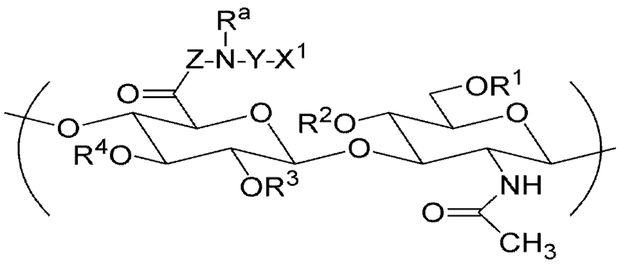

(I)

(in the formula, $R^1$, $R^2$, $R^3$, and $R^4$ each independently represent a group selected from the group consisting of a hydrogen atom, $C_{1-6}$ alkyl, formyl, and $C_{1-6}$ alkylcarbonyl,
Z represents a direct bond or a peptide linker formed of 2 or more and 30 or less of any amino acid residues,
$X^1$ represents a group selected from the group consisting of a group represented by $-NR^b-R$, $-NR^b-COO-R$, $-NR^b-CO-R$, $-NR^b-CO-NR^c-R$, -COO-R, -O-COO-R, -S-R, $-CO-Y^a-S-R$, $-O-CO-Y^b-S-R$, $-NR^b-CO-Y^b-S-R$, and -S-S-R,
$R^a$, $R^b$, and $R^c$ each independently represent a group selected from the group consisting of a hydrogen atom, $C_{1-20}$ alkyl, amino $C_{2-20}$ alkyl, and hydroxy $C_{2-20}$ alkyl,
a group selected from the group consisting of -O- and $-NR^f-$ may be inserted into an alkyl moiety of each of $R^a$, $R^b$, and $R^c$,
$R^f$ represents a group selected from the group consisting of a hydrogen atom, $C_{1-12}$ alkyl, amino $C_{2-12}$ alkyl, and hydroxy $C_{2-12}$ alkyl,
a group selected from the group consisting of -O- and -NH- may be inserted into an alkyl moiety of $R^f$,
R represents a steryl group,
Y represents $C_{2-30}$ alkylene or $-(CH_2CH_2O)_m-CH_2CH_2-$,
where a group selected from the group consisting of -O-, $-NR^g-$, and -S-S- may be inserted into the alkylene of Y,
$R^g$ represents a group selected from the group consisting of a hydrogen atom, $C_{1-20}$ alkyl, amino $C_{2-20}$ alkyl, and hydroxy $C_{2-20}$ alkyl,
a group selected from the group consisting of -O- and -NH- may be inserted into an alkyl moiety of $R^g$,
$Y^a$ represents $C_{1-5}$ alkylene,
$Y^b$ represents $C_{2-8}$ alkylene or $C_{2-8}$ alkenylene, and
m represents an integer of 1 or greater and 100 or less).

**34.** The hyaluronic acid derivative pharmaceutical composition according to Claim 33,
wherein the steryl group is a cholesteryl group.

**35.** The hyaluronic acid derivative pharmaceutical composition according to Claim 33,
wherein an introduction rate of the steryl group to the hyaluronic acid derivative is 35% or greater and less than 50%.

**36.** The hyaluronic acid derivative pharmaceutical composition according to Claim 21 or 22,
wherein an amount of the hyaluronic acid derivative is 6 mg/mL or greater and less than 45 mg/mL with respect to the hyaluronic acid derivative pharmaceutical composition.

**37.** The hyaluronic acid derivative pharmaceutical composition according to Claim 21 or 22,
wherein an amount of an organic solvent with respect to the hyaluronic acid derivative pharmaceutical composition is less than 0.8%.

**38.** A method of producing a pharmaceutical composition including a hyaluronic acid derivative (A) into which a hydrophobic group has been introduced, a solubilizing aid (B), and an active ingredient (C), the method comprising:

a step of mixing the hyaluronic acid derivative (A) with the solubilizing aid (B) to obtain a solubilizing aid-containing hyaluronic acid derivative aqueous solution; and
a mixing step of mixing the active ingredient (C) with the solubilizing aid-containing hyaluronic acid derivative aqueous solution.

**39.** A method of producing a pharmaceutical composition including a hyaluronic acid derivative (A) into which a hydrophobic group has been introduced, a solubilizing aid (B), and an active ingredient (C), the method comprising:

a step of dispersing the active ingredient (C) in the solubilizing aid (B) to obtain a dispersion liquid (I);
a step of preparing a hyaluronic acid derivative aqueous solution or a solubilizing aid-containing hyaluronic acid aqueous solution to obtain an aqueous solution (II); and
a step of mixing the dispersion liquid (I) with the aqueous solution (II).

**40.** The method of producing a pharmaceutical composition according to Claim 38 or 39, which includes the hyaluronic acid derivative (A) into which a hydrophobic group has been introduced, the solubilizing aid (B), and the active ingredient (C),
wherein the method does not include a step of removing an organic solvent.

FIG. 1

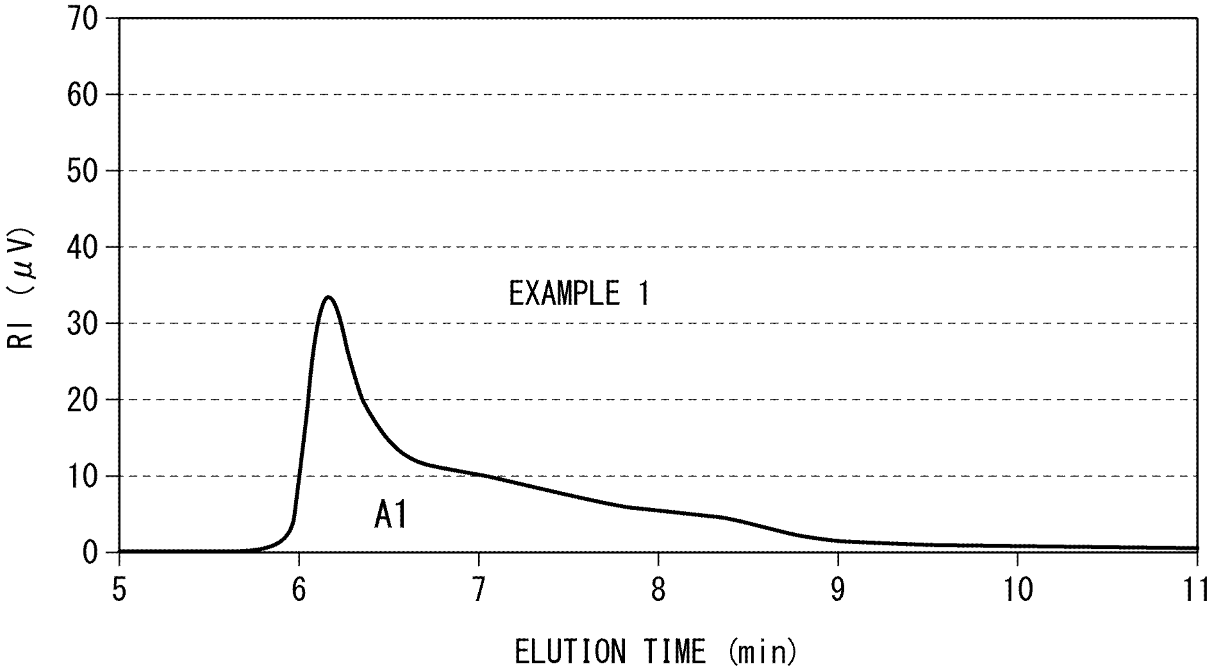
70
60
50
40
30
20
10
0
RI (μV)
EXAMPLE 1
A1
5
6
7
8
9
10
11
ELUTION TIME (min)

FIG. 2

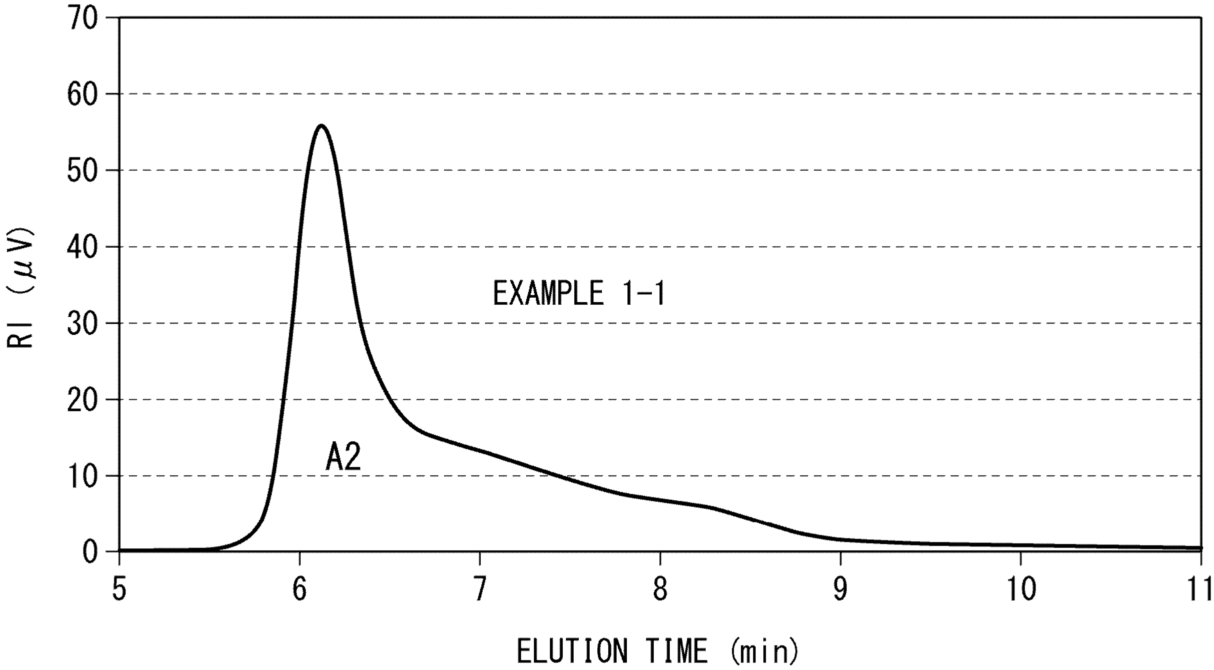
70
60
50
40
30
20
10
0
RI (μV)
EXAMPLE 1-1
A2
5
6
7
8
9
10
11
ELUTION TIME (min)

FIG. 3

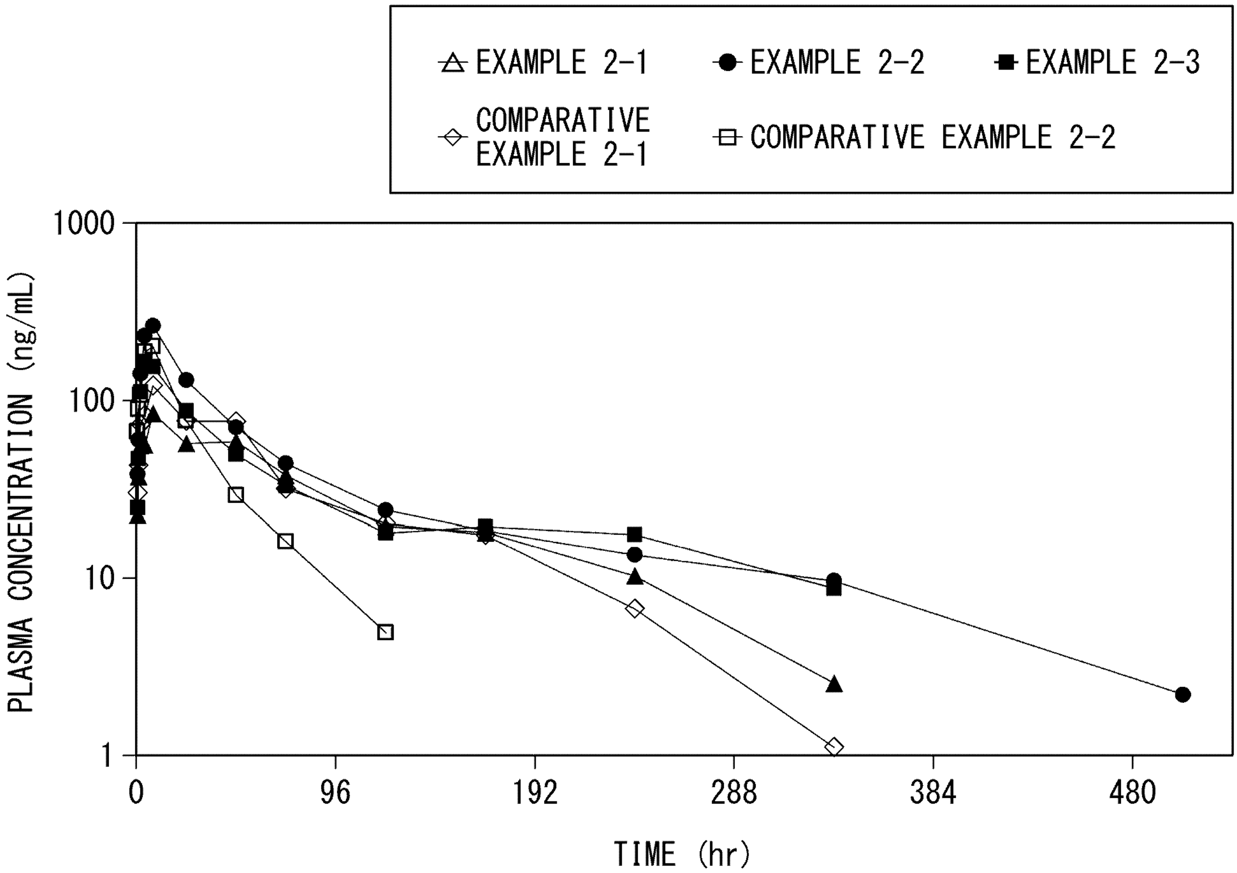
EXAMPLE 2-1
EXAMPLE 2-2
EXAMPLE 2-3
COMPARATIVE EXAMPLE 2-1
COMPARATIVE EXAMPLE 2-2
1000
100
10
1
PLASMA CONCENTRATION (ng/mL)
0
96
192
288
384
480
TIME (hr)

FIG. 4

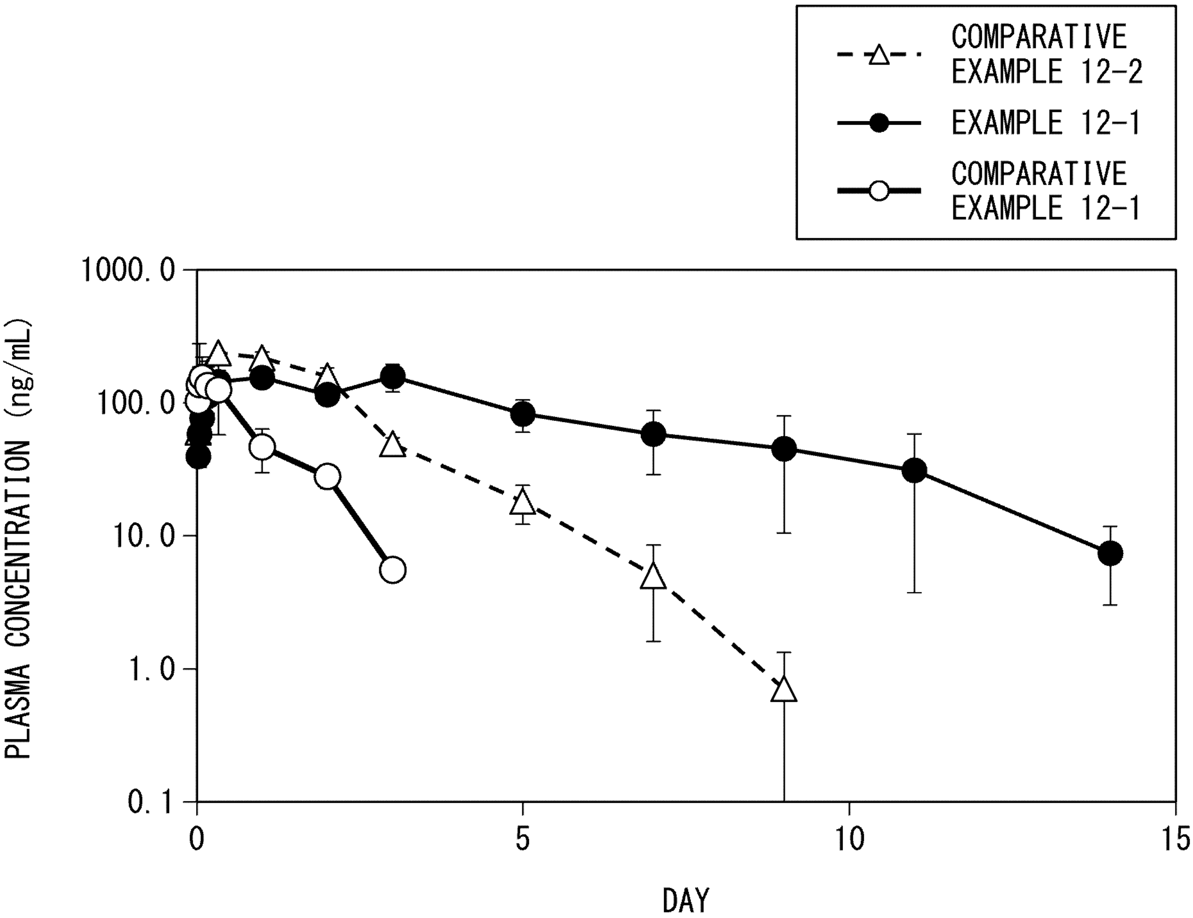
COMPARATIVE EXAMPLE 12-2
EXAMPLE 12-1
COMPARATIVE EXAMPLE 12-1
PLASMA CONCENTRATION (ng/mL)
1000.0
100.0
10.0
1.0
0.1
0
5
10
15
DAY

# INTERNATIONAL SEARCH REPORT

International application No.
**PCT/JP2023/026679**

**A. CLASSIFICATION OF SUBJECT MATTER**

***A61K 47/36***(2006.01)i; ***A61K 9/08***(2006.01)i; ***A61K 38/02***(2006.01)i; ***A61K 38/12***(2006.01)i; ***A61K 38/13***(2006.01)i; ***A61K 45/00***(2006.01)i; ***A61K 47/10***(2017.01)i; ***A61K 47/22***(2006.01)i; ***A61K 47/26***(2006.01)i; ***A61K 47/40***(2006.01)i; ***A61P 37/06***(2006.01)i

FI: A61K47/36; A61K9/08; A61K38/02; A61K38/12; A61K38/13; A61K45/00; A61K47/10; A61K47/22; A61K47/26; A61K47/40; A61P37/06

According to International Patent Classification (IPC) or to both national classification and IPC

**B. FIELDS SEARCHED**

Minimum documentation searched (classification system followed by classification symbols)

A61K47/36; A61K9/08; A61K38/02; A61K38/12; A61K38/13; A61K45/00; A61K47/10; A61K47/22; A61K47/26; A61K47/40; A61P37/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2009-79043 A (ROHTO PHARMACEUT CO LTD) 16 April 2009 (2009-04-16) claims 1-2, paragraphs [0059], [0063], [0065] | 1-5,12,16-24,38-40 |
| Y | JP 2022-44579 A (ASAHI KASEI CORP) 17 March 2022 (2022-03-17) claims 1-3, paragraphs [0026], [0057], [0088]-[0111] | 1-40 |
| Y | JP 2001-316284 A (LABORATOIRE MEDIDOM S.A) 13 November 2001 (2001-11-13) claim 1, paragraphs [0014], [0029]-[0046] | 1-40 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 October 2023** | **17 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/026679**

| Patent document cited in search report | Publication date (day/month/year) | Patent family member(s) | Publication date (day/month/year) |
|---|---|---|---|
| JP 2009-79043 A | 16 April 2009 | (Family: none) | |
| JP 2022-44579 A | 17 March 2022 | (Family: none) | |
| JP 2001-316284 A | 13 November 2001 | US 2001/0041671 A1<br>claim 1, paragraphs [0027], [0056]-[0092] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022115839 A
- JP 2022151823 A
- JP 2022115841 A
- WO 2010053140 A
- US 20200237859 A
- JP 2014534410 W
- WO 2017164409 A
- JP 4758893 B
- JP 6271672 B


### Non-patent literature cited in the description


- *Toxicology*, 2005, vol. 216, 106-121